(19) Europäisches Patentamt
European Patent Office
Office européen des brevets

(11) EP 2 265 582 B1

(12) # FASCICULE DE BREVET EUROPEEN

(45) Date de publication et mention de la délivrance du brevet:
**04.01.2012 Bulletin 2012/01**

(21) Numéro de dépôt: **09721627.9**

(22) Date de dépôt: **18.02.2009**

(51) Int Cl.:
***C07D 209/34*** (2006.01) ***C07D 401/06*** (2006.01)
***C07D 403/06*** (2006.01) ***A61K 31/404*** (2006.01)

(86) Numéro de dépôt international:
**PCT/FR2009/000179**

(87) Numéro de publication internationale:
**WO 2009/115685 (24.09.2009 Gazette 2009/39)**

(54) **NOUVEAUX DERIVES DE 3-AMINOALKYL-1,3-DIHYDRO-2H-INDOL-2-ONE, LEUR PREPARATION ET LEUR APPLICATION EN THERAPEUTIQUE.**

NEUARTIGE 3-AMINOALKYL-1,3-DIHYDRO-2H-INDOL-2-ON-DERIVATE, IHRE HERSTELLUNG UND THERAPEUTISCHE VERWENDUNG

NOVEL 3-AMINOALKYL-1,3-DIHYDRO-2H-INDOL-2-ONE DERIVATIVES, PREPARATION THEREOF AND THERAPEUTIC USE THEREOF.

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **19.02.2008 FR 0800866**

(43) Date de publication de la demande:
**29.12.2010 Bulletin 2010/52**

(73) Titulaire: **SANOFI**
**75013 Paris (FR)**

(72) Inventeurs:
- **FOULON, Loïc**
**F-75013 Paris (FR)**
- **GOULLIEUX, Laurent**
**F-75013 Paris (FR)**
- **POUZET, Brigitte**
**F-75013 Paris (FR)**
- **SERRADEIL-LE GAL, Claudine**
**F-75013 Paris (FR)**
- **VALETTE, Gérard**
**F-75013 Paris (FR)**

(74) Mandataire: **Werner, Alain Henri et al**
**Sanofi-Aventis**
**Département Brevets**
**174, avenue de France**
**75013 Paris (FR)**

(56) Documents cités:
**WO-A-01/55130** **WO-A-01/55134**
**WO-A-01/64668** **WO-A-01/98295**
**WO-A-93/15051** **WO-A-95/18105**
**WO-A-03/008407** **WO-A-2006/005609**
**WO-A-2006/100082**

EP 2 265 582 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

## Description

La présente invention se rapporte à de nouveaux dérivés de 3-aminoalkyl-1,3-dihydro-2*H*-indol-2-one, à leur préparation et à leur application en thérapeutique.

Les composés selon la présente invention présentent une forte affinité et une grande sélectivité pour les récepteurs $V_{1a}$ humains de l'arginine-vasopressine (AVP) et certains composés, selon l'invention présentent en plus une forte affinité pour les récepteurs $V_{1b}$ de l'AVP.

L'AVP est une hormone connue pour son effet antidiurétique et son effet dans la régulation de la pression artérielle. Elle stimule plusieurs types de récepteurs : $V_1$ ($V_{1a}$, $V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ils ont été clonés chez le rat et l'homme et ils régulent la majorité des effets connus de l'AVP : l'agrégation plaquettaire ; les contractions utérines ; la contraction des vaisseaux ; la contraction des cellules mésangiales rénales, la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire, anxiété, affiliation ...).

Le cortex surrénalien est aussi riche en récepteurs. $V_{1a}$ impliqués dans la production de gluco- et minéralocorticoïdes (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1a}$ et/ou $V_{1b}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1a}$ sont aussi un marqueur plus spécifique des cancers pulmonaires à petites cellules (SCLC) (P.J. WOLL et al., Biochem. Biophys. Res. Commun., 1989, 164 (1), 66-73). Ainsi, les composés selon la présente invention sont des outils de diagnostic évidents et offrent une approche thérapeutique nouvelle pour contrôler la prolifération de ces tumeurs et leur détection, à un stade même précoce (radiomarquage ; SPECT, de l'anglais Single Photon Emission Computed Tomography ; PET Scan, de l'anglais Positron Emission Tomography Scanner).

Les récepteurs $V_{1b}$ ont été initialement identifiés dans l'adénohypophyse de différentes espèces animales (rat, porc, boeuf, mouton...) y compris chez l'homme (S. Jard et al., Mol. Pharmacol., 1986, 30, 171-177 ; Y. Arsenijevic et al., J. Endocrinol., 1994, 141, 383-391 ; J. Schwartz et al., Endocrinology, 1991, 129 (2), 1107-1109 ; Y. de Keyser et al., FEBS Letters, 1994, 356, 215-220) où ils stimulent la libération d'hormone adrénocorticotrophique par l'AVP et potentialisent les effets du CRF sur la libération d'ACTH (G. E. Gdjjes et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. de Keyser, FEBS Letters, 1994,356, 215-220 ; T. Sugimoto et al., J. Biol. Chem., 1994, 269 (43), 27088-27092 ; M. Saito et al., Biochem. Biophys. Res. Commun., 1995, 212 (3), 751-757 ; S. J. Lolait et al., Neurobiology, 1996, 92, 6783-6787 ; M. A. Ventura et al., Journal of Molecular Endocrinology, 1999, 22, 251-260) et différentes études (hybridation in situ, PCR, de l'anglais Polymerase Chain Reaction...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régule le pancréas endocrine en stimulant la sécrétion d'insuline et de glucagon (B. Lee et al., Am. J. Physiol. 269 (Endocrinol. Metab. 32) : E1095-E1100, 1995) ou la production de catécholamines dans la médullo-surrénale qui est le siège d'une synthèse locale d'AVP (E. Grazzini et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétant de l'AVP et induisant de ce fait une production soutenue de catécholamines à l'origine d'hypertension résistantes aux antagonistes des récepteurs de l'angiotensine II et aux inhibiteurs de l'enzyme de conversion. Le cortex surrénalien est aussi riche en

récepteurs $V_{1a}$ impliqués dans la production de gluco- et minéralocorticoides (aldostérone et cortisol). Via ces récepteurs, l'AVP (circulante ou synthétisée localement) peut induire une production d'aldostérone avec une efficacité comparable à celle de l'angiotensine II (G. Guillon et al., Endocrinology, 1995, 136 (3), 12851295). Le cortisol est un puissant régulateur de la production d'ACTH, l'hormone du stress.

Les récepteurs $V_{1b}$ sont aussi considérés comme un marqueur des tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroidiens, induisant un syndrome de Cushing dans certains cas (J. Bertherat et al., Eur. J. Endocrinol., 1996, 135, 173 ; G. A. Wuinert et al., Lancet, 1990,335, 991-994 ; G. Dickstein et al., J. Clin. Endocrinol. Metab., 1996, 81 (8), 2934-2941). Les récepteurs $V_{1a}$ sont, quant à eux, un marqueur plus spécifique des cancers pulmonaires à petites cellules (SCLC) (P. J. Woll et al., Biochem. Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. Sugimoto et al., Molecular cloning and functional expression of V1b receptor gene, dans Neurohypophysis : Recent Progress of Vasopressin and Oxytocin Research ; T. Saito, K. Kurosawa et S. Yoshida, ed., Elsevier Science, 1995, 409413).

Des dérivés de 1,3-dihydro-2*H*-indol-2-one ont été décrits dans certaines demandes de brevet comme des ligands des récepteurs de l'arginine-vasopressine et/ou de l'ocytocine : on peut citer les demandes de brevets WO 93/15051, EP 636608, EP 636609, WO 97/15556, WO 98/25901, WO 01/55130, WO 01/55134, WO 01/64668, WO 01/98295, WO 03/008407, WO 06/080574, WO 08/025735.

La demande internationale WO 95/18105 concerne des composés de formule :

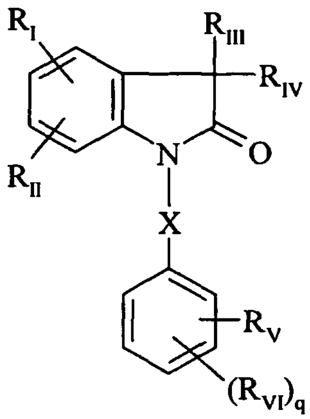

(A)

dans laquelle notamment :

- X représente $SO_2$ ;
- $R_I$, $R_{II}$, $R_{III}$, $R_{IV}$, $R_V$, $R_{VI}$ et q ont différentes valeurs.

Les composés de formule (A) ont une affinité pour les récepteurs en général de la vasopressine et/ou de l'ocytocine. En outre, cette demande ne décrit aucun exemple dans lequel $R_{II}$ est en position -6- du phényle et représente un radical méthoxy et $R_{IV}$ est toujours lié en position -3- du cycle indol-2-one par un atome d'azote.

En particulier le 3-[4-[[5,6-dichloro-3-(2-chlorophényl)-2-oxo-3-[(2-pipéridin-4-yléthyl)amino]-2,3-dihydro-1*H*-indol-1-yl]sulfonyl]phényl]-1,1-diéthylurée (composé α) est décrit à l'Exemple 220 et le 3-[4-[[5-chloro-3-(2-chlorophényl)-6-méthyl-2-oxo-3-[(2-pipéridin-4-yléthyl)amino]-2,3-dihydro-1*H*-indol-1-yl]sulfonyl]phényl]-1,1-diéthylurée (composé β) est décrit à l'Exemple 277 de WO 95/018 105.

Le composé α présente une bonne affinité pour les récepteurs $V_{1a}$ humains de l'AVP, mais aussi pour les récepteurs $V_2$ humains de l'AVP et les récepteurs de l'ocytocine ; il n'est donc pas sélectif des récepteurs $V_{1a}$ humains de l'AVP ainsi que des récepteurs $V_{1b}$ humains de l'AVP.

Le composé β présente une bonne affinité pour les récepteurs $V_{1a}$ humains de l'AVP, mais aussi pour les récepteurs $V_2$ humains de l'AVP ; il n'est donc pas sélectifs des récepteurs $V_{1a}$ humains de l'AVP ainsi que des récepteurs $V_{1b}$ humains de l'AVP.

On a maintenant trouvé de nouveaux composés qui présentent une forte affinité et une grande sélectivité pour les récepteurs $V_{1a}$ humains de l'AVP et qui sont des antagonistes desdits récepteurs et certains composés présentait en plus une forte affinité pour les récepteurs $V_{1b}$ humains de l'AVP.

La présente invention a pour objet des composés répondant à la formule (I) :

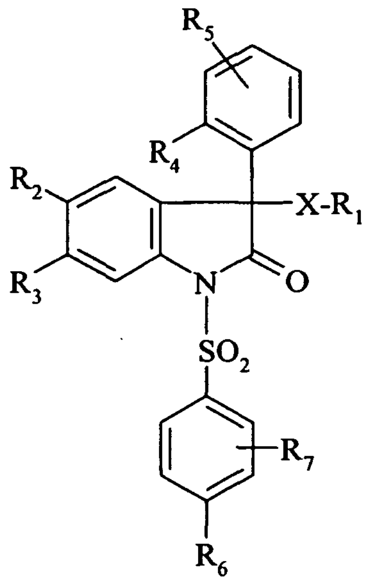

(I)

dans laquelle :

- X représente un radical bivalent $(C_1-C_5)$alkylène non substitué ou substitué une ou plusieurs fois sur un atome de carbone par un atome de fluor ou par un $(C_1-C_3)$alkyle ;
- $R_1$ représente :
  - un groupe $-NR_8R_9$ ;
  - un radical pipéridin-4-yle ou un radical pipéridin-3-yle non substitué ou substitué une ou plusieurs fois par un $(C_1-C_4)$alkyle, un $(C_3-C_5)$cycloalkyle, les atomes de carbone pouvant être également substitués par un ou plusieurs atomes de fluor ;
- $R_2$ représente un atome d'halogène, un groupe Alk, un groupe OAlk ;
- $R_3$ représente un méthoxy ;
- $R_4$ représente un atome d'hydrogène, un atome d'halogène, un groupe Alk, un hydroxy, un groupe OAlk ;
- $R_5$ représente un atome d'hydrogène, un atome d'halogène, un groupe Alk, un hydroxy, un groupe OAlk, un groupe $-CO_2Alk$, un radical $-CH_2OH$ ;
- $R_6$ représente un atome d'hydrogène, un groupe Alk, un hydroxy, un groupe OAlk, un $(C_3-C_5)$cycloalkyoxy, un groupe $-NR_{10}CONR_{11}R_{12}$ ;
- $R_7$ représente un atome d'hydrogène, un atome d'halogène, un groupe Alk, un hydroxy, un groupe OAlk ;
- ou bien $R_7$ est en position -3- du phényle et ensemble avec $R_6$ ils représentent un radical triméthylène ;
- $R_8$ et $R_9$ représentent chacun indépendamment un atome d'hydrogène ou un $(C_1-C_4)$alkyle ;
- ou bien $R_8$ et $R_9$ ensemble avec l'atome d'azote auquel ils sont liés constituent un radical hétérocyclique saturé ou insaturé, de 3 à 10 chaînons, ledit radical hétérocyclique étant non substitué ou substitué une ou plusieurs fois par un amino, un diméthylamino, un hydroxy, un groupe Alk, un $(C_3-C_5)$cycloalkyle, un groupe OAlk, un radical $-SO_2$ Alk , les atomes de carbone pouvant être également substitués par un ou plusieurs atomes de fluor ;
- $R_{10}$ représente un atome d'hydrogène ou un $(C_1-C_4)$alkyle ;
- $R_{11}$ et $R_{12}$ représentent chacun indépendamment un atome d'hydrogène ou un $(C_1-C_4)$alkyle ;
- Alk représente un $(C_1-C_4)$alkyle non substitué ou substitué une ou plusieurs fois par un atome de fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels

d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent exister sous forme d'hydrates ou de solvates, à savoir sous forme d'associations ou de combinaisons avec une ou plusieurs molécules d'eau ou avec un solvant. De tels hydrates et solvates font également partie de l'invention.

Selon la présente invention, les N-oxydes des composés comportant une amine font également partie de l'invention.

Par atome d'halogène on entend un atome de brome, de chlore, de fluor ou d'iode.

Par $(C_1\text{-}C_3)$alkyle ou respectivement $(C_1\text{-}C_4)$alkyle, on entend un radical alkyle linéaire ou ramifié de un à trois atomes de carbone ou respectivement de un à quatre atomes de carbone, tel que le radical méthyle, éthyle, propyle, isopropyle, butyle, isobutyle, *sec*-butyle, *tert*-butyle.

Par $(C_1\text{-}C_5)$alkylène on entend un radical bivalent de un à cinq atomes de carbone tel que le radical méthylène, éthylène, triméthylène, tétraméthylène ou le radical pentaméthylène.

Par $(C_1\text{-}C_4)$alcoxy on entend un radical alcoxy linéaire ou ramifié de un à quatre atomes de carbone tel que le radical méthoxy, éthoxy, propoxy, isopropoxy, butoxy, sec-butoxy, *tert*-butoxy.

Par $(C_3\text{-}C_5)$cycloalkyle on entend un radical cyclopropyle, cyclobutyle ou cyclopentyle.

Par radical hétérocyclique saturé ou insaturé de 3 ou 10 chaînons, on entend un radical hétérocyclique non aromatique mono, di ou tricyclique, condensé ou ponté, pouvant contenir un deuxième hétéroatome tel que l'azote, l'oxygène ou le soufre. Ces radicaux comprennent en particulier les radicaux suivants : 1-aziridinyle, 1-azétidinyle, 1-pyrrolidinyle, 1-pipéridinyle, 1-pipérazinyle, 1-hexahydroazépinyle, 4-morpholinyle, 4-thiomorpholinyle, 2-azabicyclo [2.2.2]oct-5-èn-2-yle, 2-méthyl-2-azoniabicy-clo[2.2.2]oct-5-èn-2-yle, 2-azaadamant-2-yle, 1,2,3,6-tétrahydropyridin-1-yle, 2-azabicyclo[2.2.1]heptan-2-yle, 2-aza-bicyclo[2.2.2]octan-2-yle, 1-azoniabicyclo[2.2.2]octan-1-yle, 1,4-diazépan-1-yle, 2,5-diazabicyclo[2.2.1]hept-2-yle, 3,8-diazabicyclo[3.2.1]oct-3-yle, 2,5-diazabicyclo[2.2.2]oct-2-yle, 1,4-diazabicyclo[3.2.1]oct-4-yle, hexahydropyrrolo[1,2-a]pyrazin-2(1H)-yle, octahydro-2H-pyrido[1,2-a]pyrazin-2-yle.

Selon la présente invention, on préfère les composés de formule (I) dans laquelle :

- X représente un radical bivalent $(C_1\text{-}C_5)$alkylène non substitué ou substitué une ou plusieurs fois sur un atome de carbone par un atome de fluor ou par un $(C_1\text{-}C_3)$ alkyle ;
- $R_1$ représente :
  - un groupe $-NR_8R_9$ ;
  - un radical pipéridin-4-yle ou un radical pipéridin-3-yle non substitué ou substitué par un méthyle ;
- $R_2$ représente un atome d'halogène, un méthyle, un méthoxy ;
- $R_3$ représente un méthoxy ;
- $R_4$ représente un atome d'hydrogène, un atome de fluor, un méthyle, un méthoxy ;
- $R_5$ représente un atome d'hydrogène, un atome d'halogène, un groupe $-CO_2Alk$, un radical $-CH_2OH$ ;
- $R_6$ représente un atome d'hydrogène, un groupe Alk, un groupe OAlk, un hydroxy, un cyclopentyloxy, un groupe $-NHCON(Et)_2$;
- $R_7$ représente un atome d'hydrogène, un atome d'halogène, un méthyle, un méthoxy, un radical $CF_3$ ;
- ou bien $R_7$ est en position -3- du phényle et ensemble avec $R_6$ ils constituent un radical triméthylène;
- $R_8$ et $R_9$ représentent chacun un méthyle ;
- ou bien $R_8$ et $R_9$ ensemble avec l'atome d'azote auquel ils sont liés constituent un radical hétérocyclique choisi parmi : la pyrrolidin-1-yle, la pipéridin-1-yle, la morpholin-4-yle, la pipérazin-1-yle, 1,4-diazépan-1-yle, 2,5-diazabicyclo[2.2.1]hept-2-yle, 3,8-diazabicyclo[3.2.1]oct-3-yle, 2,5-diazabicyclo[2.2.2]oct-2-yle, 1,4-diazabicyclo[3.2.1]oct-4-yle, hexahydropyrrolo[1,2-a]pyrazin-2(1*H*)-yle, octahydro-2H-pyrido[1,2-a]pyrazin-2-yle, ledit radical hétérocyclique étant non substitué ou substitué une ou deux fois par un atome de fluor, un amino, un diméthylamino, un hydroxy, un groupe Alk, un cyclobutyle, un radical $-SO_2Me$;
- Alk représente un $(C_1\text{-}C_4)$alkyle non substitué ou substitué une ou plusieurs fois par un atome de fluor ;

à l'état de base ou de sel d'addition à un acide, ainsi qu'à l'état d'hydrate ou de solvate.

Particulièrement, on préfère les composés de formule (I) dans laquelle :

- X représente un radical bivalent $(C_1\text{-}C_5)$alkylène;
- $R_1$ représente :
  - un diméthylamino, un 3-aminopyrrolidin-1-yle, un 3-diméthylaminopyrrolidin-1-yle, un pipéridin-1-yle, un 3,3-difluoropipéridin-1-yle, un 4,4-difluoropipéridin-1-yle, un 4-hydroxypipéridin-1-yle, un morpholin-4-yle, un 4-méthylpipérazin-1-yle, un pipérazin-1-yle, un 4-éthylpipérazin-1-yle, un 1,4-diazépan-1-yle, un 2,5-diazabicyclo [2.2.1]hept-2-yle, un 4-isopropylpipérazin-1-yle, un 3-méthylpipérazin-1-yle, un 3,4-diméthylpipérazin-1-yle, un 4-cyclobutylpipérazin-1-yle, un 5-méthyl-2,5-diazabicyclo[2.2.1]hept-2-yle, un 3,5-diméthylpipérazin-1-yle, un 4-méthyl-1,4-diazepan-1-yle, un 8-méthyl-3,8-diazabicyclo[3.2.1]oct-3-yle, un 2,6-diméthylpipérazin-1-yle, un 3-isopropylpipérazin-1-yle, un 2,2-diméthylpipérazin-1-yle, un 2,5-diazabicyclo[2.2.2]oct-2-yle, un 2,5-diméthylpipérazin-1-yle, un 2,2,4-triméthylpipérazin-1-yle, un 1,4-diazabicyclo[3.2.1]oct-4-yle, un 2-isopropylpipérazin-1-yle, un hexhydropyrrolo[1.2-a]pyrazin-2(1H)yle, un octahydro-2H-pyrido[1.2-a]pyrazin-2-yle, un 3- trifluorométhylpipérazin-1-yle, un 4-méthylsulfonylpipérazin-1-yle, un 4-(diméthylamino)pipéridin-1-yle ;
- un pipéridin-4-yle, un 1-méthylpipéridin-4-yle, un 1-méthylpipéridin-3-yle, un pipéridin-3-yle ;

- $R_2$ représente un atome de chlore, de fluor, de brome, un méthyle, un méthoxy ;
- $R_3$ représente un méthoxy ;
- $R_4$ représente un atome d'hydrogène, un atome de fluor, un méthyle, un méthoxy ;
- $R_5$ représente un atome d'hydrogène, un 3-chloro, un 5-fluoro, un 6-fluoro, un 5-méthoxycarbonyle, un 5-hydroxyméthyle ;
- $R_6$ représente un atome d'hydrogène, un méthyle, un isopropyle, un méthoxy, un éthoxy, un isopropoxy, un butyloxy, un *tert*-butyloxy, un cyclopentyloxy, un 1,1,2,2-tétrafluoroéthyloxy, un 2,2-diéthyluréido, un difluorométhoxy, un trifluorométhoxy, un hydroxy ;
- $R_7$ représente un atome d'hydrogène, un 3-méthyle, un 2-méthoxy, un 3-méthoxy, un 3-fluoro, un 3-chloro, un 3-$CF_3$ ;
- ou bien $R_7$ est en position -3- du phényle et ensemble avec $R_6$ ils constituent un radical triméthylène ;

à l'état de base ou de sel d'addition à un acide, ainsi qu'à l'état d'hydrate ou de solvat.

Plus particulièrement on préfère les composés de formule (I) dans laquelle :

- X représente un radical bivalent triméthylène ou pentaméthylène ;
- $R_1$ représente :
  - un diméthylamino, un 3-aminopyrrolidin-1-yle, un pipéridin-1-yle, un 4,4-difluoropipéridin-1-yle, un 4-hydroxypipéridin-1-yle, un 4-(diméthylamino)pipéridin-1-yle, un morpholin-4-yle, un 4-méthylpipérazin-1-yle, un pipérazin-1-yle, un 3,4-diméthylpipérazin-1-yle, un 5-méthyl-2,5-diazabicyclo[2.2.1]hept-2-yle, un 4-méthyl-1,4-diazepan-1-yle, un 8-méthyl-3,8-diazabicyclo[3.2.1]oct-3-yle, un 1,4-diazabicyclo[3.2.1]oct-4-yle ;
  - un pipéridin-4-yle ;
- $R_2$ représente un atome de chlore, de fluor, de brome ou un méthyle ;
- $R_3$ représente un méthoxy ;
- $R_4$ représente un atome d'hydrogène ou un atome de fluor ;
- $R_5$ représente un atome d'hydrogène, un 5-méthoxycarbonyle, un 5-hydroxyméthyle ;
- $R_6$ représente un atome d'hydrogène, un méthyle, un méthoxy, un isopropoxy, un éthoxy, un butyloxy, un 1,1,2,2-tétrafluoroéthyloxy, un 2,2-diéthyluréido, un difluorométhoxy, un trifluorométhoxy, un hydroxy ;
- $R_7$ représente un atome d'hydrogène, un 2-méthoxy, un 3-méthoxy, un 3-méthyle, un 3-fluoro ;

à l'état de base ou de sel d'addition à un acide, ainsi qu'à l'état d'hydrate ou de solvat.

Parmi les composés de formule (I) objets de l'invention, on peut notamment citer les composés suivants :

- 5-Chloro-1-[(2,4-diméthoxyphényl)sulfonyl]-3-(2-fluorophényl)-6-méthoxy-3-(3-pipéridin-4-ylpropyl)-1,3-dihydro-2*H* indol-2-one, isomère dextrogyre ;
- 5-Chloro-1 -[(2,4-diméthoxyphényl)sulfonyl]-3-(2-fluorophényl)-6-méthoxy-3-(3-pipéridin-4-ylpropyl)-1,3-dihydro-2*H*-indol-2-one, isomère lévogyre ;
- 5-Chloro-3-[3-(diméthylamino)propyl]-3-(2-fluorophényl)-1-[(4-isopropoxyphényl)sulfonyl]-6-méthoxy-1,3-dihydro-2*H* indol-2-one, isomère dextrogyre ;
- 3-{3-[(3*R*)-3-Aminopyrrolidin-1-yl]propyl}-5-chloro-3-(2-fluorophényl)-1-[(4-isopropoxyphényl)sulfonyll-6-méthoxy-1,3-dihydro-2*H*-indol-2-one, isomère dextrogyre ;
- 3-{3-[(3*S*)-3-Aminopyrrolidin-1-yl]propyl}-5-chloro-3-(2-fluorophényl)-1-[(4-isopropoxyphényl)sulfonyl]-6-méthoxy-1,3-dihydro-2*H*-indol-2-one, isomère dextrogyre ;
- 3-{3-[(3*R*)-3-Aminopyrrolidin-1-yl]propyl}-5-chloro-1-[(4-éthoxy-3-méthoxyphényl)sulfonyl]-3-(2-fluorophényl)-6-méthoxy-1,3-dihydro-2*H*-indol-2-one, isomère dextrogyre ;
- 3-{3-[(3*S*)-3-Aminopyrrolidin-1-yl]propyl}-5-chloro-1-[(4-éthoxy-3-méthoxyphényl)sulfonyl]-3-(2-fluorophényl)-6-méthoxy-1,3-dihydro-2*H*-indol-2-one, isomère dextrogyre ;

- 5-Chloro-1-[(2,4-diméthoxyphényl)sulfonyl]-3-(2-fluorophényl)-6-méthoxy-3-(3-pipéridin-1-ylpropyl)-1,3-dihydro-2*H*-indol-2-one, isomère dextrogyre ;
- 5-Chloro-3-[3-(4,4-difluoropipéridin-1-yl)propyl]-1-[(4-éthoxy-3-méthoxyphényl)sulfonyl]-3-(2-fluorophényl)-6-méthoxy-1,3-dihydro-2*H*-indol-2-one, isomère dextrogyre ;
- 5-Chloro-1-[(4-éthoxy-3-méthoxyphényl)sulfonyl]-3-(2-fluorophényl)-3-[3-(4-hydroxypipéridin-1-yl)propyl]-6-méthoxy-1,3-dihydro-2*H*-indol-2-one, isomère dextrogyre ;
- 5-Chloro-1-[(4-éthoxy-3-méthoxyphényl)sulfonyl]-3-(2-fluorophényl)-6-méthoxy-3-(3-morpholin-4-ylpropyl)-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-1-[(4-isopropoxyphényl)sulfonyl]-6-méthoxy-3-[3-(4-méthylpipérazin-1-yl)propyl]-1,3-dihydro-2*H*-indol-2-one, isomère dextrogyre ;
- 5-Chloro-1-[(4-éthoxy-3-méthoxyphényl)sulfonyl]-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-1-[(3,4-diméthoxyphényl)sulfonyl]-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-1-[(4-isopropoxyphényl)sulfonyl]-6-méthoxy-3-(3-pipérazin-1-ylpropyl)-1,3-dihydro-2*H*-indol-2-one, isomère dextrogyre ;
- 5-Chloro-1-[(4-éthoxy-3-méthoxyphényl)sulfonyl]-3-(2-fluorophényl)-6-méthoxy-3-(3-pipérazin-1-ylpropyl)-1,3-dihydro-2*H* indol-2-one, isomère dextrogyre ;
- 5-Chloro-1-[(3,4-diméthoxyphényl)sulfonyl]-3-(2-fluorophényl)-6-méthoxy-3-(3-pipérazin-1-ylpropyl)-1,3-dihydro-2*H* indol-2-one, isomère dextrogyre ;
- 5-Chloro-1-[(3,4-diméthylphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-6-méthoxy-1-[(4-méthoxy-3-méthylphényl)sulfonyl]-3-(3-pipérazin-1-ylpropyl)-1,3-dihydro-2*H*-indol-2-one ;
- 5-Chloro-1-[(4-éthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-1-[(4-éthoxy-3-méthylphényl)sulfonyl]-3-(2-fluorophényl)-6-méthoxy-3-(3-pipérazin-1-ylpropyl)-1,3-dihydro-2*H*-indol-2-one ;
- 5-Chloro-1-{[4-(difluorométhoxy)phényl]sulfonyl}-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-6-méthoxy-3-(3-pipérazin-1-ylpropyl)-1-{[4-(trifluorométhoxy)phényl]sulfonyl}-1,3-dihydro-2*H*-indol-2-one, isomère dextrogyre ;
- 5-Chloro-3-(2-fluorophényl)-6-méthoxy-1-(phénylsulfonyl)-3-(3-pipérazin-1-ylpropyl)-1,3-dihydro-2*H*-indol-2-one, isomère dextrogyre ;
- 5-Chloro-1-(2,3-dihydro-1*H*-inden-5-ylsulfonyl)-3-(2-fluoroph2nyl)-6-méthoxy-3-(3-pipérazin-1-ylpropyl)-1,3-dihydro-2*H*-indol-2-one, isomère dextrogyre;
- 5-Chloro-1-{[3-fluoro-4-(1-méthyléthoxy)phényl]sulfonyl}-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-6-méthoxy-3-[3-(4-méthylpipérazin-1-yl)propyl]-1-{[4-(1,1,2,2-tétrafluoroéthoxy)phényl]sulfonyl}-1,3-dihydro-2*H*-indol-2-one, isomère dextrogyre ;
- 3-(2-Fluorophenyl)-6-methoxy-5-methyl-1-{[4-(1-methylethoxy)phenyl]sulfonyl}-3-[3-(4-methylpiperazin-1-yl)propyl]-1,3-dihydro-2*H*-indol-2-one, isomère dextrogyre ;
- 5-Fluoro-3-(2-fluorophényl)-6-méthoxy-1-{[4-(1-méthyléthoxy)phényl]sulfonyl}-3-[3-(4-méthylpipérazin-1-yl)propyl]-1,3-dihydro-2*H*-indol-2-one, isomère dextrogyre ;
- 5-Bromo-3-(2-fluorophényl)-6-méthoxy-1-{[4-(1-méthyléthoxy)phényl]sulfonyl}-3-[3-(4-méthylpipérazin-1-yl)propyl]-1,3-dihydro-2*H*-indol-2-one, isomère dextrogyre ;
- 3-[4-({3-[3-(Diméthylamino)propyl]-3-(2-fluorophényl)-6-méthoxy-5-méthyl-2-oxo-2,3-dihydro-1*H*-indol-1-yl}sulfonyl)-2-fluorophényl]-1,1-diéthylurée, isomère dextrogyre ;
- 3-[4-({3-[3-(Diméthylamino)propyl]-3-(2-fluorophényl)-6-méthoxy-5-méthyl-2-oxo-2,3-dihydro-1*H*-indol-1-yl}sulfonyl)-2-méthylphényl]-1,1-diéthylurée, isomère dextrogyre ;
- 5-Chloro-3-(2-fluorophényl)-6-méthoxy-3-[3-(8-méthyl-3,8-diazabicyclo[3.2.1] oct-3-yl)propyl]-1-{[4-(1-méthyléthoxy)phényl]sulfonyl}-1,3-dihydro-2*H*-indol-2-one, isomère dextrogyre ;
- 5-Chloro-3-(2-fluorophényl)-6-méthoxy-3-[3-(5-méthyl-2,5-diazabicyclo[2.2.1] hept-2-yl)propyl]-1-{[4-(1-méthyléthoxy)phényl]sulfonyl}-1,3-dihydro-2*H*-indol-2-one, isomère dextrogyre ;

- 5-Chloro-3-(2-fluorophényl)-1-[(4-hydroxyphényl)sulfonyl]-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-6-méthoxy-3-[3-(4-méthyl-1,4-diazépan-1-yl)propyl]-1-{[4-(1-méthyléthoxy)phényl]sulfonyl}-1,3-dihydro-2*H*-indol-2-one, isomère dextrogyre ;
- 5-Chloro-3-(2-fluorophényl)-6-méthoxy-1-{[4-(1-méthyléthoxy)phényl]sulfonyl}-3-(5-pipéridin-1-ylpentyl)-1,3-dihydro-2*H*-indol-2-one, isomère dextrogyre ;
- 5-Chloro-3-[5-(diméthylamino)pentyl]-3-(2-fluorophényl)-6-méthoxy-1-{[4-(1-méthyléthoxy)phényl]sulfonyl}-1,3-dihydro-2*H*-indol-2-one, isomère dextrogyre ;
- 5-Chloro-3-{3-[(3*S*)-3,4-diméthylpipérazin-1-yl]propyl}-3-(2-fluorophényl)-6-méthoxy-1-{[4-(1-méthyléthoxy)phényl]sulfonyl}-1,3-dihydro-2*H*-indol-2-one, isomère dextrogyre ;
- 5-Chloro-6-méthoxy-1-{[4-(1-méthyléthoxy)phényl]sulfonyl}-3-[3-(4-méthylpipérazin-1-yl)propyl]-3-phényl-1,3-dihydro-2*H*-indol-2-one, isomère dextrogyre ;
- 5-Chloro-3-{3-[(5S)-1,4-diazabicyclo[3.2.1]oct-4-yl]propyl}-3-(2-fluorophényl)-6-méthoxy-1-{[4-(1-méthyléthoxy) phényl]sulfonyl}-1,3-dihydro-2*H*-indol-2-one, isomère dextrogyre ;
- 1-[(4-Butoxyphenyl)sulfonyl]-5-chloro-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-[3-[4-(diméthylamino)pipéridin-1-yl]propyl]-3-(2-fluorophényl)-1-[(4-isopropoxyphényl)sulfonyl]-6-méthoxy-1,3-dihydro-2*H*-indol-2-one ;
- 1-[(3-Fluoro-4-isopropoxyphényl)sulfonyl]-3-(2-fluorophényl)-5,6-diméthoxy-3-[3-(4-méthylpipérazin-1-yl)propyl]-1,3-dihydro-2*H*-indol-2-one ;
- 5-Fluoro-1-[(3-fluoro-4-isopropoxyphényl)sulfonyl]-3-(2-fluorophényl)-6-méthoxy-3-[3-(4-méthylpipérazin-1-yl)propyl]-1,3-dihydro-2*H*-indol-2-one ;
- 3-(2-Fluorophényl)-5,6-diméthoxy-1-[(4-méthoxy-3-méthylphényl)sulfonyl]-3-[3-(4-méthylpipérazin-1-yl)propyl]-1,3-dihydro-2*H*-indol-2-one ;
- 3-[5-Chloro-1-[(4-isopropoxyphényl)sulfonyl]-6-méthoxy-3-[3-(4-méthylpipérazin-1-yl)propyl]-2-oxo-2,3-dihydro-1*H*-indol-3-yl]-4-fluorobenzoate de méthyle ;
- 5-Chloro-3-[2-fluoro-5-(hydroxyméthyl)phényl]-1-[(4-isopropoxyphényl)sulfonyl]-6-méthoxy-3-[3-(4-méthylpipérazin-1-yl)propyl]-1,3-dihydro-2*H*-indol-2-one ;

à l'état de base ou de sels d'addition à des acides, ainsi qu'à l'état d'hydrates ou de solvates.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Group in Organic Synthesis », Green et al., 4th Edition, John Wiley & Sons, Inc., New York, 2007.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advanced Organic Chemistry », M. B. Smith and J. March, 6th Edition, Wiley Interscience, 2007, p.496-501.

Conformément à l'invention, on peut préparer les composés de formule (I) selon un procédé qui est caractérisé en ce que :

on fait réagir, en présence d'une base, un composé de formule :

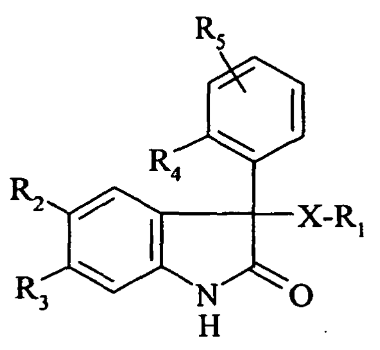

(II)

dans laquelle X, $R_1$, $R_2$, $R_3$, $R_4$ et $R_5$ sont tels que définis pour un composé de formule (I), avec un halogénure de sulfonyle de formule :

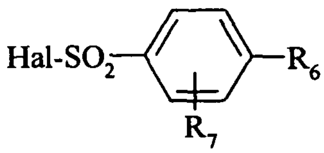

(III)

dans laquelle $R_6$ et $R_7$ sont tels que définis pour un composé de formule (I) et Hal représente un atome d'halogène.

Eventuellement, on transforme le composé de formule (I) en l'un de ses sels avec des acides minéraux ou organiques.

La réaction s'effectue en présence d'une base forte comme un hydrure métallique tel que l'hydrure de sodium ou un alcoolate alcalin tel que le tert-butylate de potassium, dans un solvant anhydre tel que le N,N-diméthylformamide ou le tétrahydrofurane et à une température comprise entre -70°C et +60°C. La réaction s'effectue de préférence en utilisant un composé de formule (III) dans laquelle Hal = Cl.

Selon une variante du procédé, on peut préparer les composés de formule (I) selon un procédé qui est caractérisé en ce que :

on fait réagir un composé de formule :

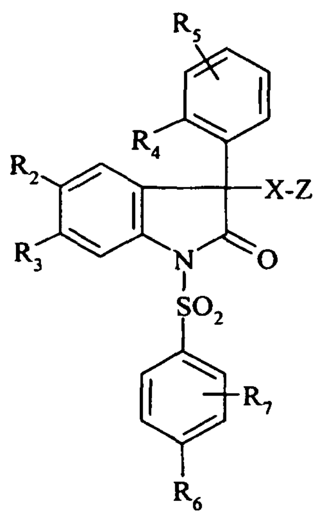

(IV)

dans laquelle X, $R_2$, $R_3$, $R_4$, $R_5$, $R_6$ et $R_7$ sont tels que définis pour un composé de formule (I) et Z représente un groupe partant tel qu'un atome d'halogène ou un groupe méthanesulfonate ou p-toluènesulfonate, avec un composé de formule :

$R_1$-H (V)

dans laquelle $R_1$ est tel que défini pour un composé de formule (I).

Eventuellement on transforme le composé de formule (I) en l'un de ses sels avec des acides minéraux ou organiques.

La réaction s'effectue en présence d'un carbonate de métal alcalin tel que le carbonate de sodium et en présence d'un halogénure de métal alcalin tel que l'iodure de sodium, dans un solvant tel que l'acétonitrile ou le N,N-diméthylformamide et à une température comprise entre la température ambiante et la température de reflux du solvant.

Les composés de formule (I) ainsi obtenus peuvent être ultérieurement séparés du milieu réactionnel et purifiés selon les méthodes classiques, par exemple par cristallisation ou chromatographie.

Les composés de formule (I) ainsi obtenus sont isolés sous forme de base libre ou de sel, selon les techniques classiques.

Les composés de formule (II) se préparent par réaction d'un composé 1,3-dihydro-2*H*-indol-2-one de formule :

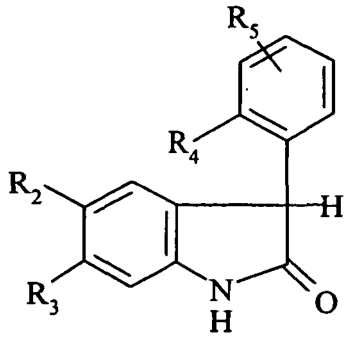

(VI)

dans laquelle $R_2$, $R_3$, $R_4$ et $R_5$ sont tels que définis pour un composé de formule (I) avec un composé de formule :

$Z\text{-}X\text{-}R_1$ (VII)

dans laquelle X et $R_1$ sont tels que définis pour un composé de formule (I) et Z représente un groupe partant tel qu'un atome d'halogène, de préférence l'iode ou le brome, ou un groupe méthanesulfonate ou p-toluène sulfonate.

La réaction s'effectue en présence d'une base forte comme un alcoolate alcalin tel que le *tert*-butylate de potassium dans un solvant tel que le tétrahydrofurane ou le N,N-diméthylformamide et à une température comprise entre -50°C et la température ambiante.

On peut également préparer les composés de formule (II) par réaction d'un composé de formule :

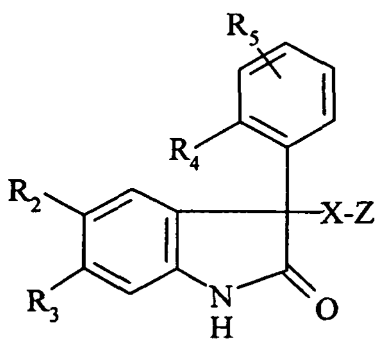

(VIII)

dans laquelle $R_2$, $R_3$, $R_4$ et $R_5$ sont tels que définis pour un composé de formule (I) et Z représente un groupe partant tel que précédemment décrit avec un composé de formule $R_1H$ (V). La réaction s'effectue selon les conditions opératoires décrites précédemment pour la réaction d'un composé de formule (IV) avec un composé de formule (V).

Les composés de formule (III) sont commerciaux, connus ou préparés par des méthodes connues telles que celles décrites dans EP 0 469 984 B et WO 95/18 105. Par exemple, les composés de formule (III) peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant tel qu'un hydrocarbure halogéné ou le N,N-diméthylformamide et à une température comprise entre -10°C et 200°C.

On peut également préparer les composés de formule (III) par réaction de l'acide chlorosulfonique sur un composé de formule :

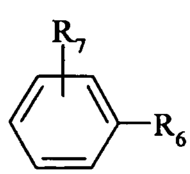

(IX)

dans laquelle $R_6$ et $R_7$ sont tels que définis pour un composé de formule (I). La réaction s'effectue selon les modes opératoires décrits dans Chlorosulfonic Acid ; R. J. Cremlyn ; The Royal Society of Chemistry, 2002.

Les composés de formule (IV) se préparent par réaction d'un composé de formule :

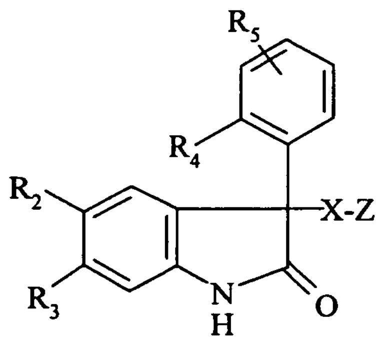

(VIII)

dans laquelle X, $R_2$, $R_3$, $R_4$ et $R_5$ sont tels que définis pour un composé de formule (I) et Z représente un groupe partant tel que précédemment décrit avec un composé de formule (III). La réaction s'effectue selon les conditions opératoires décrites précédemment pour la réaction d'un composé de formule (II) avec un composé de formule (III).

Les composés de formule (V) sont commerciaux, connus ou préparés selon des méthodes connues de l'homme de l'art.

Les composés de formule (VI) sont connus et se préparent selon des méthodes connues, telles que celles décrites dans WO 95/18 105 ou dans WO 01/55 130, qui utilisent la chimie d'isatines commerciales, connues ou préparées selon des méthodes connues telles que décritent par Katristzky et coll. dans « Advances in Heterocyclic Chemistry; Academic Press » 1975 ; Vol 18, pp 1-58. Les 3-hydroxyindolinones précurseurs directs des composés (VI) sont réduits selon des méthodes connues qui mettent en oeuvre des réducteurs tels que le triéthylsilane dans l'acide trifluoroacétique ou en présence d'acide de Lewis comme le complexe trifluorure de bore, diéthyl éther (Bioorg. Med. Chem. Lett. 175-178).

On peut également préparer les composés de formule (VI) selon le SCHEMA 1 ci-après dans lequel R représente un ($C_1$-$C_4$)alkyle.

SCHEMA 1

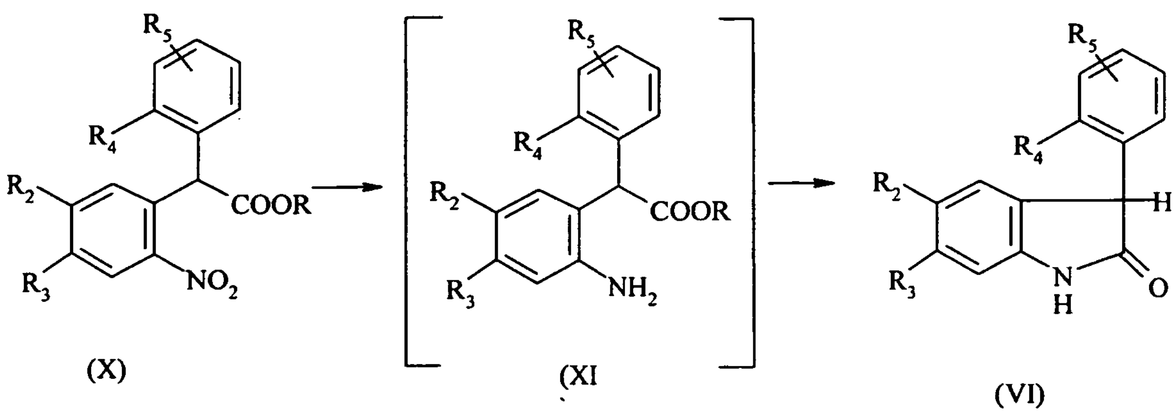

(X) (XI (VI)

Selon le SCHEMA 1, on obtient les composés de formule (VI) par cyclisation de l'amine de formule (XI) générée in situ par réduction du groupe nitro des composés de formule (X). La réaction s'effectue en présence d'un métal tel que l'étain ou le fer en milieu acide tel que l'acide acétique, dans un solvant tel que le méthanol et à une température comprise entre la température ambiante et 100°C.

Les composés de formule (VII) sont disponibles dans le commerce ou préparés selon des méthodes connues.

Les composés de formule (VIII) se préparent par réaction d'un composé de formule :

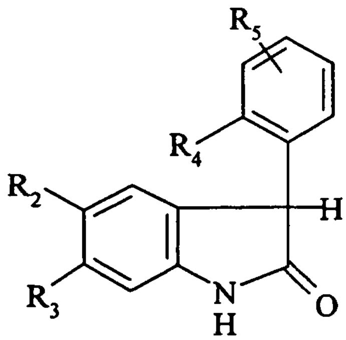

(VI)

dans laquelle $R_2$, $R_3$, $R_4$ et $R_5$ sont tels que définis pour un composé de formule (I) avec un composé de formule :

Hal-X-Z (XII)

dans laquelle X, est tel que défini pour un composé de formule (I), Z est tel que défini précédemment et Hal représente un atome d'halogène.

La réaction s'effectue en présence d'une base forte comme un alcoolate alcalin tel que le *tert*-butylate de potassium dans un solvant tel que le tétrahydrofurane ou le N,N-diméthylformamide et à une température comprise entre -50°C et la température ambiante.

Les composés de formule (IX) sont connus ou se préparent selon des méthodes connues.

Les composés de formule (X) se préparent par réaction de composés de formule :

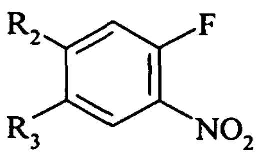

(XIII)

dans laquelle $R_2$ et $R_3$ sont tels que définis pour un composé de formule (I) avec un composé de formule :

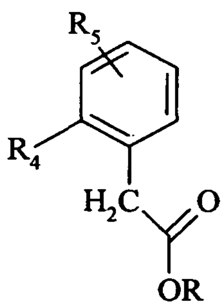

(XIV)

dans laquelle $R_4$ et $R_5$ sont tels que définis pour un composé de formule (I).

La réaction s'effectue en présence d'une base forte comme un alcoolate alcalin tel que le *tert*-butylate de potassium ou comme un hydrure métallique tel que l'hydrure de sodium, dans un solvant anhydre tel que le le N,N-diméthylformamide et à une température comprise entre -50°C et la température ambiante.

Les composés de formule (XII), (XIII) et (XIV) sont préparés selon des méthodes bien connues de l'homme du métier.

Les N-oxydes des composés comportant une amine sont préparés selon les méthodes connues de l'homme du métier par réaction de l'amine avec des peracides organiques tels que les acides peracétique, trifluoroperacétiques, performique, perbenzoïques ou ses dérivés tel l'acide 3-chloroperbenzoïque, à des températures comprises entre 0°C et 90°C, de préférence à des températures inférieures à 50°C.

Pour obtenir les composés de formule (I) sous forme d'isomères optiquement purs, on peut utiliser les techniques classiques de séparation : par exemple des recristallisations fractionnées d'un sel formé à partir de la base racémique avec un acide optiquement actif dont le principe est bien connu ou les techniques classiques de chromatographie

supercritique préparative sur phase chirale.

On peut également préparer les composés de formule (I) optiquement purs à partir d'un composé intermédiaire optiquement pur utile pour la préparation des composés de formule (I) selon les techniques décrites dans WO 03/008407.

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU (IV) ci-après, qui illustre les structures chimiques et les propriétés physiques de quelques composés selon l'invention.

Dans les Préparations et dans les Exemples on utilise les abréviations suivantes :

éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de $KHSO_4$ et 32,32 g de $K_2SO_4$ dans 1 litre d'eau.

Les spectres de résonance magnétique nucléaire du proton (RMN $^1H$) sont enregistrés dans le DMSO-$d_6$. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi, dd : doublet dédoublé.

Les pouvoirs rotatoires sont mesurés sur un polarimètre PERKIN-ELMER 241.

Les mélanges de solvants sont quantifiés en rapport volumétriques.

Les composés selon l'invention sont analysés par couplage LC/UV/MS (chromatographie liquide/détection UV/spectrométrie de masse). On mesure le pic moléculaire ($MH^+$) et le temps de rétention (tr) en minutes.

**Système 1 à pH3 : Méthode 1 (M1)**

Instrument (Agilent) : Chaine HPLC : série 1100 ;
Spectromètre de masse : MSD SL (Agilent)
Logiciel : Chemstation version B.01.03 de Agilent LC/UV
Colonne : Symmetry C18 3.5 µm (2.1x50mm) (Waters)
Température colonne : 25°C
Eluants : A : $H_2O$ + 0,005% TFA
B : $CH_3CN$ + 0,005% TFA
Débit : 0,4ml/min
Gradient :

| Temps (min.) | % (v/v) A/B |
|---|---|
| 0 | 100/0 |
| 10 | 10/90 |
| 15 | 10/90 |

Détection UV : 220 nm
Volume d'injection : 2 µl d'une solution à 0,5 mg/ml MS

Mode d'ionisation : Electrospray mode positif $ESI^+$
Gamme de masse : 90-1500 uma

**Système 2 à pH7 : Méthode 2 (M2)**

Instrument (Agilent) : Chaine HPLC : série 1100 ;
Spectromètre de masse : MSD SL (Agilent)
Logiciel : Chemstation version B.01.03 de Agilent LC/UV
Colonne : X Terra C18 3.5 µm (2.1x50mm) (Waters)
Température colonne : 30°C
Eluants : A : Tampon Acétate d'ammonium 10mM pH7
B : $CH_3CN$
Débit : 0,4ml/min
Gradient :

| Temps (min.) | % (v/v) A/B |
|---|---|
| 0 | 100/0 |
| 10 | 10/90 |
| 15 | 10/90 |

Détection UV : 220 nm
Volume d'injection : 2 µl d'une solution à 0,5 mg/ml MS
Mode d'ionisation : Electrospray mode positif $ESI^+$
Gamme de masse : 90-1500 uma

**Système 3 à pH2.2 : Méthode 3 (M3)**

Instrument (Waters) : Chaine HPLC : Alliance 2695 ;
Détecteur UV : PDA 996
Spectromètre de masse : Platform LCZ (Micromass)
Logiciel : MassLynx version 4.0 de Waters-Micromass LC/UV
Colonne : Symmetry C18 3.5 µm (2.1x50mm) (Waters)
Température colonne : 40°C
Eluants : A : $H_2O$ + 0,05% TFA
B : $CH_3CN$ + 0,035% TFA
Débit : 0,5ml/min
Gradient :

| Temps (min.) | % (v/v) A/B |
|---|---|
| 0,0 | 100/0 |
| 6,0 | 0/100 |
| 7,0 | 0/100 |
| 7,1 | 100/0 |
| 10,0 | 100/0 |

Détection UV : 220 nm
Volume d'injection : 2 µl d'une solution à 0,5 mg/ml MS
Mode d'ionisation : Electrospray mode positif $ESI^+$
Gamme de masse : 120-1500 uma

**Système 4 à pH3 : Méthode 4 (M4)**

Instrument (Agilent) : Chaine HPLC : série 1100 ;
Spectromètre de masse : MSD SL (Agilent)
Logiciel : Chemstation version B.01.03 de Agilent LC/UV
Colonne : Symmetry C 18 3.5 μm (2.1x50mm) (Waters)
Température colonne : 25˚C
Eluants : A : $H_2O$ + 0,005% TFA
B : $CH_3CN$ + 0,005% TFA
Débit : 0,4ml/min
Gradient :

| Temps (min.) | % (v/v) A/B |
|---|---|
| 0 | 100/0 |
| 10 | 0/100 |
| 15 | 0/100 |

Détection UV : 220 nm
Volume d'injection : 2 μl d'une solution à 0,5 mg/ml MS
Mode d'ionisation : Electrospray mode positif $ESI^+$
Gamme de masse : 90-1500 uma

**Système 5 à pH3 : Méthode 5 (M5)**

Instrument (Agilent) : Chaine HPLC : série 1100 ;
Spectromètre de masse : MSD SL (Agilent)
Logiciel : Chemstation version B.01.03 de Agilent LC/UV
Colonne : Symmetry C 18 3.5 μm (2.1x50mm) (Waters)
Température colonne : 25˚C
Eluants : A : $H_2O$ + 0,005% TFA
B : $CH_3CN$ + 0,005% TFA
Débit : 0,4ml/min
Gradient :

| Temps (min.) | % (v/v) A/B |
|---|---|
| 0 | 100/0 |
| 30 | 0/100 |
| 35 | 0/100 |

Détection UV : 220 nm
Volume d'injection : 2 μl d'une solution à 0,5 mg/ml MS
Mode d'ionisation : Electrospray mode positif $ESI^+$
Gamme de masse : 90-1500 uma

**Système 6 à pH2.2 : Méthode 6 (M6)**

Instrument (Waters) : Chaine HPLC : Alliance 2695 ;
Détecteur UV : PDA 996
Spectromètre de masse : ZQ (Micromass)
Logiciel : MassLynx version 4.1 de Waters-Micromass LC/UV

Phenomenex Luna C18(2)-HST 2,5 µm 2.0 x 30 mm
Température colonne : 50˚C
Eluants : A : $H_2O$ + 0,05% TFA
B : $CH_3CN$ + 0,035% TFA
Débit : 1 ml/min
Gradient :

| Temps (min.) | % (v/v) AB |
|---|---|
| 0,0 | 100/0 |
| 2.5 | 0/100 |
| 3.5 | 0/100 |
| 3.6 | 100/0 |
| 5 | 100/0 |

Détection UV : 220 nm
Volume d'injection : 2 µl d'une solution à 0,5 mg/ml MS
Mode d'ionisation : Electrospray mode positif $ESI^+$
Gamme de masse : 120-1500 uma

PREPARATIONS

1. Préparations des composés de formule (VI).

Préparation 1.1

5-Chloro-3-(2-chlorophényl)-6-méthoxy-1,3-dihydro-2*H*-indol-2-one.

(VI) : $R_2$ = Cl ; $R_3$ = OMe ; $R_4$ = Cl ; $R_5$ = H

A- (5-Chloro-4-méthoxy-2-nitrophényl)(2-chlorophényl)acétate de méthyle.

On refroidit à -5˚C une suspension de 0,6 g d'hydrure de sodium à 60% dans l'huile dans 10 ml de DMF, ajoute goutte à goutte une solution de 1,03 g de 1-chloro-5-fluoro-2-méthoxy-4-nitrobenzène (préparé selon EP 061 741) et 0,93 g de (2-chlorophényl)acétate de méthyle dans 25 ml de DMF et laisse 1 heure sous agitation en laissant remonter la température à 3˚C. On ajoute une solution saturée de $NH_4Cl$, extrait à l'AcOEt, lave la phase organique à l'eau, sèche sur $Na_2SO_4$ et évapore les solvants sous vide. On chromatographie le résidu sur gel de silice en éluant par le mélange cyclohexane/AcOEt (97/3 ; v/v). On obtient le composé attendu sous forme liquide.

B-5-Chloro-3-(2-chlorophényl)-6-méthoxy-1,3-dihydro-2*H*-indol-2-one.

A un mélange de 1,1 g du composé de l'étape précédente dans 15 ml de MeOH on ajoute 0,8 g de fer puis 3,7 ml d'AcOH et chauffe à reflux pendant 16 heures. On concentre partiellement les solvants sous vide, ajoute une solution de $NaHCO_3$ à 5% puis de l'AcOEt et laisse sous agitation à TA. On filtre le mélange réactionnel, décante le filtrat, lave la phase organique par une solution de $NaHCO_3$ à 5%, sèche sur $Na_2SO_4$ et évapore le solvant sous vide. On reprend le résidu dans l'éther iso, essore le précipité formé et le sèche à 50 ˚C sous vide. On obtient le composé attendu, F = 249˚C.

Préparation 1.2

5-Chloro-3-(2-fluorophényl)-6-méthoxy-1,3-dihydro-2*H*-indol-2-one.

(VI) : $R_2$ = Cl ; $R_3$ = OMe ; $R_4$ = F ; $R_5$ = H

A- (5-Chloro-4-méthoxy-2-nitrophényl)(2-fluorophényl)acétate de méthyle.

On refroidit à -20°C une suspension de 2,92 g d'hydrure de sodium à 60% dans l'huile dans 50 ml de DMF, ajoute goutte à goutte une solution de 5 g de 1-chloro-5-fluoro-2-méthoxy-4-nitrobenzène (préparé selon EP 061 741) et de 4,5 g de (2-fluorophényl)acétate de méthyle dans 60 ml de DMF et laisse 1 heure sous agitation en laissant remonter la température à 3°C. On ajoute une solution saturée de $NH_4Cl$, extrait le mélange réactionnel à l'AcOEt, lave la phase organique à l'eau, sèche sur $Na_2SO_4$ et évapore les solvants sous vide. On chromatographie le résidu sur gel de silice en éluant par le mélange cyclohexane/AcOEt (97/3 ; v/v). On obtient le composé attendu sous forme solide, F = 87°C.
RMN$^1$H : DMSO-d6 (250 MHz) : δ (ppm) : 3,70 : s : 3H ; 4,01 : s : 3H ; 5,78 : s : 1H ;

B-5-Chloro-3-(2-fluorophényl)-6-méthoxy-1,3-dihydro-2*H*-indol-2-one.

A un mélange de 1,1 g du composé de l'étape précédente dans 15 ml de MeOH on ajoute 0,8 g de fer puis 3,7 ml d'AcOH et chauffe à reflux pendant 16 heures. On concentre partiellement les solvants sous vide, ajoute une solution de $NaHCO_3$ à 5% puis de l'AcOEt et laisse sous agitation à TA. On filtre le mélange réactionnel, décante le filtrat, lave la phase organique par une solution de $NaHCO_3$ à 5%, sèche sur $Na_2SO_4$ et évapore le solvant sous vide. On reprend le résidu dans l'éther iso, essore le précipité formé et le sèche à 50 °C sous vide. On obtient le composé attendu, F = 214°C.

RMN$^1$H : DMSO-d6 (250 MHz) : δ (ppm) : 3,86 : s : 3H ; 4,99 : s : 1H ; 6,66 : s : 1H ; 6,96 : s : 1H ; 7,14-7,43 : mt : 4H ; 10,71 : s : 1H.

Selon les modes opératoires décrits dans les Préparations ci-dessus, à partir d'orthofluoronitrobenzènes (XIII) et de phénylacétates d'alkyle (XIV) appropriés et connus, on prépare les composés (VI), rassemblés dans le tableau I ci-après :

TABLEAU I

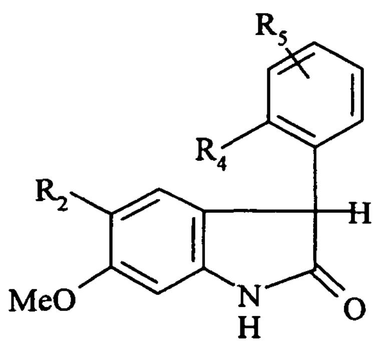

(VI)

$R_3$ = OMe

| Préparations | $R_2$ | $R_4$ | $R_5$ | F°C<br>$MH^+$ ; tr (mn)<br>(Conditions) |
|---|---|---|---|---|
| 1.3 | Cl | Me | H | -<br>288 ; 7,73<br>(M 4) |
| 1.4 | Cl | F | 6-F | -<br>310 ; 8,02 mn<br>(M 4) |

(suite)

| Préparations | $R_2$ | $R_4$ | $R_5$ | F°C<br>$MH^+$ ; tr (mn)<br>(Conditions) |
|---|---|---|---|---|
| 1.5 | Br | F | H | -<br>337 ; 2,24<br>(M 6) |
| 1.5a | Cl | F | 5-$CO_2Me$ | 220<br>350 ; 7,95<br>(M4) |

Préparation 1.6

5-fluoro-3-(2-fluorophényl)-6-méthoxy-1,3-dihydro-2*H*-indol-2-one.

(VI) : $R_2$ = F ; $R_3$ = OMe ; $R_4$ = F ; $R_5$ = H

A- 5-Fluoro-6-méthoxyisatine :

A1. N-(4-fluoro-3-méthoxyphényl)-2-(hydroxyimino)éthanamide :

A la suspension de 14,7 g de 4-fluoro-3-méthoxyaniline, dans 695 ml d'eau et 35 ml d'acide chlorhydrique 2N, on ajoute dans l'ordre 118,4 g de sulfate de sodium, 26,3 g de chlorure d'hydroxylamine et 20,89 g d'hydrate de chloral. On chauffe le mélange réactionnel à 55° C pendant 6 heures. On refroidit vers 15° C. On obtient le composé attendu qui est filtré, lavé et séché.
$MH^+$ = 213 ; tr = 5,94 mn ; (M 4).

A2. 5-Fluoro-6-méthoxyisatine :

A 125 ml d'acide sulfurique concentré chauffé vers 50° C, on ajoute, lentement et par portion, 19,43 g du composé obtenu à l'étape précédente. On chauffe vers 60° C pendant 20 minutes avant de refroidir puis de couler sur 2l d'eau à la température de 45° C. On laisse refroidir vers 20° C, sous agitation pendant une nuit. On obtient le composé attendu qui est filtré, lavé et séché.
F = 260° C ; $MH^+$ = 196 ; tr = 1,52 mn ; (M 6).

B-5-Fluoro-3-(2-fluorophényl)-3-hydroxy-6-méthoxy-1,3-dihydro-2H-indol-2-one :

A 76,5ml d'une solution à 15 % du complexe chlorure d'isopropylmagnésium, chlorure de magnésium dans le THF, refroidie à 0° C, on ajoute lentement 13,45 g de 2-bromo-fluorobenzène. Après 1 heure d'agitation à 0° C, on introduit lentement, le mélange de 6 g du composé obtenu à l'étape précédente dans 50 ml de THF. On laisse ensuite la température remonter à 20° C pendant la nuit. On coule le milieu sur 20 ml d'acide chlorhydrique 1N et extrait avec 150 ml d'AcOEt. On filtre sur buchner et laisse décanter, la phase organique est séchée sur $Na_2SO_4$ et évaporée à sec. Le résidu solide est repris par de l'éther isopropylique. On obtient le composé attendu par filtration et séchage.
$MH^+$ = 292 ; tr = 4,31 mn ; (M 3).

C- 5-Fluoro-3-(2-fluorophényl)- 6-méthoxy-1,3-dihydro-2*H*-indol-2-one :

A 11 g du composé obtenu à l'étape précédente dans 127 ml de 1,2-dichloroéthane, on ajoute 31,6ml de triéthylsilane puis 2,53 ml de trifluoroborane, diéthyléther. On chauffe à 75° C pendant 1 heure 30 minutes. On refroidit à 20° C, évapore sous pression réduite et reprend le résidu avec 20 ml d'AcOEt. On obtient le composé attendu par filtration et séchage.
$MH^+$ = 276 ; tr = 4,73 mn ; (M 3).
Selon le mode opératoire décrit dans la Préparation ci-dessus, à partir d'isatines (préparées comme décrit précédemment) et de phénylmagnésiens (commerciaux ou préparés comme précédemment ou plus classiquement avec des copeaux de magnésium) appropriés, on obtient les composés (VI), rassemblés dans le tableau II ci-après :

TABLEAU II

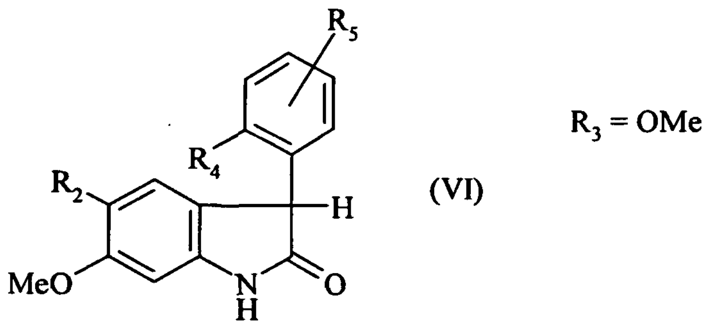

| Préparations | $R_2$ | $R_4$ | $R_5$ | F°C MH+ ; tr (mn) (Conditions) |
|---|---|---|---|---|
| 1.7 | Cl | F | 5-F | 225<br>310 ; 7,52<br>(M 4) |
| 1.8 | Cl | H | H | 248<br>274 ; 7,43<br>(M 4) |
| 1.9 | Cl | F | 3-Cl | 225<br>326 ; 7,98<br>(M 4) |
| 1.10 | Me | F | H | -<br>272 ; 5,02<br>(M 4) |
| 1.11 | OMe | F | H | -<br>288 ; 4,17<br>(M 4) |

2a. Préparations de composés de formule (VIII)

Préparation 2a.1

5-Chloro-3-(4-chlorobutyl)-3-(2-chlorophényl)-6-méthoxy-1,3-dihydro-2*H*-indol-2-one.

(VIII) $R_2$ = Cl ; $R_3$ = OMe; $R_4$ = Cl; $R_5$ = H; X = $-(CH_2)_4-$ ; Z = Cl

On refroidit à -50°C, sous atmosphère d'argon, une solution de 0,46 g du composé de la Préparation 1.1 dans 11 ml de DMF, ajoute 0,19 g de *tert*-butylate de potassium et laisse sous agitation en laissant remonter la température à -20°C. On refroidit le mélange réactionnel à -50°C, ajoute goutte à goutte une solution de 0,19 ml de 1-bromo-4-chlorobutane dans 5 ml de DMF et laisse 16 heures sous agitation en laissant remonter la température à 10°C. On ajoute une solution saturée de $NH_4Cl$, extrait à l'AcOEt, lave la phase organique à l'eau, sèche sur $Na_2SO_4$ et évapore le solvant sous vide. On chromatographie le résidu sur gel de silice en éluant par le gradient du mélange cyclohexane/AcOEt de (100/0 ; v/v) à (60/40 ; v/v). On obtient le composé attendu, F = 80°C.

Préparation 2a.2

5-Chloro-3-(3-chloropropyl)-3-(2-fluorophényl)-6-méthoxy-1,3-dihydro-2*H*-indol-2-one, isomère dextrogyre et isomère lévogyre.

(VIII) : $R_2$ = Cl ; $R_3$ = OMe ; $R_4$ = F ; $R_5$ = H ; X = $-(CH_2)_3-$ ; Z = Cl

On refroidit à -50˚C sous atmosphère d'argon, une solution de 6g du composé de la Préparation 1.2 dans 200 ml de DMF, ajoute 2,65 g de tert-butylate de potassium et laisse sous agitation en laissant remonter la température à -20˚C. On refroidit le mélange réactionnel à -50˚C, ajoute goutte à goutte 2,29 ml de 1-chloro-3-iodopropane et laisse 16 heures sous agitation en laissant remonter la température à 15˚C. On ajoute une solution saturée de $NH_4Cl$, extrait à l'AcOEt, lave la phase organique à l'eau, sèche sur $Na_2SO_4$ et évapore le solvant sous vide. On reprend le résidu à l'éther iso et essore le précipité formé. On obtient le composé attendu, F = 179˚C.

Les énantiomères du composé ainsi obtenu sont séparés par chromatographie liquide sur phase chirale dans les conditions suivantes :

Appareillage : Système de chromatographie Waters Delta Prep 4000.
Colonne chirale : CHIRALPAK AS-VCSP :
Phase mobile : acétonitrile 100 % ;
Débit: 120 ml/minute ;
Pression : 50 bar ;
Détection UV : 254 nm ;

Après séparation des énantiomères on obtient :

- l'isomère dextrogyre :

$$\alpha_D^{20} = +79° \text{ (c = 1 ; AcOEt) ;}$$

RMN[1]H : DMSO-d6 (250 MHz) : δ (ppm) : 1,21-1,43: mt : 1H ; 1,43-1,65 : mt : 1H ; 2,16-2,47 : mt : 2H ; 3,61 : t : 2H ; 3,861 : s : 3H ; 6,664 : s : 1H ; 6,96 : s : 1H ; 7,03-7,136 : mt : 1H ; 7,23-7,41 : mt : 2H ; 7,57-7,66 : mt : 1H ; 10,80 : s : 1H.

- l'isomère lévogyre :

$$\alpha_D^{20} = -77° \text{ (c = 1 ; AcOEt).}$$

Dans des conditions semblables, à partir de composés précédemment décrits de formule (VI) et de composés de formule (XII) appropriés, on obtient les composés de formule (VIII), racémates ou chiraux, rassemblés dans le tableau III ci-après :

TABLEAU III

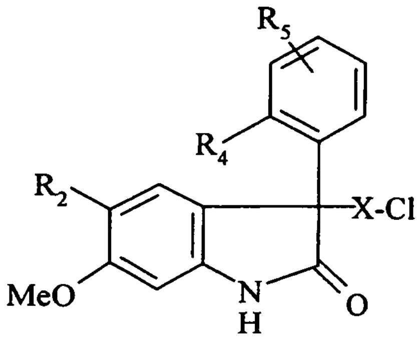

(VIII)

Z = Cl
$R_3$ = OMe

| Préparation | X | $R_2$ | $R_4$ | $R_5$ | $\alpha_D^{20}$ (c ; solvant) (c = 1, solvant) | F°C MH+; tr (mn) (Conditions) |
|---|---|---|---|---|---|---|
| 2a.3 | $(CH_2)3$ | Cl | Me | H | +71.8° (AcOEt) | 84<br>364 ; 9,11<br>(M 4) |
| 2a.4 | $(CH_2)_3$ | Cl | F | 6-F | +117.3° (AcOEt) | 90<br>386 ; 8,48<br>(M 4) |
| 2a.5 | $(CH_2)_3$ | Cl | F | 4-F | +57.8° (AcOEt) | 138<br>386 ; 8,50<br>(M 4) |
| 2a.6 | $(CH_2)_3$ | Br | F | H | Racémate | -<br>412 ; 5,66<br>(M 3) |
| 2a.7 | $(CH_2)_3$ | F | F | H | +115.3° (AcOEt) | -<br>351 ; 7,88<br>(M 4) |
| 2a.8 | $(CH_2)_3$ | Cl | F | 5-F | +82° (AcOEt) | -<br>386 ; 8,92<br>(M 4) |
| 2a.9 | $(CH_2)_3$ | Cl | H | H | Racémate | 200<br>350 ; 8,44<br>(M 4) |
| 2a.10 | $(CH_2)_3$ | Cl | F | 3-Cl | + 95° (AcOEt) | -<br>402 ; 9,38<br>(M 4) |
| 2a.11 | $(CH_2)_3$ | Me | F | H | +84.9° (AcOEt) | -<br>348 ; 8,30<br>(M 4) |
| 2a.12 | $(CH_2)_3$ | OMe | F | H | +86.6° (AcOEt) | -<br>364 ; 7,64<br>(M 4) |
| 2a.13 | $(CH_2)_4$ | Cl | F | H | racémate | -<br>382 ; 9,05<br>(M 4) |

(suite)

| Préparation | X | $R_2$ | $R_4$ | $R_5$ | $\alpha_D^{20}$ (c ; solvant) (c = 1, solvant) | F°C MH+; tr (mn) (Conditions) |
|---|---|---|---|---|---|---|
| 2a.14 | $(CH_2)_5$ | Cl | F | H | +66°C (c = 1, MeOH) | - 396 ; 9,45 (M 4) |
| 2a.15 | $(CH_2)_2$ | Cl | F | H | racémate | - 354 ; 8,69 (M 4) |
| 2a.16 | $(CH_2)_3$ | Cl | F | 5-$CO_2$Me | +132.4°C (c = 0,5 ; AcOEt) | - 426 ; 8,78 (M4) |

2. Préparations des composés de formule (II).

Préparation 2.1

4-[3-[5-Chloro-3-(2-fluorophényl)-6-méthoxy-2-oxo-2,3-dihydro-1*H*-indol-3-yl]propyl]pipéridine-1-carboxylatede*tert*-butyle.

(II):

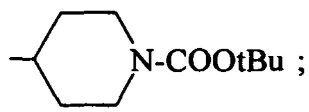

$R_2$ = Cl ; $R_3$ = OMe ; $R_4$ = F ; $R_5$ = H ; X = $-(CH_2)_3-$

On refroidit à -40°C sous atmosphère d'argon, une solution de 2,5 g du composé de la Préparation 1.2 dans 60 ml de DMF, ajoute 1,09 g de *tert*-butylate de potassium et laisse sous agitation en laissant remonter la température à -15°C. On refroidit le mélange réactionnel à -50°C, ajoute goutte à goutte, une solution de 3,28 g de 4-(3-iodopropyl) pipéridine-1-carboxylate de *tert*-butyle (préparé selon J. Med. Chem., 1994, 37(16), 2537-2551) dans 40 ml de DMF et laisse 16 heures sous agitation en laissant remonter la température à 12°C. On ajoute une solution saturée de $NH_4Cl$, extrait à l'AcOEt, lave la phase organique à l'eau, sèche sur $Na_2SO_4$ et évapore le solvant sous vide. On chromatographie le résidu sur gel de silice en éluant par le gradient du mélange cyclohexane/AcOEt de (95/5 ; v/v) à (70/30 ; v/v). On obtient le composé attendu, F = 203°C.

RMN$^1$H : DMSO-d6 (250 MHz) : $\delta$ (ppm) : 0,73-0,96 : mt : 3H ; 0,99-1,22 : mt : 4H ; 1,36 : s : 9H ; 1,40-1,54 : mt : 2H ; 1,99-2,17 : mt : 1H ; 2,18-2,36 : mt : 1H ; 2,53-2,72 : mt : 2H ; 3,76-3,95 : mt : 5H ; 6,64 : s : 1H ; 6,94 : s : 1H ; 7,0-7,12 : mt : 1H ; 7,20-7,40 : mt : 2H ; 7,58-7,68 : mt : 1H ; 10,73 : mt : 1H.

Les énantiomères du composé ainsi obtenu sont séparés par chromatographie supercritique sur phase chirale dans les conditions suivantes :

Appareillage : Système de chromatographie supercritique Berger Prep SFC
Colonne chirale : Chiralpak IC 5 µm
Phase mobile : $CO_2$ /MeOH (65/ 35 ; v/v)
Débit : 50 ml/minute ;
Pression : 100 bar ;
Détection UV : 220 nm ;

Après séparation des énantiomères on obtient :

- l'isomère dextrogyre : MH+ = 517 ; tr = 10,2 mn ; (M 4) (c = 1 ; MeOH);

$$\alpha_D^{20} = +53° \text{ (c = 1 ; MeOH) ;}$$

- l'isomère lévogyre : $MH^+$ = 517 ; tr = 10,3 mn ; (M 4)

$$\alpha_D^{20} = -48° \text{ (c = 1 ; MeOH).}$$

Selon le mode opératoire décrit à la Préparation 2.1, à partir d'un composé de formule (VI) et des composés de formule (VII) appropriés connus, on prépare les composés de formule (II) isomères dextrogyres, rassemblés dans le TABLEAU IV ci-après :

TABLEAU IV

| 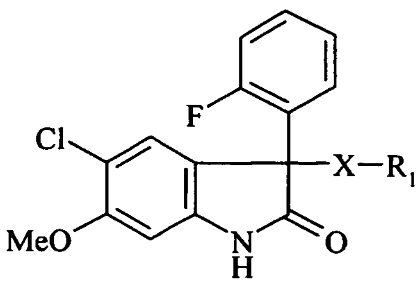 | | | (II) : $R_2$ = Cl<br>$R_3$ = OMe<br>$R_4$ = F<br>$R_5$ = H |
|---|---|---|---|
| Préparation | X | $R_1$ | $MH^+$ ; tr (mn)<br>Conditions<br>$\alpha_D^{20}$ (c; solvant) |
| 2.1.1 | $(CH_2)_2$ | 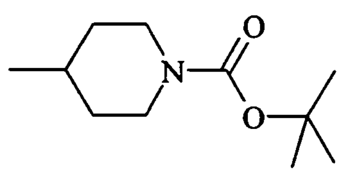 | 503 ; 6,1<br>(M 4)<br>+ 43° (c = 1, MeOH) |
| 2.1.2 | $CH_2$ | 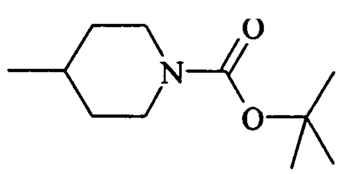 | 489 ; 9,47<br>(M 4)<br>+ 45° (c = 1, MeOH) |
| 2.1.3 | $CH_2$ | 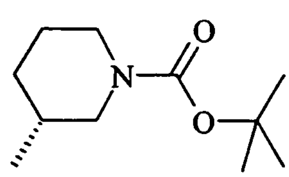 | 489 ; 9,68<br>(M 4)<br>+ 86° (c = 0,5, MeOH) |

(suite)

| Préparation | X | $R_1$ | $MH^+$ ; tr (mn) Conditions $\alpha_D^{20}$ (c; solvant) |
|---|---|---|---|
| 2.1.4 Diastéréoisomère1 | $(CH_2)_3$ | 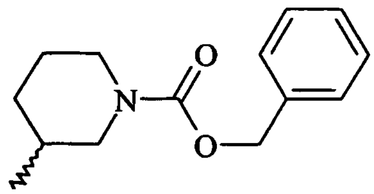 | 551 ; 9,70 (M 4) + 36° (c = 0,25, MeOH) |
| 2.1.5 Diastéréoisomère2 | $(CH_2)_3$ | 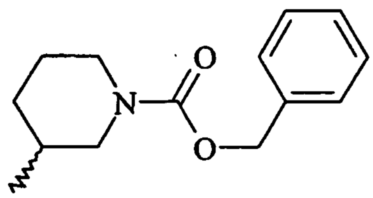 | 551 ; 9,73 (M 4) + 32° (c = 0,25, MeOH) |

Préparation 2.2

5-Chloro-3-(2-chlorophényl)-3-[4-(diméthylamino)butyl]-6-méthoxy-1,3-dihydro-2*H*-indol-2-one.

(II) : $R_1$ = -N(Me)$_2$ ; $R_2$ = Cl ; $R_3$ = OMe ; $R_4$ = Cl ; $R_5$ = H ; X = $-(CH_2)_4-$

On chauffe à 50° C pendant 48 heures, dans un récipient fermé, un mélange de 0,3 g du composé de la Préparation 2a.1, 8 ml d'une solution 2 M de diméthylamine dans le THF et 2 ml d'une solution aqueuse de diméthylamine à 40 %, 0,5 g de $Na_2CO_3$ et 0,4 g de NaI dans 10 ml d'acétonitrile. On refroidit le mélange réactionnel à 15° C, ajoute de l'eau, extrait à l'AcOEt, lave la phase organique à l'eau, sèche sur $Na_2SO_4$ et évapore le solvant sous vide. On chromatographie le résidu sur gel de silice en éluant par le gradient du mélange DCM/MeOH de (100/0 ; v/v) à (80/20 ; v/v). On obtient le composé attendu, F = 86°C.

Préparation 2.2.1

5-Chloro-3-phényl-3-[3-(4-*tert*-butyloxycarbonylpipérazin-1-yl)propyl]-6-méthoxy-1,3-dihydro-2*H*-indol-2-one.

(II): $R_1$=

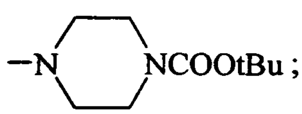

$R_2$ = Cl ; $R_3$ = OMe ; $R_4$ = H ; $R_5$ = H ; X = $-(CH_2)_3-$

A 3,7g du composé de la Préparation 2a.9 dans 45 ml de DMF, on ajoute 5,9 g de 4-tert-butoxycarbonylpipérazine, 1,68 g de carbonate de sodium et 1,58 g d'iodure de sodium. On chauffe 3 heures sous agitation, refroidit, coule le milieu sur 450 ml d'eau et extrait avec de l'AcOEt. On évapore à sec la phase organique et purifie par chromatographie sur silice, en éluant par le gradient du mélange DCM/MeOH de (100/0 ; v/v) à (90/10 ; v/v). On obtient le composé attendu, F = 72° C.

Les énantiomères du composé ainsi obtenu sont séparés par chromatographie supercritique sur phase chirale dans des conditions proches de l'exemple 1.

On obtient le composé 2.2.1 énantiomère lévogyre :

$$\alpha_D^{20} = -75.1° \text{ ( AcOEt) ;}$$

MH⁺ = 500 ; tr = 5,75 mn ; (M 4)

et le composé 2.2.1 énantiomère dextrogyre :

$$\alpha_D^{20} = +70.3° \text{ ( AcOEt) ;}$$

$MH^+$ = 500 ; tr = 5,83 ; (M 4)

Selon le mode opératoire décrit à la Préparation 2.2.1, à partir de composés précédemment décrits de formule (VIII) et de composés de formule (V) appropriés, on obtient les composés de formule (II), rassemblés dans le tableau V suivant :

TABLEAU V

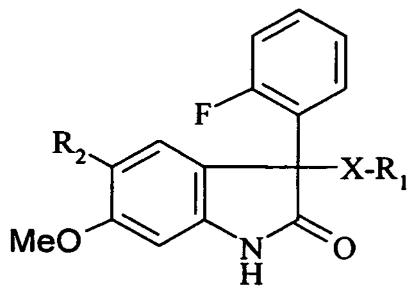

(II) : $R_3$ = OMe
$R_4$ = F
$R_5$ = H

| Préparation | Composé précurseur (VIII) | $R_2$ | X | $R_1$ | $MH^+$; tr (mn) (Conditions) $\alpha_D^{20}$ (c ; solvant) |
|---|---|---|---|---|---|
| 2.2.2 | 2a.6 | Br | $(CH_2)_3$ | 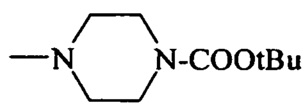 | 562 ; 4,42 (M 3) + 40 °(c = 1, MeOH) |
| 2.2.3 | 2a.13 | Cl | $(CH_2)_4$ | 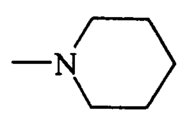 | 431 ; 5,12 (M 4) + 55 °(c = 1, MeOH) |

Préparation 2.3

5-Chloro-3-(2-fluorophényl)-3-[3-(diméthylamino)propyl]-6-méthoxy-1,3-dihydro-2*H*-indol-2-one, isomère unique.

(II) : $R_1$ = -N(Me)$_2$ ; $R_2$ = Cl ; $R_3$ = Ome ; $R_4$ = F ; $R_5$ = H ; X = -$(CH_2)_3$-.

Dans un récipient fermé, on chauffe à 50°C pendant 48 heures un mélange de 0,59 g du composé dextrogyre obtenu dans la Préparation 2a.2, 20 ml d'une solution 2M de diméthylamine dans le THF, 0,5 g de carbonate de sodium, 0,4 g d'iodure de sodium dans 10 ml d'acétonitrile. On refroidit le mélange réactionnel à 15°C, ajoute de l'eau, extrait à l'AcOEt, lave la phase organique à l'eau, sèche sur $Na_2SO_4$ et évapore le solvant sous vide. On chromatographie le

résidu sur gel de silice en éluant par le gradient du mélange DCM/MeOH de (100/0 ; v/v) jusqu'à (80/20; v/v). On obtient le composé attendu sous forme de résine blanche.

$MH^+$ = 377 ; tr = 5,66 mn ; (M 1).

RMN$^1$H : DMSO-d6 (400 MHz) : δ (ppm) : 0,88-1,00 : mt : 1H ; 1,17-1,28 : mt : 1H ; 2,01 : s : 6H ; 1,60-1,85 : mt : 4H ; 3,86 : s : 3H ; 6,66 : s : 1H ; 6,94 : s : 1H ; 7,05-7,40 : mt : 3H ; 7,60-7,66 : mt : 1H ; 10,77 : s : 1H.

Préparation 2.4

5-Chloro-3-(2-fluorophényl)-6-méthoxy-3-[3-(4-méthylpipérazin-1-yl)propyl]-1,3-dihydro-2*H*-indol-2-one, isomère unique.

(II) :

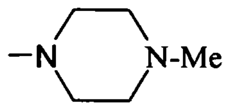 ;

$R_2$ = Cl ; $R_3$ = OMe ; $R_4$ = F ; $R_5$ = H ; X = $-(CH_2)_3-$

On chauffe à 100°C pendant 3 heures un mélange de 0,15 g du composé dextrogyre obtenu dans la préparation 2a.2 , 0,14 ml de 1-méthylpipérazine, 0,05 g de $Na_2CO_3$ et 0,067 g de NaI dans 2 ml de DMF. On refroidit le mélange réactionnel à 15°C, ajoute de l'eau, extrait à l'AcOEt, lave la phase organique à l'eau, sèche sur $Na_2SO_4$ et évapore le solvant sous vide. On chromatographie le résidu sur gel de silice en éluant par le gradient du mélange DCM/MeOH de (100/0; v/v) à (70/30 ; v/v). On obtient le composé attendu.

RMN$^1$H : DMSO-d6 (250 MHz) : δ (ppm) : 0,85-1,07 : mt : 1H ; 1,13-1,34 : mt : 1H ; 2,00-2,45 : mt : 12H ; 2,111 : s : 3H ; 3,856 : s : 3H ; 6,651 : s : 1H ; 6,926 : s : 1H ; 7,01-7,12 : mt : 1H ; 7,21-7,40 : mt : 2H ; 7,56-7,66 : mt : 1H ; 10,73 : s: 1H.

Selon les modes opératoires décrits dans les Préparations ci-dessus, à partir de composés précédemment décrits de formule (VIII) et de composés de formule (V) appropriés, on obtient les composés de formule (II), rassemblés dans le tableau VI suivant :

TABLEAU VI

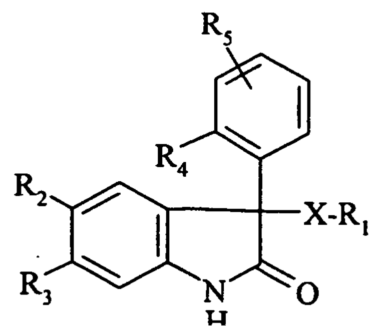

(II) $R_3$ = OMe

| Préparation | $R_2$ | $R_4$ | $R_5$ | X | $R_1$ | $MH^+$ ; tr (mn) Conditions |
|---|---|---|---|---|---|---|
| 2.5 | Cl | F | H | $(CH_2)_3$ | 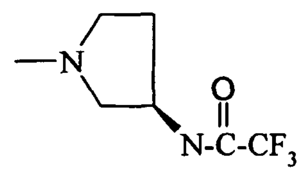 | 514 ; 4,34 (M 3) |
| 2.6 | Cl | F | H | $(CH_2)_3$ | 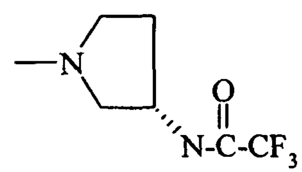 | 514 ; 4,30 (M 3) |
| 2.7 | Cl | F | H | $(CH_2)_3$ | 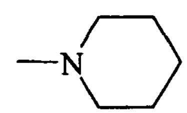 | 417 ; 5,89 (M 1) |

(suite)

| Préparation | $R_2$ | $R_4$ | $R_5$ | X | $R_1$ | $MH^+$ ; tr (mn) Conditions |
|---|---|---|---|---|---|---|
| 2.8 | Cl | F | H | $(CH_2)_3$ | 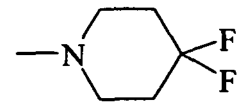 | 453 ; 4,24 (M 3) |
| 2.9 | Cl | F | H | $(CH_2)_3$ | 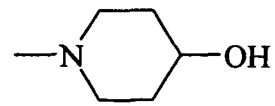 | 433 ; 4,00 (M 3) |
| 2.10 | Cl | F | H | $(CH_2)_3$ | 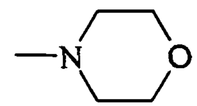 | 419 ; 5,23 (M 1) |
| 2.11 | Cl | F | H | $(CH_2)_3$ | 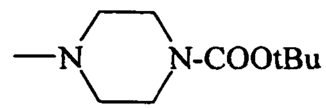 | 518 ; 6,62 (M 1) |
| 2.12 | Cl | F | H | $(CH_2)_3$ | 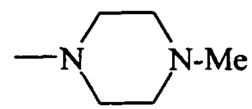 | 432 ; 5,55 (M 1) |
| 2.13 | Cl | F | H | $(CH_2)_3$ | 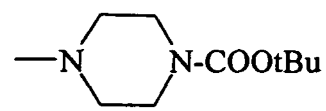 | 518 ; 6,43 (M 1) |

(suite)

| Préparation | $R_2$ | $R_4$ | $R_5$ | X | $R_1$ | $MH^+$ ; tr (mn) Conditions |
|---|---|---|---|---|---|---|
| 2.14 | Cl | F | H | $(CH_2)_3$ | 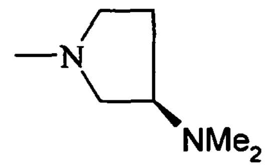 | 446 ; 4,43 (M 4) |
| 2.15 | Cl | F | H | $(CH_2)_3$ | 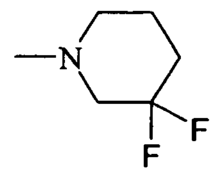 | 453 ; 4,27 (M 3) |
| 2.16 | Cl | Me | H | $(CH_2)_3$ | 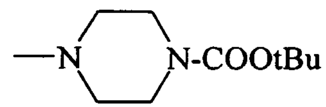 | 514 ; 5,94 (M 4) |
| 2.17 | Cl | F | 5-F | $(CH_2)_3$ | 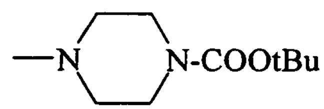 | 536 ; 4,45 (M 3) |
| 2.18 | Cl | F | 6-F | $(CH_2)_3$ | 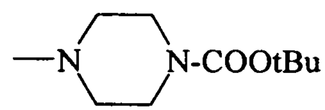 | 536 ; 5,95 (M 4) |
| 2.19 | Cl | F | 3-Cl | $(CH_2)_3$ | 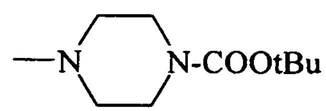 | 552 ; 4,59 (M 3) |

(suite)

| Préparation | $R_2$ | $R_4$ | $R_5$ | X | $R_1$ | $MH^+$ ; tr (mn) Conditions |
|---|---|---|---|---|---|---|
| 2.20 | Me | F | H | $(CH_2)_3$ | 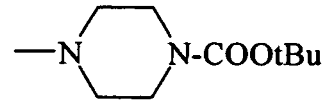 | 498 ; 5,76 (M 4) |
| 2.21 | Me | F | H | $(CH_2)_3$ | 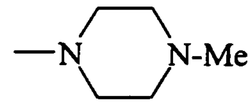 | 412 ; 4,70 (M 4) |
| 2.22 | Me | F | H | $(CH_2)_3$ | 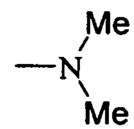 | 357 ; 5,01 (M 4) |
| 2.23 | Ome | F | H | $(CH_2)_3$ | 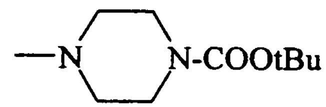 | 514 ; 5,59 (M 4) |
| 2.24 | Ome | F | H | $(CH_2)_3$ | 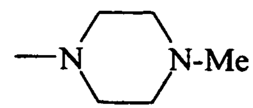 | 428 ; 4,97 (M 4) |
| 2.25 | Cl | F | H | $(CH_2)_3$ | 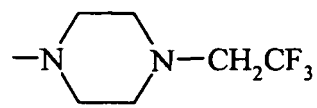 | 500 ; 6.16 (M4) |
| 2.26 | Cl | F | H | $(CH_2)_3$ | 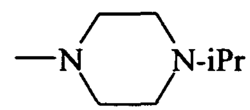 | 460 ; 4.98 (M4) |

(suite)

| Préparation | $R_2$ | $R_4$ | $R_5$ | X | $R_1$ | $MH^+$ ; tr (mn) Conditions |
|---|---|---|---|---|---|---|
| 2.27 | Cl | F | H | $(CH_2)_5$ | 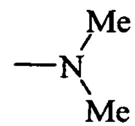 | 405 ; 5.90 (M4) |
| 2.28 | Cl | F | H | $(CH_2)_3$ | 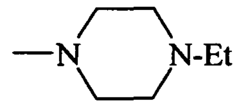 | 446 ; 5.45 (M4) |
| 2.29 | Cl | F | H | $(CH_2)_3$ | 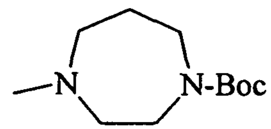 | 532 ; 5.78 (M4) |
| 2.30 | Cl | F | H | $(CH_2)_5$ | 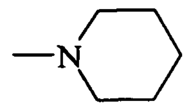 | 445 ; 6.12 (M4) |
| 2.31 | Cl | F | H | $(CH_2)_2$ | 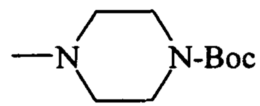 | 504 ; 6.36 (M4) |
| 2.32 | Cl | F | 5-$CO_2$Me | $(CH_2)_3$ | 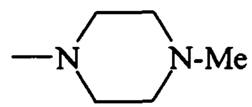 | 490 ; 5,60 (M4) |

Préparation 2.33

5-Chloro-3-[2-fluoro-5-(hydroxyméthyl)phényl]-6-méthoxy-3-[3-(4-méthylpipérazin-1-yl)propyl]-1,3-dihydro-2*H*-indol-2-one, isomère unique.

(II :

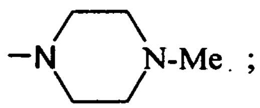

$R_2$ = Cl ;$R_3$ = OMe ; $R_4$ = F ; $R_5$ = 5-$CH_2OH$ ; X = -($CH_2$

A la solution de 0,22 g de 3-{5-chloro-6-méthoxy-3-[3-(4-méthylpipérazin-1-yl)propyl]-2-oxo-2,3-dihydro-1*H*-indol-3-yl}-4-fluoro benzoate de méthyle isomère unique (Préparation 2.32) dans 4 ml de DCM, refroidie à -50˚C, on ajoute 1,5 ml d'hyrure de diisobutyl aluminium 1M dans du toluène. On laisse lentement remonter la température à 20˚ C pendant 24 heures. On refroidit à -10˚C, ajoute une solution aqueuse de carbonate de sodium, extrait au DCM, sèche la phase organique sur $Na_2SO_4$ et évapore le solvant sous vide. On chromatographie le résidu sur gel de silice en éluant par le gradient du mélange DCM/MeOH de (90/10 ; v/v) jusqu'à (80/20 ; v/v). On obtient le composé attendu sous forme de résine.

$MH^+$ = 462 ; tr = 5,00 mn ; (M 4).

3. Préparations des composés de formule (III) :

Préparation 3.1

Chlorure de 2,4-diméthoxybenzènesulfonyle.

(III) : $R_6$ = -OMe ; $R_7$= 2-Ome ; Hal = Cl.

On prépare ce composé selon J. Am. Chem. Soc., 1952, 74, 2008.

Préparation 3.2

Chlorure de 4-éthoxy-3-méthoxybenzènesulfonyle.

(III) : $R_6$ = -Oet ; $R_7$= 3-Ome ; Hal = Cl.

A- 1-Ethoxy-2-méthoxybenzène.

A un mélange de 10 g de -2-méthoxyphénol dans 100 ml de DMF, on ajoute 28 g de carbonate de césium puis 25,77 ml d'iodoéthane et chauffe à 50˚C pendant 8 heures. On ajoute 450 ml d'eau au mélange réactionnel, extrait à l'AcOEt, lave la phase organique à l'eau, sèche sur $Na_2SO_4$ et évapore le solvant sous vide. On distille le résidu sous vide et obtient le composé attendu, Eb = 102-105˚C sous 24 mbar.

B- Chlorure de 4-éthoxy-3-méthoxybenzènesulfonyle.

On refroidit à -10˚C 10 g du composé obtenu à l'étape précédente, ajoute goutte à goutte 21,84 ml d'acide chlorosulfonique puis 13,68 g de pentachlorure de phosphore et laisse 1 heure sous agitation à -5˚C. On coule le mélange réactionnel sur 250 ml de glace et d'eau, extrait au DCM, sèche la phase organique sur $Na_2SO_4$ et évapore le solvant sous vide. On chromatographie le résidu sur gel de silice en éluant par le gradient du mélange cychlohexane/AcOEt de (95/5 ; v/v) à (85/15 ; v/v). On obtient le composé attendu, F = 93˚C.

RMN$^1$H : $CDCl_3$ (250 MHz) : δ (ppm) : 1,55 : t : 3H ; 3,99 : s : 3H ; 4,23 : q : 2H ; 6,97-7,02 : mt : 1H ; 7,46-7,48 : mt : 1H ; 7,66-7,71 : mt : 1H.

Préparation 3.3

Chlorure de 4-(1,1,2,2-tétrafluoroéthoxy)benzènesulfonyle.

(III) : $R_6$ = $-OCF_2CHF_2$ ; $R_7$= H ; Hal = Cl.

On ajoute goutte à goutte 1,94 g de (1,1,2,2-tétrafluoroéthoxy)benzène sur 5,83 g d'acide chlorosulfonique refroidi à 5˚C. On coule le mélange réactionnel sur 250 ml de glace et d'eau, extrait au DCM, sèche la phase organique sur $MgSO_4$ et évapore le solvant sous vide. On chromatographie le résidu sur gel de silice en éluant par le gradient du mélange heptane/DCM de (8/2 ; v/v) à (1/1 ; v/v). On obtient le composé attendu.
RMN $^1H$ : $CDCl_3$ (300 MHz) : δ (ppm) : 5.99 : t : 1 H ; 7.49 : d : 2H ; 8.13 : d : 2H.

Préparation 3.4

Chlorure de 3-méthoxy-4-(1-méthyléthoxy)benzènesulfonyle.

(III) : $R_6$ = -OiPr ; $R_7$= 3-Ome ; Hal = Cl.

A- 1-Méthoxy-2-(1-méthyléthoxy)benzène.

A un mélange de 10 g de 2-méthoxyphénol (commercial) dans 100 ml d'acétonitrile, on ajoute 16,7 g de carbonate de potassium, puis 12 ml de 2-iodopropane et chauffe à reflux pendant 8 heures. On ajoute 200 ml d'eau au mélange réactionnel, extrait à l'AcOEt, lave la phase organique par une solution de NaOH 1N, par une solution saturée de NaCl, sèche sur $MgSO_4$ et évapore le solvant sous vide. On obtient le composé attendu sous forme d'une huile.

B- 3-Méthoxy-4-(1-méthyléthoxy)benzenesulfonate de sodium.

On ajoute goutte à goutte 2,7 ml d'acide chlorosulfonique sur une solution refroidie à 0˚ C de 6,6 g du composé précédent dans 25 ml de DCM. Le mélange est agité pendant 30 minutes à 0˚ C, puis on le coule sur 100 ml de glace et d'eau, élimine la phase organique, basifie la phase aqueuse avec de la soude 40 % et essore le précipité formé.

C- Chlorure de 3-méthoxy-4-(1-méthyléthoxy)benzènesulfonyle.

A une suspension de 3,2 g du composé précédent dans 60 ml de DCM, sont ajoutés 2 gouttes de DMF, puis 3,1 ml de chlorure d'oxalyle. Après agitation à TA pendant 18 heures, le mélange est concentré et chromatographié sur gel de silice en éluant par le gradient du mélange heptane/$iPr_2O$ de (95/5 ; v/v) à (7/3 ; v/v).
RMN$^1H$ : $CDCl_3$ (300 MHz) : δ (ppm) : 1.30 : d : 6H ; 3.78 : s : 3H ; 4.77 : mt : 1H ; 6.59 : d : 1H ; 7.24 : s : 1H ; 7.58 : d : 1H.

Préparation 3.5

Chlorure de 4-(1-méthyléthoxy)-3-(trifluorométhyl)benzènesulfonyle.

(III) : $R_6$ = -OiPr ; $R_7$= 3-$CF_3$ ; Hal = Cl.

Le composé est obtenu en utilisant le mode opératoire de la préparation 3.4, à partir du 2-(trifluorométhyl)phénol.
RMN$^1H$ : $CDCl_3$ (300 MHz) : δ (ppm) : 1.32 : d : 6H ; 4.59 : m : 1H ; 7.22 : d : 1H ; 7.92 : d : 1H ; 8.12 : s : 1H.

Préparation 3.6

Chlorure de 4-(3,3-diéthyl-uréido)-3-méthyl-benzènesulfonyle.

A-1,1-Diéthyl-3-o-tolyl-urée :

A 5 g d'o-méthyl-aniline en solution dans 25 ml de DMF, on ajoute 7,75g de carbonate de potassium et 7,1ml de N,N-diéthyl-chlorure de carbamoyle. On agite le milieu à 60˚ C pendant 4 heures. Le milieu refroidit à 20˚ C est coulé sur de la glace et extrait avec de l'AcOEt, la phase organique est lavée avec de l'acide chlorhydrique 1N puis à la soude 0.5N, séchée sur $Na_2SO_4$ et concentrée sous pression réduite pour donner le produit attendu sous forme solide.

RMN[1]H : $CDCl_3$ (300 MHz) : δ (ppm) : 1,11 : t :6H ; 2,17 : s : 3H ; 3,33 : q : 4H ; 6,96-7,23 : m : 4H ; 7,66 : s : 1H.

B- Chlorure de 4-(3,3-Diéthyl-uréido)-3-méthyl-benzènesulfonyle :

A 42,5ml d'acide chlorosulfonique refroidie à 0° C, on ajoute 6,6 g de 1,1-Diéthyl-3-o-tolyl-urée (préparé en A). Après 2 heures d'agitation à température voisine de 5° C, on coule sur de la glace et extrait avec de l'AcOEt, la phase organique est séchée sur $Na_2SO_4$ et concentrée sous pression réduite. Le résidu est lavé avec un mélange DCM / pentane pour donner, après séchage à 40° C, le composé attendu sous forme solide.
RMN[1]H : DMSO-d6 (250 MHz) : δ (ppm) : 1,17 : t : 6H ; 2,17 : s : 3H ; 3,32 : q : 4H ; 6,42 : s : 1H ; 7,67-7,79 : m : 2H ; 8,26 : d : 1H.

Préparation 3.7

Chlorure de 4-(3,3-Diéthyl-uréido)-3-fluoro-benzènesulfonyle

A- 1,1-Diéthyl-3-(2-fluoro-phényl)-urée :

A 5 g d'o-fluoro-aniline en solution dans 25 ml de DMF, on ajoute 7,46 g de carbonate de potassium et 6,84 ml de N,N-diéthyl-chlorure de carbamoyle. On agite le milieu à 60° C pendant 2 heures. Le milieu refroidit à 20° C est coulé sur de la glace et extrait avec de l'acétate d'éthyle, la phase organique est lavée avec de l'acide chlorhydrique 1N puis à la soude 0,5 N, séchée sur sulfate de sodium et concentrée sous pression réduite pour donner le composé attendu sous forme solide.
RMN[1]H : DMSO-d6 (400 MHz) : δ (ppm) : 1,,1 : t : 6H ; 3,33 : q : 4H ; 7,06-7,12 : m : 1 H ; 7,13-7,20 : m : 1 H ; 7,41-7,49 : m : 1 H ; 7,49 : s : 1 H.

B- Chlorure de 4-(3,3-Diéthyl-uréido)-3-fluoro-benzènesulfonyle :

A 9,5ml d'acide chlorosulfonique refroidie à 0° C, on ajoute 1 g de 1,1-Diéthyl-3-(2-fluoro-phényl)-urée (préparé en A). Après 4 heures d'agitation à température voisine de 15°C, on coule sur de la glace et extrait avec de l'AcOEt, la phase organique est séchée sur $Na_2SO_4$ et concentrée sous pression réduite. Le résidu est lavé avec un mélange DCM / pentane pour donner, après séchage à 40°C, le composé attendu sous forme solide.
RMN[1]H : DMSO-d6 (250 MHz) : δ (ppm) : 1,16 : t : 6H ; 3,31 : q : 4H ; 6,80 : d : 1H ; 7,60-7,74 : m : 2H ; 8,47 : q : 1H.

Selon les modes opératoires décrits dans les Préparations ci-dessus, on prépare les composés de formule (III) rassemblés dans le TABLEAU VII ci-après :

TABLEAU VII

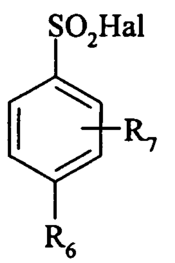

(III)

| Préparations | $R_6$ | $R_7$ | Hal |
|---|---|---|---|
| 3.8 | -OiPr | H | Cl |
| 3.9 | -Ome | 3-Ome | Cl |
| 3.10 | -Me | 3-Me | Cl |
| 3.11 | -Ome | 3-Me | Cl |
| 3.12 | -Oet | H | Cl |
| 3.13 | -Oet | 3-Me | Cl |
| 3.14 | $-OCHF_2$ | H | Cl |

(suite)

| Préparations | $R_6$ | $R_7$ | Hal |
|---|---|---|---|
| 3.15 | $-OCF_3$ | H | Cl |

4. Préparations des composés de formule (IV) :

Préparation 4.1

5-Chloro-3-(3-chloropropyl)-3-(2-fluorophényl)-6-méthoxy-1-{[4-(1-méthyléthoxy)phényl]sulfonyl}-1,3-dihydro-2*H*-indol-2-one isomère unique.

(IV) : $R_2$ = Cl ; $R_3$ = Ome ; $R_4$ = F; $R_5$ = H ; X = $-(CH_2)_3-$ ; Z = Cl ; $R_6$ = OiPr ; $R_7$ = H

A une solution de 11 g de l'isomère dextrogyre issu de la préparation 2a.2 dans 100 ml de THF à TA, on ajoute 3,7 g de *tert*-butylate de potassium, puis 7,7 g de chlorure de 4-(1-méthyléthoxy)benzènesulfonyle (commercial) et laisse sous agitation pendant 3 heures. On ajoute 200 ml de solution aqueuse de chlorure d'ammonium à 10%, extrait à l'AcOEt, sèche la phase organique sur $MgSO_4$ et évapore le solvant sous vide. On chromatographie le résidu sur gel de silice en éluant par le gradient du mélange heptane/AcOEt de (99/1 ; v/v) à (1/1 ; v/v). On obtient le composé attendu sous forme d'une résine beige.
$MH^+$ = 564 ; tr = 10,9 mn ; (M4)

Préparation 4.2

1-[(4-*tert*-butoxyphényl)sulfonyl]-5-chloro-3-(3-chloropropyl)-3-(2-fluorophényl)-6-méthoxy-1,3-dihydro-2*H*-indol-2-one isomère unique.

(IV) : $R_2$ = Cl ; $R_3$ = Ome ; $R_4$ = F ; $R_5$ = H ; X = $-(CH_2)_3-$ ; Z = Cl ; $R_6$ = OtBu; $R_7$=H

A-1-{[4-(benzyloxy)phényl]sulfonyl}-5-chloro-3-(3-chloropropyl)-3-(2-fluorophényl)-6-méthoxy-1,3-dihydro-2*H*-indol-2-one.

Le composé est obtenu en utilisant le mode opératoire de la préparation 4.1, à partir du chlorure de 4-(benzyloxy) benzènesulfonyle (commercial).
$MH^+$ = 614 ; tr = 6,93 mn ; (M3)

B- 5-chloro-3-(3-chloropropyl)-3-(2-fluorophényl)-1-[(4-hydroxyphényl) sulfonyl]-6-méthoxy-1,3-dihydro-2H-indol-2-one

A une solution de 0,61 g du composé précédent dans 40 ml de THF/MeOH (1/1), sont ajoutés 122 mg de palladium sur charbon à 10%, puis 189 mg de formiate d'ammonium. Après 1 heure sous agitation à TA, le milieu réactionnel est filtré sur talc et le filtrat concentré sous vide. Le résidu est repris dans l'AcOEt, lavé par une solution de chlorure d'ammonium à 10%, par une solution saturée de NaCl, séché sur MgSO4 et concentré sous vide. On chromatographie le résidu sur gel de silice en éluant par le gradient du mélange heptane/AcOEt de (4/1 ; v/v) à (2/3 ; v/v). On obtient le composé attendu sous forme d'une résine beige.
$MH^+$ = 524 ; tr = 6,02 mn ; (M4)

C- 1-[(4-*tert*-butoxyphényl)sulfonyl]-5-chloro-3-(3-chloropropyl)-3-(2-fluorophényl)-6-méthoxy-1,3-dihydro-2*H*-indol-2-one.

Le composé attendu est obtenu en utilisant la méthode décrite dans J. Org. Chem., 2006, 71, 9580.
A une suspension de 1,82 g du composé précédent dans 15 ml DCM, sont ajoutés 85 mg de triflate de scandium, puis 2,65 g de di-*tert*-butyl dicarbonate. Après 18 heures sous agitation à TA, le mélange est concentré sous vide et le résidu est chromatographié sur gel de silice en éluant par le gradient du mélange heptane/DCM de (1/1 ; v/v) à (1/9 ; v/v). On obtient le composé attendu sous forme d'une résine blanche.
$MH^+$ = 580 ; tr = 6,94 mn ; (M4)

EXEMPLE 1 : Composé N° 1 et composé N° 2

Chlorhydrate de 5-chloro-1-[(2,4-diméthoxyphényl)sulfonyl]-3-(2-fluorophényl)-6-méthoxy-3-(3-pipéridin-4-ylpropyl)-1,3-dihydro-2*H*-indol-2-one, isomère dextrogyre et isomère lévogyre.

A- 4-[3[5-Chloro-1-[(2,4-diméthoxyphényl)sulfonyl]-3-(2-fluorophényl)-6-méthoxy-2-oxo-2,3-dihydro-1*H*-indol-3-yl]propyl]pipéridin-1-carboxylate de *tert*-butyle.

On refroidit à -30°C une solution de 0,28 g du composé de la Préparation 2.1 dans 10 ml de THF, ajoute 0,073 g de *tert*-butylate de potassium et laisse sous agitation en laissant remonter la température à 0°C. On refroidit le mélange réactionnel à -60°C, ajoute 0,147 g du composé de la Préparation 3.1 et laisse une nuit sous agitation à 20°C. On hydrolyse le mélange réactionnel par ajout d'eau, extrait à l'AcOEt, sèche la phase organique sur $Na_2SO_4$ et évapore le solvant sous vide. On obtient le composé attendu.

B- Chlorhydrate de 5-chloro-1-[(2,4-diméthoxyphényl)sulfonyl]-3-(2-fluorophényl)-6-méthoxy-3-(3-pipéridin-4-ylpropyl)-1,3-dihydro-2*H*-indol-2-one, isomère dextrogyre et isomère lévogyre.

On reprend le composé de l'étape précédente par une solution d'éther chlorhydrique 2N et laisse 16 heures sous agitation à 20°C. On essore le précipité formé et obtient le composé attendu sous forme racémique, F = 155°C.

Les énantiomères du composé ainsi obtenu sont séparés par chromatographie supercritique sur phase chirale dans les conditions suivantes :

Appareillage : Système SFC METTLER TOLEDO APS1010 ;
Colonne chirale : CHIRALPAK AD-H :
Phase mobile : $CO_2$ / (MeOH + 0,5% propan-2-yl amine) : (70/30 ; v/v) ;
Débit : 50 ml/minute ;
Pression / Température : 100 bar / 40°C ;
Détection UV : 220 nm ;

On obtient les deux énantiomères, respectivement repris dans une solution d'éther chlorhydrique 2 N pour donner :

- composé N° 1 : isomère dextrogyre : F = 163°C ;

$$\alpha_D^{20} = + 105{,}7° \ (c = 1 ; MeOH) ;$$

RMN$^1$H: DMSO-d6 (250 MHz) : δ (ppm) : 0,61-0,85 : mt : 2H ; 0,98-1,20 : mt : 4H ; 1,26-1,56 : mt : 3H ; 1,99-2,15 : mt : 1H ; 2,26-2,42 : mt : 1H ; 2,67-2,86 : mt : 2H ; 3,10-3,22 : mt : 2H ; 3,65 : s : 3H ; 3,88 : s : 3H ; 3,93 : s : 3H ; 6,72-6,79 : mt : 2H ; 6,99-7,1 : mt : 2H ; 7,104 : s : 1H ; 7,23-7,43 : mt : 2H : 7,59-7,69 : mt : 2H ; 7,88-7,94 : mt : 1H ; 8,43 : mt : 2H.

- composé N° 2 : isomère lévogyre : F = 175°C ;

$$\alpha_D^{20} = -104{,}5° \ (c = 1 ; MeOH) ;$$

RMN$^1$H : DMSO-d6 (250 MHz) : δ (ppm) : 0,61-0,85 : mt : 2H ; 0,99-1,19 : mt : 4H ; 1,27-1,57 : mt : 3H ; 1,99-2,16 : mt : 1H ; 2,26-2,85 : mt : 2H ; 3,10-3,22 : mt : 2H ; 3,65 : s : 3H ; 3,88 : s : 3H ; 6,72-6,79 : mt : 2H ; 6,99-7,1 : mt : 1H ; 7,104 : s : 1H ; 7,23-7,43 : mt : 2H : 7,59-7,69 : mt : 2H ; 7,88-7,94 : mt : 1H ; 8,43 : mt : 2H.

EXEMPLE 2 : Composé N° 3

Chlorhydrate de 5-chloro-3-[3-(diméthylamino)propyl]-3-(2-fluorophényl)-1-[(4-isopropoxyphényl)sulfonyl]-6-méthoxy-1,3-dihydro-2*H*-indol-2-one, isomère dextrogyre.

On refroidit à -30°C une solution de 0,17 g du composé de la Préparation 2.3 dans 2 ml de THF, ajoute 0,54 ml de *tert*-butylate de potassium 1M en solution dans le THF et laisse sous agitation en laissant remonter la température à 0°C. On refroidit le mélange réactionnel à -60°C, ajoute 0,127 g de chlorure de 4-isopropoxybenzènesulfonyle commercial et laisse une nuit sous agitation à 20°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le gradient du mélange DCM/MeOH de (100/0 ; v/v) à (90/10 ; v/v). On obtient le produit attendu sous forme de résine blanche, F = 173°C.

$$\alpha_D^{20} = +88{,}1° \ (c = 1 ; AcOEt).$$

EXEMPLE 3 : Composé N° 4

Chlorhydrate de 3-[3-[(3R)-aminopyrrolidin-1-yl]propyl]-5-chloro-3-(2-fluorophényl)-1-[(4-isopropoxyphényl)sulfonyl]-6-méthoxy-1,3-dihydro-indol-2-one, isomère dextrogyre.

On refroidit à -30°C une solution de 0,177 g du composé de la Préparation 2.5 dans 3 ml de THF, ajoute 0,48 ml d'une solution 1M de *tert*-butylate de potassium dans le THF et laisse sous agitation en laissant remonter la température à 0°C. On refroidit le mélange réactionnel à -60°C, ajoute 0,112 g de chlorure de 4-isopropoxybenzènesulfonyle et laisse une nuit sous agitation à 20°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le gradient du mélange DCM/MeOH de (100/0 ; v/v) à (98/2 ; v/v). On obtient le N-[(1R)-[3-[5-chloro-3-(2-fluorophényl)-1-[(4-isopropoxyphényl) sulfonyl]-6-méthoxy-2-oxo-2,3-dihydro-1*H*-indol-3-yl]propyl]pyrrolidin-3-yl]-2,2,2-trifluoroacétamide. On reprend le composé ainsi obtenu dans une solution 12 N d'HCl dans l'éthanol, chauffe à 80°C pendant 2 heures et concentre sous vide. On reprend le résidu par une solution d'éther chlorhydrique et essore le précipité formé. On obtient le composé attendu, F = 215°C.

$$\alpha_D^{20} = +122° \ (c = 1 ; MeOH).$$

EXEMPLE 4 : Composé N° 13

Chlorohydrate de 5-chloro-3-(2-fluorophényl)-1-[(4-isopropoxyphényl)sulfonyl]-6-méthoxy-3-[3-(4-méthylpipérazin-1-yl) propyl]-1,3-dihydroindol-2-one, isomère dextrogyre.

On refroidit à -30°C une solution de 0,128 g du composé de la Préparation 2.4 dans 2 ml de THF, ajoute 0,066 g de tert-butylate de potassium et laisse sous agitation en laissant remonter la température à 0°C. On refroidit à -30°C le mélange réactionnel, ajoute 0,09 g de chlorure de 4-isopropoxybenzènesulfonyle et laisse une nuit sous agitation à 10°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le mélange DCM/MeOH. On reprend le produit ainsi obtenu dans de l'éther chlorhydrique 2N, essore le précipité formé et le sèche. On obtient le composé attendu, F = 205°C.
$MH^+$ = 630 ; tr = 7,09 mn (M1).

$$\alpha_D^{20} = +114{,}8° \ (c = 1 ; MeOH).$$

RMN$^1$H : DMSO-d6 (400 MHz) : δ (ppm) : 1,24-1,38 : mt : 8H ; 2,18-2,30 : mt : 1H ; 2,35-2,46 : mt : 1H ; 2,782 : s : 3H ;

2,95-3,89 : mt : 10H ; 3,986 : s : 3H ; 4,76-4,84 : mt : 1H ; 6,909 : q : 1H ; 7,105 : s : 1H ; 7,171 : d : 2H ; 7,23-7,30 : mt : 1H ; 7,32-7,41 : mt : 1H : 7,62-7,70 : mt : 2H ; 8,014 : d : 2H ; 10,72-11,96 : mt : 2H.

EXEMPLE 5 : Composé N° 14

Chlorhydrate de 5-chloro-[(4-éthoxy-3-méthoxyphényl)sulfonyl]-3-(2-fluorophényl)-6-méthoxy-3-[3-(4-méthylpipérazin-1-yl)propyl]-1,3-dihydro-indol-2-one, isomère dextrogyre.

On refroidit à -30°C une solution de 0,128 g du composé de la Préparation 2.4 dans 2 ml de THF, ajoute 0,066 g de tert-butylate de potassium et laisse sous agitation en laissant remonter la température à 0°C. On refroidit le mélange réactionnel à -30°C, ajoute 0,097 g de chlorure de 4-éthoxy-3-méthoxybenzènesulfonyle (décrit dans Chem. Ber., 1906, 39, 2777) et laisse une nuit sous agitation à 10°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le mélange DCM/MeOH. On reprend le produit ainsi obtenu dans de l'éther chlorhydrique 2N, essore le précipité formé et le sèche. On obtient le composé attendu, F = 180°C.

$$\alpha_D^{20} = + 106{,}2° \text{ (c = 1 ; MeOH).}$$

$MH^+$ = 646 ; tr = 6,76 mn (M1).

RMN$^1$H : DMSO-d6 (400 MHz) : δ (ppm) : 1,15-1,34 : mt : 2H ; 1,369 : t : 3H ; 2,18-2,31 : mt : 1H ; 2,34-2,45 : mt : 1H ; 2,788 : s : 3H ; 2,93-3,34 : mt : 2H ; 3,42-3,77 : mt : 8H ; 3,798 : s : 3H ; 3,978 : s : 3H ; 4,16 : q : 2H ; 6,986 : q : 1 H ; 7,111 : s : 1H : 7,19-7,24 : mt : 1 H ; 7,25-7,31: mt : 1 H ; 7,33-7,41 : mt : 1 H ; 7,491 : d : 1 H ; 7,681 : t : 1H ; 7,742 : q : 1H ; 10,96-11,9 : mt : 2H.

EXEMPLE 6 : Composé N° 17

Chlorhydrate de 5-chloro-3-[(2-fluorophényl)-1-[(4-isopropoxyphényl)sulfonyl]-6-méthoxy-3-[3-(pipérazin-1-yl)propyl]-1,3-dihydro-indol-2-one, isomère dextrogyre.

A- 4-[3-[5-Chloro-3-(2-fluorophényl)-1-[(4-isopropoxyphényl)sulfonyl]-6-méthoxy-2-oxo-2,3-dihydro-1*H*-indol-3-yl]propyl]piperazine-1-carboxylate de *tert*-butyle.

On refroidit à -30°C une solution de 0,2 g du composé de la Préparation 2.11 dans 3 ml de THF, ajoute 0,086 g de *tert*-butylate de potassium et laisse sous agitation en laissant remonter la température à 0°C. On refroidit le mélange réactionnel à -30°C, ajoute 0,107 g de chlorure de 4-isopropoxybenzène sulfonyle (commercial) et laisse une nuit sous agitation à 10°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le mélange DCM/MeOH et obtient le composé attendu sous forme de résine.

B- Chlorhydrate de 5-chloro-3-[(2-fluorophényl)-1-[(4-isopropoxyphényl)sulfonyl]-6-méthoxy-3-[3-(pipérazin-1-yl)propyl]-1,3-dihydro-indol-2-one, isomère dextrogyre.

On solubilise le composé attendu à l'étape précédente dans un minimum de DCM, ajoute de l'éther chlorhydrique 2N, laisse 16 heures sous agitation à 20°C, essore le précipité formé et le sèche. On obtient le composé attendu, F = 232°C.

$$\alpha_D^{20} = + 120{,}8° \text{ (c = 1 ; MeOH),}$$

,
$MH^+$ = 616 ; tr = 6,51 mn ; (M1).

RMN$^1$H : DMSO-d6 (400 MHz) : δ (ppm) : 1,15-1,39 : mt : 8H ; 2,17-2,26 : mt : 1H ; 2,35-2,52 : mt : 1H ; 3,03-3,75 : mt : 10H ; 3,99 : s : 3H ; 4,75-4,85 : mt : 1H ; 6,91 : q : 1 H ; 7,109 : s : 1H ; 7,15-7,41 : mt : 4H ; 7,63-7,72 : mt : 2H ; 7,95-8,06 : mt : 2H ; 9,4-9,8 : mt : 2H ; 11,1-11,5 : mt : 1H.

EXEMPLE 7 : Composé N° 55

5-Chloro-3-(2-fluorophényl)-6-méthoxy-3-[3-(4-méthyl-1,4-diazepan-1-yl)propyl]-1-{[4-(1-méthyléthoxy)phényl]sulfonyl}-1,3-dihydro-2*H*-indol-2-one, isomère dextrogyre.

A-4-{3-[5-chloro-3-(2-fluorophényl)-6-méthoxy-1-{[4-(1-méthyléthoxy)phényl]sulfonyl}-2-oxo-2,3-dihydro-1*H*-indol-3-yl]propyl}-1,4-diazepane-1-carboxylate de *tert*-butyle.

A une solution de 250 mg de l'isomère unique issu de la préparation 4.1 dans 3 ml d'acétonitrile, sont ajoutés 106 mg de 1,4-diazepane-1-carboxylate de *tert*-butyle, 61 mg de carbonate de potassium, 37 mg d'iodure de potassium, puis le mélange est porté à 120° sous microondes (CEM discover) dans un tube fermé pendant 30 min. Le mélange est filtré, puis le filtrat est concentré. On chromatographie le résidu sur gel de silice en éluant par le gradient du mélange DCM/MeOH (100/0 ; v/v) à (95/5 ; v/v) et obtient le composé attendu sous forme de résine.
$MH^+$ = 730 ; tr = 7,90 mn ; (M4).

B- Chlorhydrate de 5-chloro-3-[3-(1,4-diazepan-1-yl)propyl]-3-(2-fluorophényl)-6-méthoxy-1-{[4-(1-méthyléthoxy)phényl]sulfonyl}-1,3-dihydro-2*H*-indol-2-one.

On solubilise 285 mg du composé précédent dans un minimum de DCM, ajoute 2 ml d'éther chlorhydrique 2N, laisse 16 heures sous agitation à 20°C, dilue avec 20 ml d'éther, essore le précipité formé et le sèche.
$MH^+$ = 630 ; tr = 6,24 mn ; (M4).

C- Chlorhydrate de 5-chloro-3-(2-fluorophényl)-6-méthoxy-3-[3-(4-méthyl-1,4-diazepan-1-yl)propyl]-1-{[4-(1-méthyléthoxy)phényl]sulfonyl}-1,3-dihydro-2*H*-indol-2-one.

A une solution de 280 mg du composé précédent dans un minimum de DCM, sont ajoutés 33 μl d'une solution aqueuse de formaldéhyde à 40 %, 123 mg de triacétoxyborohydrure de sodium, puis le mélange est agité à TA pendant 4 heures. On hydrolyse le mélange réactionnel par ajout d'une solution aqueuse de bicarbonate de sodium à 10 %, extrait à l'AcOEt, sèche la phase organique sur $MgSO_4$ et évapore le solvant sous vide. On chromatographie le résidu sur gel de silice en éluant par le gradient du mélange DCM/MeOH (100/0 ; v/v) à (90/10 ; v/v) et obtient le composé attendu sous sa forme basique. Cette dernière est reprise dans 3 ml DCM, puis on ajoute de l'éther chlorhydrique 2N, puis dilue avec 20 ml d'éther, essore le précipité formé et le sèche
$MH^+$ = 644 ; tr = 6,32 mn ; (M4).

EXEMPLE 8 : Composé N° 72

1-[(4-*tert*-butoxyphényl)sulfonyl]-5-chloro-3-(2-fluorophényl)-6-méthoxy-3-(3-pipérazin-1-ylpropyl)-1,3-dihydro-2*H*-indol-2-one, isomère dextrogyre.

A-4-(3-{1-[(4-*tert*-butoxyphényl)sulfonyl]-5-chloro-3-(2-fluorophényl)-6-méthoxy-2-oxo-2,3-dihydro-1*H*-indol-3-yl}propyl)pipérazine-1-carboxylate de benzyle.

A une solution de 171 mg de l'isomère unique issu de la préparation 4.2 dans 3 ml d'acétonitrile, sont ajoutés 71 μl de pipérazine-1-carboxylate de benzyle, 42 mg de carbonate de potassium, 50 mg d'iodure de potassium, puis le mélange est porté à 120° sous microondes (CEM discover) dans un tube fermé pendant 1 heure. Le mélange est filtré, puis le filtrat est concentré. On chromatographie le résidu sur gel de silice en éluant par le gradient du mélange heptane/AcOEt (4/1 ; v/v) à (1/1 ; v/v). On obtient le composé attendu sous forme d'une résine blanche.
$MH^+$ = 764 ; tr = 5,51 mn ; (M3).

B-1-[(4-*tert*-butoxyphényl)sulfonyl]-5-chloro-3-(2-fluorophényl)-6-méthoxy-3-(3-pipérazin-1-ylpropyl)-1,3-dihydro-2*H*-indol-2-one.

A une solution de 200 mg du composé précédent dans 10 ml de THF/MeOH (1/1), sont ajoutés 50 mg de palladium sur charbon à 10 %, puis 66 mg de formiate d'ammonium. Après 1 heure sous agitation à TA, le milieu réactionnel est filtré sur talc et le filtrat concentré sous vide. Le résidu est repris dans l'AcOEt, lavé par une solution de chlorure d'ammonium à 10 %, à la saumure, séché sur $MgSO_4$ et concentré sous vide. On chromatographie le résidu sur gel de silice en éluant par le gradient du mélange DCM/MeOH (100/0 ; v/v) à (90/10 ; v/v). On obtient le composé attendu sous forme d'un précipité blanc.

$MH^+$ = 630 ; tr = 6,79 mn ; (M4).

EXEMPLE 9 : Composé N° 59

Chlorohydratede5-chloro-3-(2-fluorophényl)-6-méthoxy-3-[3-(4-méthylpipérazin-1-yl)propyl]-1-{[4-(1,1,2,2-tétrafluoroéthoxy)phényl]sulfonyl}-1,3-dihydro-2*H*-indol-2-one, isomère dextrogyre.

A une solution de 236 mg du composé N° 58 dans 5 ml de DCM, sont ajoutés 29 μl d'une solution aqueuse de formaldéhyde à 40 %, 104 mg de triacétoxyborohydrure de sodium, puis le mélange est agité à TA pendant 2 heures. On hydrolyse le mélange réactionnel par ajout d'une solution aqueuse de bicarbonate de sodium à 10 %, extrait à l'AcOEt, sèche la phase organique sur $MgSO_4$ et évapore le solvant sous vide. Le résidu est repris dans 3 ml DCM, puis on ajoute 0,5 ml d'éther chlorhydrique 2N, puis dilue avec 20 ml d'éther, essore le précipité formé et le sèche.
On obtient le composé attendu.
F = 164° C ; $MH^+$ = 688 ; tr = 7,11 mn ; (M4).

$$\alpha_D^{20} = +148° \text{ (c = 1 ; MeOH).}$$

RMN[1]H : DMSO-d6 (400 MHz) : δ (ppm) : 1,35 : m : 2H ; 2.23-2.33 : mt : 1H ; 2,46-2,51 : mt : 1H ; 2,79 : s : 3H ; 2,90-3,90 : mt : 10H ; 4.01 : s : 3H ; 6.74-6.86 : mt : 1H ; 6,89-7.17 : mt : 1H ; 7,22-7.30 : mt : 1H ; 7,31-7.39 : mt : 1H ; 7,60-7,75 : mt : 4H ; 8.21-8.29 : mt : 2H : 11.2-12.2 : m : 2H.

EXEMPLE 10 : Composé N° 63

Chlorohydratede5-chloro-1-{[3-fluoro-4-(1-méthyléthoxy)phényl]sulfonyl}-3-(2-fluorophényl)-6-méthoxy-3-[3-(4-méthylpipérazin-1-yl)propyl]-1,3-dihydro-2*H*-indol-2-one, isomère dextrogyre.

A une solution de 139 mg du composé N° 62 dans 5 ml de DCM, sont ajoutés 18 μl d'une solution aqueuse de formaldéhyde à 40 %, 65 mg de triacétoxyborohydrure de sodium, puis le mélange est agité à TA pendant 2 heures. On hydrolyse le mélange réactionnel par ajout d'une solution aqueuse de bicarbonate de sodium à 10 %, extrait à l'AcOEt, sèche la phase organique sur $MgSO_4$ et évapore le solvant sous vide. Le résidu est repris dans 3 ml DCM, puis on ajoute 0,5 ml d'éther chlorhydrique 2N, puis dilue avec 20 ml d'éther, essore le précipité formé et le sèche.
On obtient le composé attendu.
F = 188° C ; $MH^+$ = 648 ; tr = 6.68 min ; (M4).

$$\alpha_D^{20} = +145° \text{ (c = 1 ; MeOH).}$$

RMN[1]H : DMSO-d6 (400 MHz) : δ (ppm) : 1,12-1.52 : mt : 8H ; 2.21-2.34 : mt : 1H ; 2,36-2,51 : mt : 1H ; 2,79 : s : 3H ; 2,88-3,90 : mt : 10H ; 4.00 : s : 3H ; 4.82-4.94 : mt : 1H ; 6,86-6.95 : mt : 1H ; 7,14 : s : 1H ; 7,24-7.31 : mt : 1H ; 7,32-7,41 : mt : 1H ; 7.46-7.53 : mt : 1H : 7.62-7.75 : mt : 2H ; 7.79-8.0 : mt : 2H ; 11.1-12.1 : m : 2H.

EXEMPLE 11 : Composé N° 118

3-(2-Fluorophényl)-1-[(4-isopropoxyphényl)sulfonyl]-6-méthoxy-5-méthyl-3-[3-(4-méthylpipérazin-1-yl)propyl]-1,3-dihydroindol-2-one, isomère dextrogyre.

On refroidit à 0° C une solution de 0,135 g du composé de la Préparation 2.29 dans 3,3 ml de THF, ajoute 0,041 g de tert-butylate de potassium puis 0,085 g de chlorure de 4-isopropoxybenzènesulfonyle et laisse agiter 3 heures à 20° C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le mélange DCM/MeOH. On obtient le composé attendu sous forme d'une résine blanche.
F = 70° C ; $MH^+$ = 610 ; tr = 13,23 mn ; (M5).

$$\alpha_D^{20} = +69,6° (c = 0,54 ; AcOEt).$$

RMN[1]H : DMSO-d6 (400 MHz) : δ (ppm) : 0,55-0,65 : m : 2H ; 1,30 : d : 6H ; 2,00-2,40 : m : 18H ; 3,90 : s : 3H ; 4,71-4,78 : mt : 1H ; 6,23 : s : 1H ; 6,96-7,16 : mt : 3H ; 7,24-7,63 : mt : 4H; 7,96-7,98 : mt : 2H

EXEMPLE 12 : Composé N° 119

5-Fluoro-3-(2-fluorophényl)-1-[(4-isopropoxyphényl)sulfonyl]-6-méthoxy-3-[3-(4-méthylpipérazin-1-yl)propyl]-1,3-dihydroindol-2-one, isomère dextrogyre.

A une solution de 203 mg du composé N° 115 dans 3 ml du mélange DCM/MeOH (66/34 ; v/v), refroidie à 0° C, sont ajoutés 30 μl d'une solution aqueuse de formaldéhyde à 40 % et 20 μl d'acide acétique. Après 10 mn d'agitation, à 0° C, on ajoute 192 mg de triacétoxyborohydrure de sodium. Le mélange est agité à TA pendant une nuit. On hydrolyse le mélange réactionnel par ajout d'une solution aqueuse de bicarbonate de sodium à 10 %, extrait à l'AcOEt, sèche la phase organique sur $MgSO_4$ et évapore le solvant sous vide. On chromatographie le résidu sur gel de silice en éluant par le mélange DCM/MeOH. On obtient le composé attendu sous forme d'une résine blanche.
F = 72° C ; $MH^+$ = 614 ; tr = 6,94 mn ; (M4).

$$\alpha_D^{20} = +74° (c = 0,53 ; AcOEt).$$

RMN[1]H : DMSO-d6 (400 MHz) : δ (ppm) : 0,51-0,68 : m : 2H ; 1,30 : d : 6H ; 1,91-2,31 : m : 15H ; 3,96 : s : 3H ; 4,71-4,80 : mt : 1H ; 6,95-8,00 : mt : 10H

EXEMPLE 13 : Composé N° 136

3-[4-({3-[3-(Diméthylamino)propyl]-3-(2-fluorophényl)-6-méthoxy-5-méthyl-2-oxo-2,3-dihydro-1*H*-indol-1-yl} sulfonyl)-2-fluorophényl]-1,1-diéthylurée, isomère dextrogyre.

On refroidit à 0° C une solution de 0,17 g du composé de la Préparation 2.30 dans 5 ml de THF, ajoute 0,059 g de tert-butylate de potassium puis 0,162 g de chlorure de 4-(3,3-diéthyl-uréido)-3-fluoro-benzènesulfonyle et laisse une nuit sous agitation à 20°C. On hydrolyse le mélange réactionnel par ajout d'eau, extrait à l'AcOEt, sèche la phase organique sur $Na_2SO_4$ et évapore le solvant sous vide. On chromatographie le résidu sur gel de silice en éluant par le mélange DCM/MeOH. On obtient le composé attendu sous forme d'une résine blanche.
F = 82° C ; $MH^+$ = 629 ; tr = 6,87 mn ; (M4).

$$\alpha_D^{20} = +86,9° (c = 0,52 ; AcOEt).$$

RMN[1]H : DMSO-d6 (400 MHz) : δ (ppm) : 0,65-0,70 : m : 2H ; 1,10 : t : 6H ; 1,89 : s : 6H ; 2,04 : s : 3H ; 2,11-2,23 : mt : 4H ; 3,34-3,39 : mt : 4H ; 3,91 : s : 3H ; 6,74 : s : 1H ; 6,96-7,36 : mt : 3H; 7,50 : s : 1H ; 7,61-7,96 : 4H ; 8,35 : s : 1H.
Les tableaux qui suivent illustrent les structures chimiques et les propriétés physiques de quelques exemples de composés selon l'invention, obtenus en suivant les modes opératoires décrits dans les Exemples ci-dessus.
Dans ces tableaux :

- Me, Et, iPr, Bu, et tBu représentent respectivement des groupes méthyle, éthyle, isopropyle, n-butyle et *tert*-butyle.

TABLEAU VIII

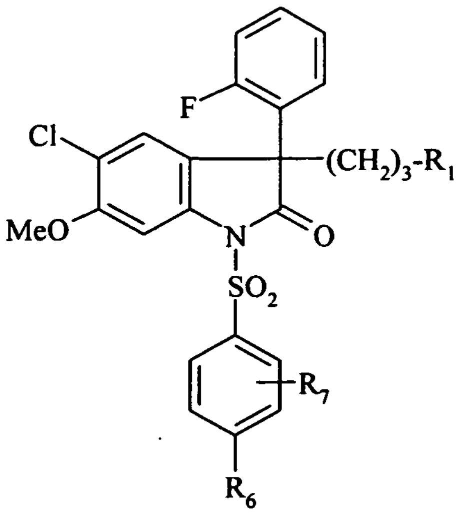

(I) : $R_2$ = Cl
$R_3$ = OMe
$R_4$ = F
$R_5$ = H
X = $(CH_2)_3$

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 1 | 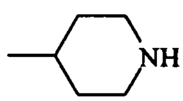 | -OMe | 2-OMe | HCl + 105,7° (c = 1 ; MeOH) | 163°C 617 ; 6,42 (M1) |
| 2 | 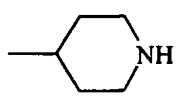 | -OMe | 2-OMe | HCl - 104,5° (c = 1 ; MeOH) | 175°C 617 ; 6,42 (M1) |
| 3 | -N $(Me)_2$ | -OiPr | H | HCl + 88,1° (c = 1 ; AcOEt) | 173°C 575 ; 9,58 (M2) |
| 4 | 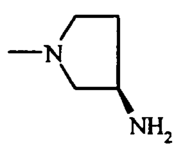 | -OiPr | H | HCl + 122° (c = 1 ; MeOH) | 215°C 616 ; 5,98 (M2) |
| 5 | 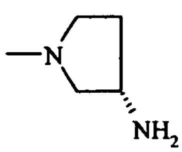 | -OiPr | H | HCl + 116,3° (c = 1 ; MeOH) | 206°C 616 ; 6,24 (M4) |
| 6 | 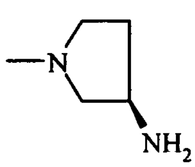 | -OEt | 3-OMe | HCl + 120,2° (c = 1 ; MeOH) | 210°C 632 ; 8,50 (M2) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 7 | 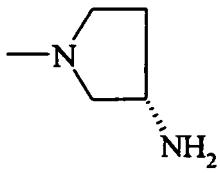 | -OEt | 3-OMe | HCl + 110,2° (c = 1 ; MeOH) | 238°C 632 ; 5,88 (M1) |
| 8 | 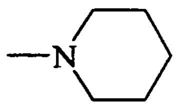 | -OMe | 2-OMe | Base + 88° (c = 1 ; AcOEt) | 105°C 617 ; 6,59 (M1) |
| 9 | 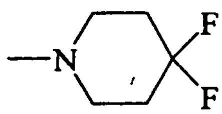 | -OEt | 3-OMe | Base + 61,5° (c = 1 ; AcOEt) | 88°C 667 ; 7,26 (M1) |
| 10 | 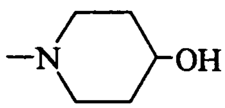 | -OEt | 3-OMe | Base + 60,8° (c = 1 ; AcOEt) | 102°C 647 ; 6,64 (M1) |
| 11 | 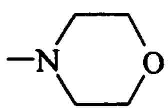 | -OEi | 3-OMe | Base + 64,8° (c = 1 ; AcOEt) | 153°C 633 ; 6,65 (M1) |
| 12 | 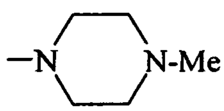 | -OMe | 2-OMe | Base + 71,6° (c = 1 ; AcOEt) | 123°C 632 ; 6,90 (M1) |
| 13 | 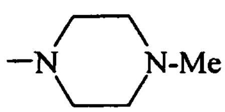 | -OiPr | H | HCl + 114,8° (c = 1 ; MeOH) | 205°C 630 ; 7,09 (M1) |
| 14 | 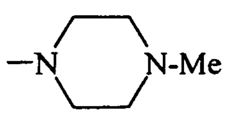 | -OEt | 3-OMe | HCl + 106,2° (c = 1 ; MeOH) | 180°C 646 ; 6,76 (M1) |
| 15 | 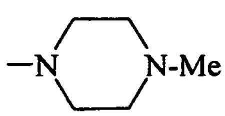 | -OMe | 3-OMe | Base + 60,3° (c = 1 ; AcOEt) | 90°C 632 ; 6,44 (M1) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 16 | 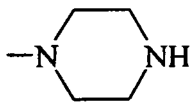 | -OMe | 2-OMe | HCl + 122° (c = 1 ; MeOH) | 243°C 618 ; 6,38 (M1) |
| 17 | 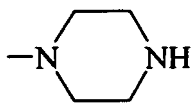 | -OiPr | H | HCl + 120,8° (c = 1 ; MeOH) | 232°C 616 ; 6,51 (M1) |
| 18 | 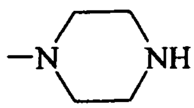 | -OEt | 3-OMe | HCl + 114,2° (c = 1 ; MeOH) | 220°C 632 ; 6,51 (M1) |
| 19 | 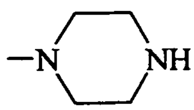 | -OMe | 3-OMe | HCl + 111,3° (c = 1 ; MeOH) | 190°C 618 ; 6,57 (M1) |
| 20 | 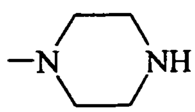 | -Me | 3-Me | HCl + 123,7° (c = 1 ; MeOH) | 192°C 586 ; 6,40 (M1) |
| 21 | 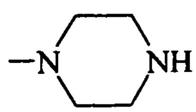 | -OMe | 3-Me | HCl + 132,7° (c = 1 ; MeOH) | 234°C 602 ; 6,53 (M2) |
| 22 | 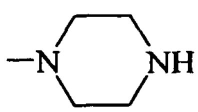 | -OEt | H | HCl + 123,5° (c = 1 ; MeOH) | 213°C 602 ; 8,94 (M2) |
| 23 | 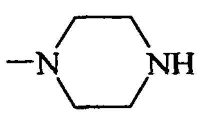 | -OEt | 3-Me | HCl + 142,6° (c = 1 ; MeOH) | 226°C 616 ; 6,84 (M2) |
| 24 | 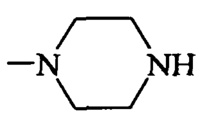 | $-OCHF_2$ | H | HCl + 121,8° (c = 1 ; MeOH) | 218°C 624 ; 6,45 (M1) |

(suite)

| Composés N° | $R_1$ | $R_6$ | $R_7$ | Sels Base $\alpha_D^{20}$ (c ; solvant) | F°C $MH^+$ ; tr (conditions) |
|---|---|---|---|---|---|
| 25 | 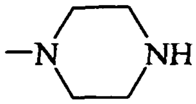 | $-OCF_3$ | H | HCl + 135° (c = 1 ; MeOH) | 220°C 642 ; 6,86 (M1) |
| 26 | 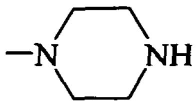 | H | H | HCl + 123,5° (c = 1 ; MeOH) | 238°C 558 ; 6,58 (M1) |
| 27 | 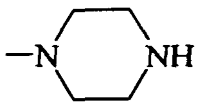 | $R_6 + R_7 = -(CH_2)_3-$ | | HCl + 129° (c = 1 ; MeOH) | 242°C 598 ; 7,06 (M1) |
| 28 | 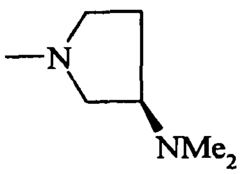 | -OMe | 3-Me | Base + 71,2° (c = 0,5 ; MeOH) | 76°C 630 ; 5,62 (M4) |
| 29 | 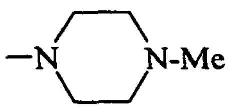 | -OMe | 3-Me | Base + 67,3° (c = 0,53 ; MeOH) | 96°C 616 ; 6,18 (M4) |
| 30 | 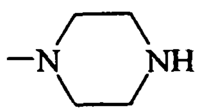 | -OMe | 3-F | HCl + 121,8° (c = 0,5 ; MeOH) | 170°C 606 ; 6,49 (M4) |
| 31 | 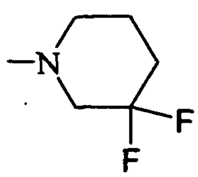 | $-NHCO-NEt_2$ | 3-Me- | Base + 52°° (c = 1 ; MeOH) | 150°C 721 ; 7,77 (M1) |
| 32 | $-N(Me)_2$ | $-NHCO-NEt_2$ | 3-Me | Base + 65° (c = 1 ; MeOH) | 122°C 645 ; 7,03 (M1) |
| 33 | 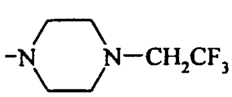 | -OiPr | H | HCl + 114° (c = 1 ; MeOH) | 272°C 698 ; 7.71 (M4) |

(suite)

| Composés N° | $R_1$ | $R_6$ | $R_7$ | Sels Base $\alpha_D^{20}$ (c ; solvant) | F°C $MH^+$ ; tr (conditions) |
|---|---|---|---|---|---|
| 34 | 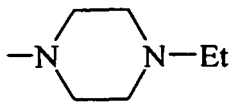 | -OiPr | H | HCl + 131° (c = 1 ; MeOH) | 316°C 644 ; 7.03 (M4) |
| 35 | 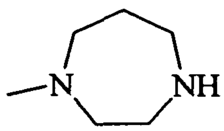 | -OiPr | H | HCl + 153° (c = 1 ; MeOH) | 175°C 630 ; 6.24 (M4) |
| 36 | 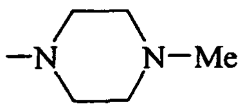 | -OMe | H | HCl + 125° (c = 1 ; MeOH) | - 602 ; 6.63 (M4) |
| 37 | 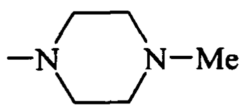 | H | 3-Cl | HCl + 151° (c = 1 ; MeOH) | 228°C 606 ; 6.91 (M4) |
| 38 | 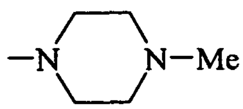 | H | 3-Ome | HCl + 132° (c = 1 ; MeOH) | 147°C 602 ; 6.14 (M4) |
| 39 | 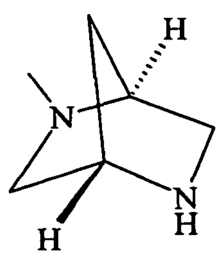 | -OiPr | H | HCl + 160° (c = 1 ; MeOH) | 200°C 628 ; 6.38 (M4) |
| 40 | 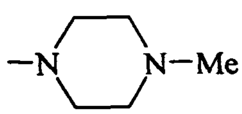 | -iPr | H | HCl + 143° (c = 1 ; MeOH) | 173°C 614 ; 6.70 (M4) |
| 41 | 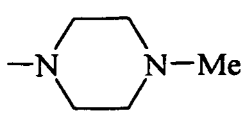 | -OH | H | - + 69° (c = 1 ; MeOH) | 169°C 588 ; 15.3 (M2) |

(suite)

| Composés N° | $R_1$ | $R_6$ | $R_7$ | Sels Base $\alpha_D^{20}$ (c ; solvant) | F°C $MH^+$ ; tr (conditions) |
|---|---|---|---|---|---|
| 42 | 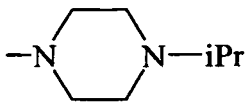 | -OiPr | H | HCl + 146° (c = 1 ; MeOH) | 194°C 658 ; 6.76 (M4) |
| 43 | 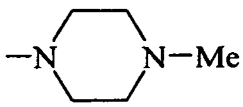 | -Obu | H | HCl + 129° (c = 1 ; MeOH) | 156°C 644 ; 14.4 (M5) |
| 44 | 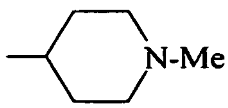 | -OiPr | H | HCl + 109° (c = 1 ; MeOH) | 160°C 629; 15.1 (M5) |
| 45 | 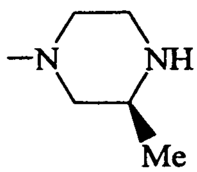 | -OiPr | H | HCl + 137° (c = 1 ; MeOH) | 190°C 630 ; 6.46 (M4) |
| 46 | 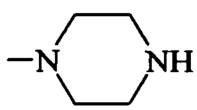 | -OH | H | HCl +97° (c = 1 ; DMF) | 292°C 574 ; 6.21 (M4) |
| 47 | 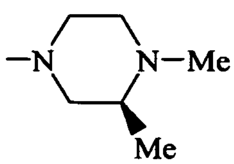 | -OiPr | H | HCl + 135° (c = 1 ; MeOH) | 203°C 644 ; 7.17 (M4) |
| 48 | 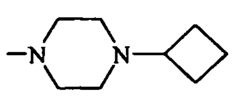 | -OiPr | H | HCl + 127° (c = 1 ; MeOH) | 182°C 670 ; 7.35 (M4) |
| 49 | 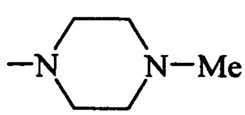 | 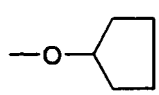 | H | HCl + 160° (c = 1 ; MeOH) | 211°C 656 ; 7.36 (M4) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 50 | 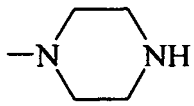 | H | 3-Cl | HCl + 175° (c = 1 ; MeOH) | 193°C 592 ; 6.13 (M4) |
| 51 | 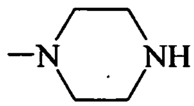 | -H | 3-OMe | HCl + 156° (c = 1 ; MeOH) | 161°C 588 ; 5.92 (M4) |
| 52 | 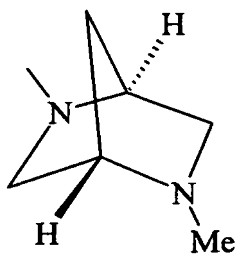 | -OiPr | H | HCl + 151° (c = 1 ; MeOH) | 184°C 642 ; 6.54 (M4) |
| 53 | 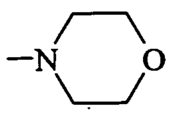 | -OiPr | H | HCl + 148° (c = 1 ; MeOH) | 154°C 617 ; 6.92 (M4) |
| 54 | 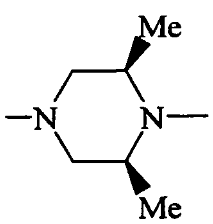 | -OiPr | H | HCl + 121° (c = 1 ; MeOH) | 177°C 658 ; 7.17 (M4) |
| 55 | 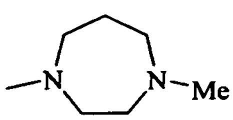 | -OiPr | H | HCl + 145° (c = 1 ; MeOH) | 202°C 644 ; 6.32 (M4) |
| 56 | 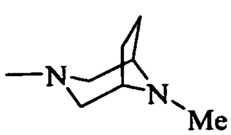 | -OiPr | H | HCl + 118° (c = 1 ; MeOH) | 200°C 656 ; 7.34 (M4) |
| 57 | 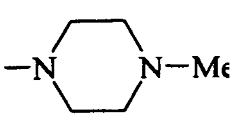 | -OEt | H | HCl + 126° (c = 1 ; MeOH) | 211°C 616 ; 6.41 (M4) |

(suite)

| Composés N° | $R_1$ | $R_6$ | $R_7$ | Sels Base $\alpha_D^{20}$ (c ; solvant) | F°C MH$^+$ ; tr (conditions) |
|---|---|---|---|---|---|
| 58 | 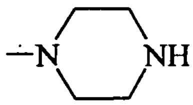 | $-OCF_2CHF_2$ | H | HCl + 135° (c = 1 ; MeOH) | 199°C 674 ; 6.40 (M4) |
| 59 | 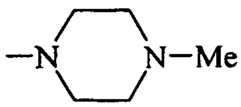 | $-OCF_2CHF_2$ | H | HCl + 148° (c = 1 ; MeOH) | 164°C 688 ; 6.66 (M4) |
| 60 | 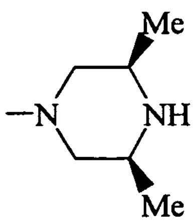 | -OiPr | H | HCl + 130° (c = 1 ; MeOH) | 219°C 644 ; 6.55 (M4) |
| 61 | 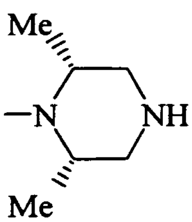 | -OiPr | H | HCl + 147° (c = 1 ; MeOH) | 226°C 644 ; 6.70 (M4) |
| 62 | 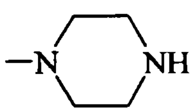 | -OiPr | 3-F | HCl + 126° (c = 1 ; MeOH) | 174°C 634 ; 6.39 (M4) |
| 63 | 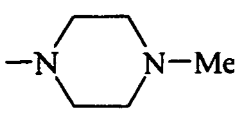 | -OiPr | 3-F | HCl + 145° (c = 1 ; MeOH) | 207°C 648 ; 6.66 (M4) |
| 64 | 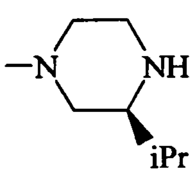 | -OiPr | H | HCl + 123° (c = 1 ; MeOH) | 196°C 658 ; 6.72 (M4) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 65 | 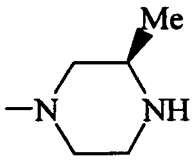 | -OiPr | H | HCl + 148° (c = 1 ; MeOH) | 203°C 630 ; 6.49 (M4) |
| 66 | 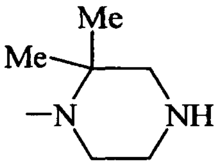 | -OiPr | H | HCl + 122° (c = 1 ; MeOH) | 203°C 644 ; 6.52 (M4) |
| 67 | 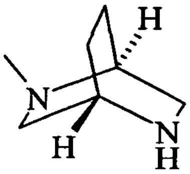 | -OiPr | H | HCl + 151° (c = 1 ; MeOH) | 210°C 642 ; 5.98 (M4) |
| 68 | 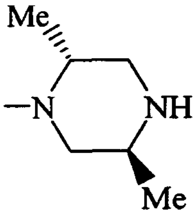 | -OiPr | H | HCl + 144° (c = 1 ; MeOH) | 218°C 644 ; 6.48 (M4) |
| 69 | 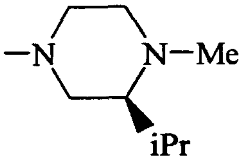 | -OiPr | H | HCl + 133° (c = 1 ; MeOH) | 188°C 672 ; 7.29 (M4) |
| 70 | 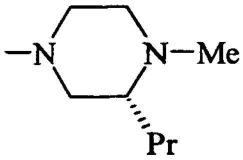 | -OiPr | H | HCl + 134° (c = 1 ; MeOH) | 173°C 672 ; 7.40 (M4) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 71 | 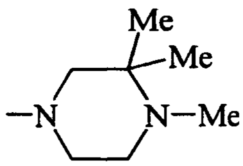 | -OiPr | H | HCl + 131° (c = 1 ; MeOH) | 195°C 658 ; 7.36 (M4) |
| 72 | 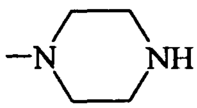 | -OtBu | H | - + 90° (c = 1 ; DMF) | 630 ; 6.79 (M4) |
| 73 | 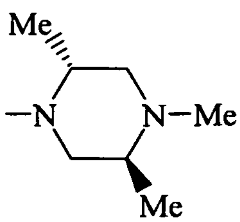 | -OiPr | H | HCl + 130° (c = 1 ; MeOH) | 201°C 658; 7.18 (M4) |
| 74 | 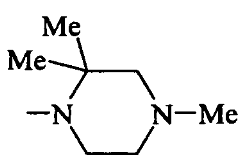 | -OiPr | H | HCl + 138° (c = 1 ; MeOH) | - 658 ; 6.86 (M4) |
| 75 | 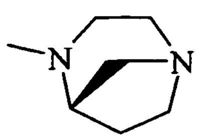 | -OiPr | H | HCl + 166° (c = 1 ; MeOH) | - 642 ; 5.79 (M4) |
| 76 | 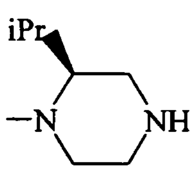 | -OiPr | H | HCl + 136° (c = 1 ; MeOH) | - 658 ; 6.73 (M4) |
| 77 | 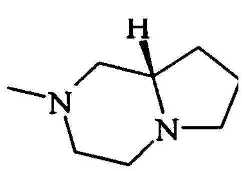 | -OiPr | H | HCl + 128° (c = 1 ; MeOH) | - 656 ; 6.76 (M4) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 78 | 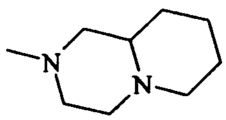 | -OiPr | H | HCl + 136° (c = 1 ; MeOH) | - 670 ; 6.80 (M4) |
| 79 | 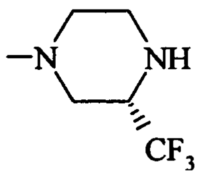 | -OiPr | H | HCl + 121° (c = 1 ; MeOH) | - 684 ; 7.55 (M4) |
| 80 | 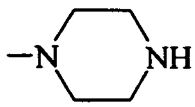 | -OiPr | 3-Cl | HCl + 153° (c = 1 ; MeOH) | - 650 ; 6.57 (M4) |
| 81 | 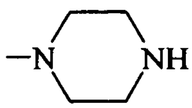 | -OiPr | 2-OMe | HCl + 122° (c = 1 ; MeOH) | - 646; 6.60 (M4) |
| 82 | 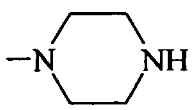 | -OiPr | 3-OMe | HCl + 128° (c = 1 ; MeOH) | - 646 ; 6.61 (M4) |
| 83 | 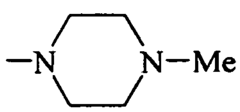 | -OiPr | 3-$CF_3$ | HCl + 112° (c = 1 ; MeOH) | - 698 ; 7.48 (M4) |
| 84 | 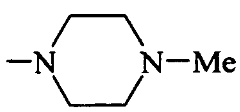 | -OiPr | 3-OMe | HCl + 118° (c = 1 ; MeOH) | - 660; 6.81 (M4) |
| 85 | 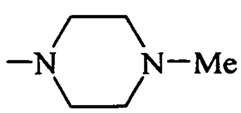 | -OiPr | 3-Me | HCl + 121° (c = 1 ; MeOH) | - 644 ; 7.30 (M4) |

(suite)

| Composés N° | $R_1$ | $R_6$ | $R_7$ | Sels Base $\alpha_D^{20}$ (c ; solvant) | F°C $MH^+$ ; tr (conditions) |
|---|---|---|---|---|---|
| 86 | 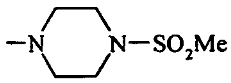 | -OiPr | H | HCl<br>+ 124°<br>(c = 1 ; MeOH) | -<br>694 ; 7.47<br>(M4) |
| 87 | 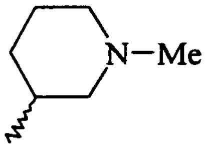 | -OiPr | H | HCl<br>+ 117°<br>(c = 1 ; MeOH) | -<br>629 ; 7.52<br>(M4) |
| 88 | 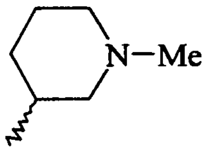 | -OiPr | H | HCl<br>+ 132°<br>(c = 1 ; MeOH) | -<br>629; 7.48<br>(M4) |
| 89 | 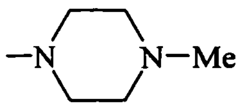 | $-OCHF_2$ | H | HCl<br>+ 116°<br>(c = 1 ; MeOH) | -<br>638 ; 6.84<br>(M4) |
| 90 | 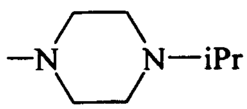 | $-OCF_2CHF_2$ | H | HCl<br>+ 121°<br>(c = 1 ; MeOH) | -<br>716; 7.25<br>(M4) |
| 91 | 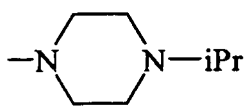 | -OiPr | 3-F | HCl<br>+ 124°<br>(c = 1 ; MeOH) | -<br>676; 7.25<br>(M4) |
| 92 | 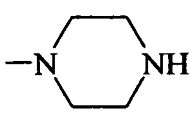 | -Oet | 3-F | HCl<br>+ 126°<br>(c = 1 ; MeOH) | -<br>620 ; 6.65<br>(M4) |
| 93 | 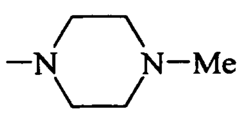 | -OEt | 3-F | HCl<br>+ 110°<br>(c = 1 ; MeOH) | -<br>634 ; 6.92<br>(M4) |

(suite)

| Composés N° | $R_1$ | $R_6$ | $R_7$ | Sels Base $\alpha_D^{20}$ (c ; solvant) | F°C MH+ ; tr (conditions) |
|---|---|---|---|---|---|
| 94 | 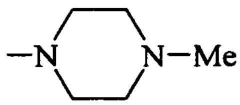 | -OtBu | H | - <br> + 56° <br> (c = 1 ; DMF) | - <br> 644 ; 7.19 <br> (M4) |
| 94a | 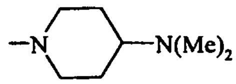 | -OiPr | H | HCl <br> +142° <br> (c = 1 ; MeOH) | - <br> 658 ; 6,05 <br> (M4) |

TABLEAU IX

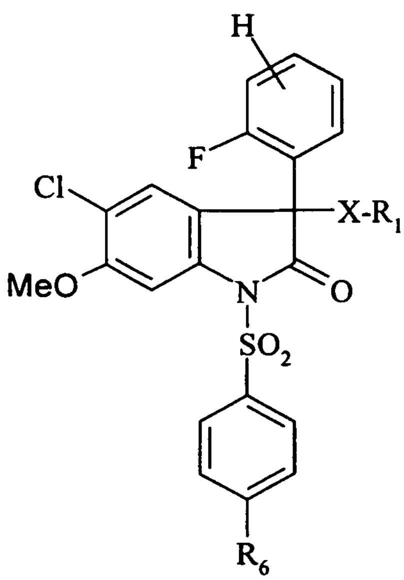

(I) : $R_2$ = Cl
$R_3$ = OMe
$R_4$ = F
$R_5$ = H
$R_7$ = H

| Composés N° | $R_1$ | X | $R_6$ | Sels / Base $\alpha_D^{20}$ (c ; solvant) | F°C MH+ ; tr (conditions) |
|---|---|---|---|---|---|
| 95 | 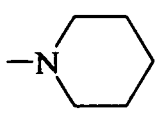 | $(CH_2)_4$ | -OiPr | HCl <br> + 111° <br> (c = 1 ; MeOH) | 140°C <br> 629 ; 7,52 <br> (M4) |
| 96 | 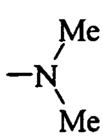 | $(CH_2)_5$ | -OiPr | HCl <br> + 128° <br> (c = 1 ; MeOH) | 125°C <br> 603 ; 7,43 <br> (M4) |

(suite)

| Composés N° | $R_1$ | X | $R_6$ | Sels / Base $\alpha_D^{20}$ (c ; solvant) | F°C $MH^+$ ; tr (conditions) |
|---|---|---|---|---|---|
| 97 | 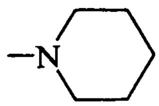 | $(CH_2)_5$ | -OiPr | HCl + 116° (c = 1 ; MeOH) | 203°C 643 ; 7,16 (M4) |
| 98 | 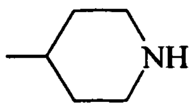 | $(CH_2)_2$ | -OiPr | HCl + 102° (c = 1 ; MeOH) | 162°C 601 ; 7,41 (M4) |
| 99 | 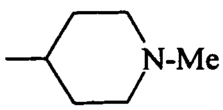 | $(CH_2)_2$ | -OiPr | HCl + 112° (c = 1 ; MeOH) | 162°C 615 ; 6,89 (M4) |
| 100 | 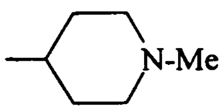 | $CH_2$ | -OiPr | HCl + 139° (c = 1 ; MeOH) | 189°C 601 ; 7,19 (M4) |
| 101 | 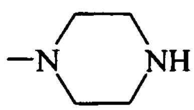 | $(CH_2)_2$ | -OiPr | HCl + 186° (c = 1 ; MeOH) | 188°C 602 ; 6,68 (M4) |
| 102 | 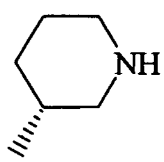 | $CH_2$ | -OiPr | HCl + 202° (c = 1 ; MeOH) | - 587 ; 13,9 (M5) |
| 103 | 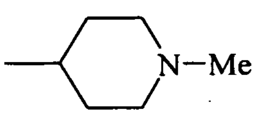 | $(CH_2)_2$ | $-OCF_2CHF_2$ | HCl + 104° (c = 1 ; MeOH) | - 673 ; 14,9 (M5) |

TABLEAU X

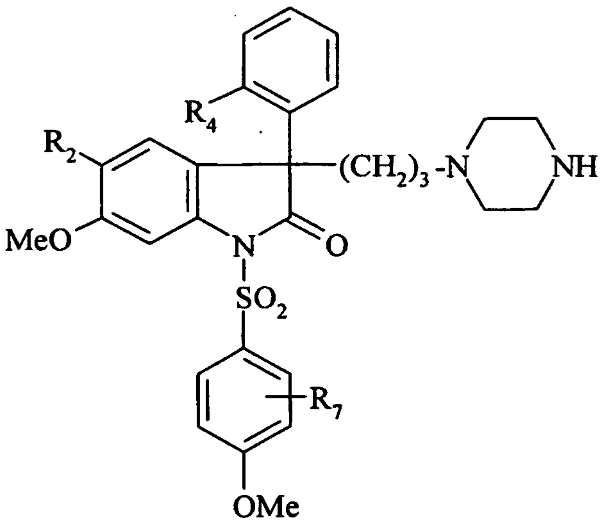

(I) : $R_1$ = 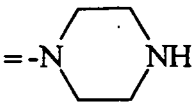

$R_3$ = OMe
$R_5$ = H
$R_6$ = OMe
X = $(CH_2)_3$

| Composés N° | $R_2$ | $R_4$ | $R_7$ | Sels / Base $\alpha_D^{20}$ (c ; solvant) | F°C MH+ ; tr (conditions) |
|---|---|---|---|---|---|
| 104 | Cl | Me | 3-Me | HCl + 147,6° (c = 1 ; MeOH) | 172°C 598 ; 6,32 (M4) |
| 105 | Cl | H | 3-Me | HCl + 60,4° (c = 0.52 ; MeOH) | 170°C 584 ; 6,12 (M4) |
| 106 | Me | F | 3-Me | HCl + 120,6° (c = 0,5 ; MeOH) | 164°C 582 ; 6,50 (M4) |
| 107 | F | F | 3-Me | HCl + 113,6° (c = 0,5 ; MeOH) | 152°C 586 ; 6,38 (M4) |
| 108 | OMe | F | 3-Me | HCl + 96,3° (c = 0,51 ; MeOH) | 160°C 598 ; 6,16 (M4) |
| 109 | Cl | Me | 2-OMe | HCl + 131° (c = 1 ; MeOH) | 184°C 614 ; 5,96 (M4) |
| 110 | Cl | H | 2-OMe | HCl + 26,1° (c = 0,51 ; MeOH) | 174°C 600; 11,1 (M5) |
| 111 | Me | F | 2-OMe | HCl + 117,2° (c = 0,53 ; MeOH) | 222°C 598 ; 6,16 (M4) |
| 112 | F | F | 2-OMe | HCl + 105,4° (c = 0,5 ; MeOH) | 178°C 602 ; 6,05 (M4) |

(suite)

| Composés N° | $R_2$ | $R_4$ | $R_7$ | Sels / Base $\alpha_D^{20}$ (c ; solvant) | F°C<br>$MH^+$ ; tr<br>(conditions) |
|---|---|---|---|---|---|
| 113 | OMe | F | 2-OMe | HCl<br>+ 89,8°<br>(c = 0,53 ; MeOH) | 228°C<br>614 ; 5,73<br>(M4) |

TABLEAU XI

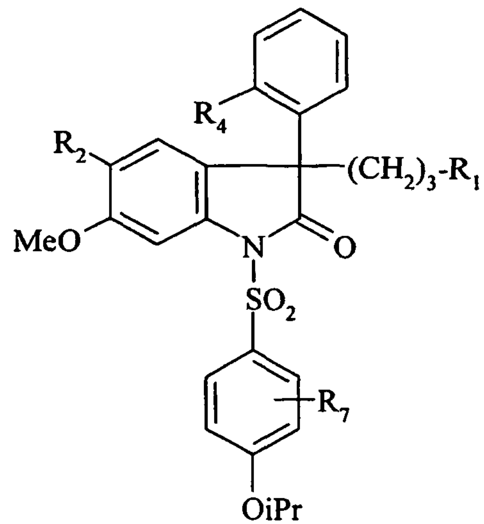

(I) : $R_3$ = OMe
$R_5$ = H
$R_6$ = OiPr
X = $(CH_2)_3$

| Composés N° | $R_1$ | $R_2$ | $R_4$ | $R_7$ | Sels/Base $\alpha_D^{20}$ (c ; solvant) | F°C MH+; tr (conditions) |
|---|---|---|---|---|---|---|
| 114 | 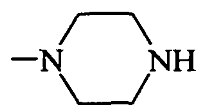 | Cl | H | H | HCl + 61,6° (c = 0,5 ; MeOH) | 204°C 599 ; 6,33 (M4) |
| 115 | 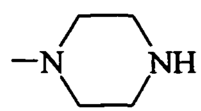 | F | F | H | HCl + 112,9° (c = 0,54 ; MeOH) | 162°C 600 ; 6,59 (M4) |

(suite)

| Composés N° | $R_1$ | $R_2$ | $R_4$ | $R_7$ | Sels/Base $\alpha_D^{20}$ (c ; solvant) | F°C $MH^+$; tr (conditions) |
|---|---|---|---|---|---|---|
| 116 | 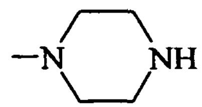 | OMe | F | H | HCl + 93,2° (c = 0,51 ; MeOH) | 164°C 612 ; 6,26 (M4) |
| 117 | 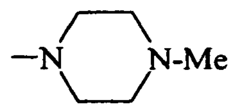 | Cl | H | H | Base - 39,7° (c = 1 ; MeOH) | 97°C 612 ; 7,04 (M4) |
| 118 | 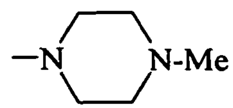 | Me | F | H | Base + 69,6° (c = 0,54 ; AcOEt) | 70°C 610 ; 13,23 (M5) |
| 119 | 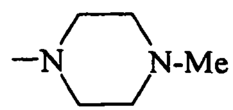 | F | F | H | Base + 74° (c = 0,53 ; AcOEt) | 72°C 614 ; 6,94 (M4) |
| 120 | 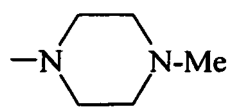 | OMe | F | H | Base + 63,8° (c = 0,5 ; AcOEt) | 80°C 626 ; 6,68 (M4) |
| 121 | 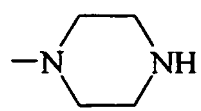 | F | F | 3-F | HCl + 109° (c = 0,51 ; MeOH) | 152°C 618 ; 6,83 (M4) |

(suite)

| Composés N° | $R_1$ | $R_2$ | $R_4$ | $R_7$ | Sels/Base $\alpha_D^{20}$ (c ; solvant) | F°C MH+; tr (conditions) |
|---|---|---|---|---|---|---|
| 122 | 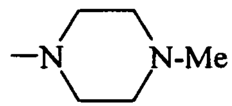 | Cl | H | 3-F | Base<br>- 60°<br>(c = 0,5 ; AcOEt) | 93°C<br>629 ; 7,37<br>(M4) |
| 123 | 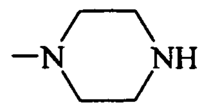 | Br | F | H | -<br>+ 151°<br>(c = 1 ; DMF) | -<br>660 ; 6,82<br>(M4) |
| 124 | 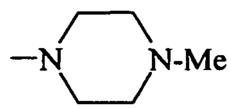 | Br | F | H | -<br>+ 144°<br>(c = 1 ; DMF) | -<br>674 ; 7,12<br>(M4) |
| 124a | 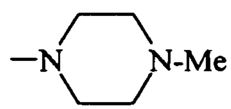 | OMe | F | 3-F | Base<br>+71,2°<br>(c = 0,5 ; MeOH) | 74°C<br>644 ; 6,57<br>(M4) |
| 124b | 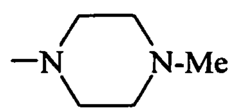 | F | F | 3-F | Base<br>+78°<br>(c = 0,53 ; MeOH) | 72°C<br>632 ; 6,92<br>(M4) |

TABLEAU XII

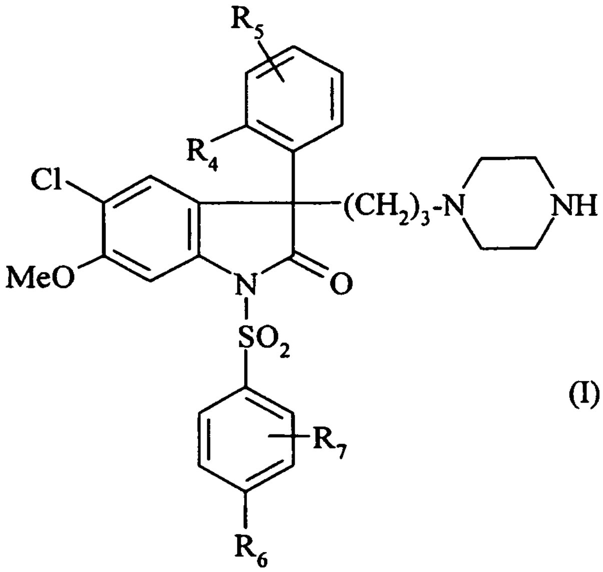

(I) : $R_1$ = 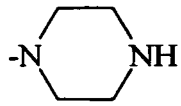

$R_2$ = Cl
X = $(CH_2)_3$

| Composés N° | $R_4$ | $R_5$ | $R_6$ | $R_7$ | Sels Base $\alpha_D^{20}$ (c ; solvant) | F°C MH+ ; tr (conditions) |
|---|---|---|---|---|---|---|
| 125 | F | 5-F | -OMe | 3-Me | HCl + 137,3° (c = 0.52 ; MeOH) | 172°C 620 ; 6,23 (M4) |
| 126 | F | 3-Cl | -OMe | 3-Me | HCl + 136° (c = 0.5 ; MeOH) | 174°C 636 ; 6,82 (M4) |
| 127 | F | 6-F | -OiPr | H | HCl + 161,5° (c = 1 ; MeOH) | 182°C 634 ; 6,78 (M4) |
| 128 | F | 3-Cl | -OiPr | H | HCl + 135° (c = 0,51 ; MeOH) | 170°C 650 ; 7,02 (M4) |
| 129 | F | 5-F | -OMe | 2-OMe | HCl + 117,6° (c = 0,5 ; MeOH) | 174°C 636 ; 5,91 (M4) |
| 130 | F | 3-Cl | -OMe | 2-OMe | HCl + 111,2° (c = 0,51 ; MeOH) | 174°C 652 ; 6,50 (M4) |

TABLEAU XIII

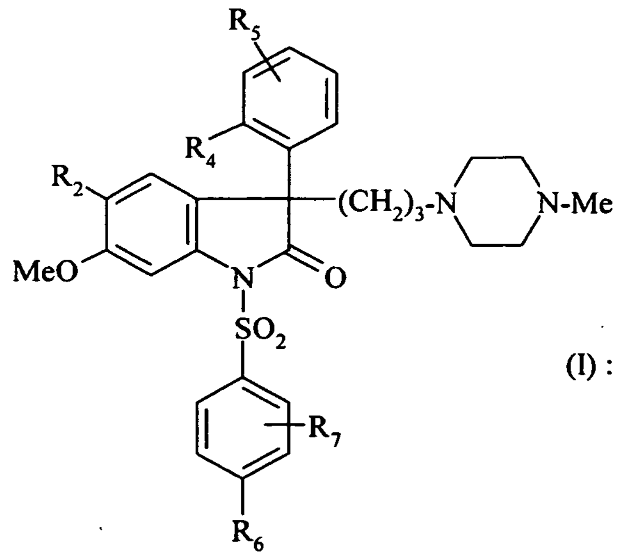

(I) : $R_1$ = 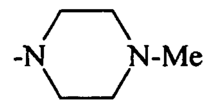

X = $(CH_2)_3$

| Composés N° | $R_2$ | $R_4$ | $R_5$ | $R_6$ | $R_7$ | Sels Base $\alpha_D^{20}$ (c ; solvant) | F°C $MH^+$ ; tr (conditions) |
|---|---|---|---|---|---|---|---|
| 131 | Me | F | H | -OMe | 3-Me | Base + 65° (c = 0,53 ; AcOEt) | 86°C 596 ; 12,5 (M5) |
| 132 | Cl | F | 3-Cl | -OMe | 3-Me | Base +78,2° (c=0,51;MeOH) | 86°C 650 ; 7,2 (M4) |
| 133 | Me | F | H | -OEt | 3-Me | Base + 61,1° (c = 0,54 ; AcOEt) | 74°C 610 ; 13,14 (M5) |

(suite)

| Composés N° | $R_2$ | $R_4$ | $R_5$ | $R_6$ | $R_7$ | Sels Base $\alpha_D^{20}$ (c ; solvant) | F°C MH+ ; tr (conditions) |
|---|---|---|---|---|---|---|---|
| 134 | Cl | H | H | $-OCF_2-CHF_2$ | H | Base - 42° (c = 0,5 ; AcOEt) | 80°C 670 ; 7,17 (M4) |
| 134a | OMe | F | H | -OMe | 3-Me | Base +61,2° (c = 0,5 ; MeOH) | 78°C 612 ; 6,05 (M 4) |
| 134b | Cl | F | $5-CO_2Me$ | -OiPr | H | Base + 96,8° (c = 0,5 ; MeOH) | 100°C 688 ; 13,37 (M 5) |
| 134c | Cl | F | $5-CH_2OH$ | -OiPr | H | Base + 88,2° (c = 0,5 ; MeOH) | 97°C 660 ; 12,78 (M 5) |

TABLEAU XIV

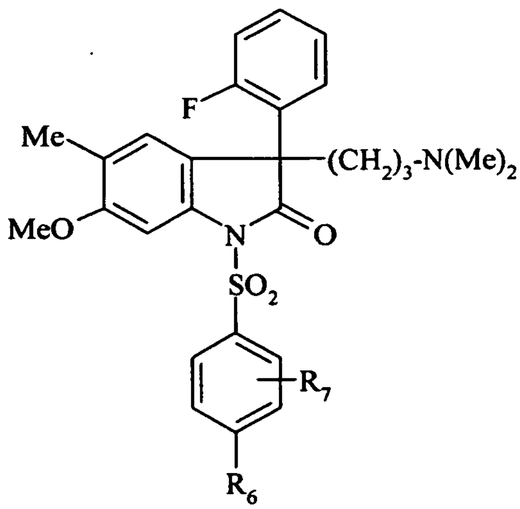

(I) : $R_1$ = -N(Me)$_2$
$R_2$ = Me
$R_3$ = OMe
$R_4$ = F
$R_5$ = H
X = $(CH_2)_3$

| Composés N° | $R_6$ | $R_7$ | Sels Base $\alpha_D^{20}$ (c ; solvant) | F°C MH$^+$ ; tr (conditions) |
|---|---|---|---|---|
| 135 | -NHCO- $NEt_2$ | 3-Me | Base +68,2° (c = 0,54 ; AcOEt) | 98°C 625 ; 6,78 (M4) |
| 136 | -NHCO- $NEt_2$ | 3-F | Base +86,9° (c = 0,52 ; AcOEt) | 82°C 629 ; 6,87 (M4) |

Les composés selon l'invention ont fait l'objet d'essais pharmacologiques.

L'affinité des composés de formule (I) selon l'invention pour les récepteurs $V_{1a}$ de l'arginine-vasopressine a été déterminée *in vitro* en utilisant la méthode décrite par M. Thibonnier et al., J. Biol. Chem., 1994, 269, 3304-3310. Cette méthode consiste à étudier *in vitro* le déplacement de l'arginine-vasopressine tritiée ([$^3$H]-AVP) aux récepteurs $V_{1a}$ présents sur des préparations membranaires ou cellulaires portant les récepteurs $V_{1a}$ de rat ou humains. Les composés de formule (I) présentent une affinité pour les récepteurs $V_{1a}$ humains de l'arginine-vasopressine avec des $CI_{50}$ (concentration inhibitrice de 50 % de la liaison de l'arginine-vasopressine tritiée ([$^3$H]-AVP à ses récepteurs)) généralement inférieures à 10 nanomolaires ($10^{-8}$ M) dans ce dernier test.

L'affinité des composés de formule (I) selon l'invention pour les récepteurs $V_{1b}$ de l'arginine-vasopressine a été déterminée *in vitro* en utilisant la méthode décrite par Y. de Keyser et al. dans Febs Letters, 1994, 356, 215-220. Cette méthode consiste à étudier *in vitro* le déplacement de l'arginine-vasopressine tritiée ([$^3$H]-AVP) aux récepteurs $V_{1b}$ présents sur des préparations cellulaires portant les récepteurs $V_{1b}$ humains. Certains composés étudiés présentent en plus une affinité pour les récepteurs $V_{1b}$ humains avec des $CI_{50}$ inférieures à 30 nanomolaires ($3.10^{-8}$ M) dans ce dernier test.

L'affinité des composés de formule (I) selon l'invention pour les récepteurs $V_2$ de la vasopressine a également été étudiée (méthode décrite par M. Birnbaumer et al. dans Nature (Lond.), 1992, 357, 333-335). Les composés étudiés sont peu ou pas affins pour les récepteurs $V_2$ humains avec des $CI_{50}$ supérieures à $10^{-7}$ M.

L'affinité des composés selon l'invention pour les récepteurs de l'ocytocine a été déterminée dans un test de liaison *in vitro* en utilisant la méthode décrite par J. Elands et al. dans Eur. J. Pharmacol., 1987, 147, 197-207. Cette méthode consiste à étudier *in vitro* le déplacement d'un analogue radioiodé de l'ocytocine aux récepteurs de l'ocytocine dans une préparation membranaire de cellules transfectée avec le récepteur ocytocique humain utérin.

Les composés étudiés sont peu ou pas affins pour les récepteurs humains de l'ocytocine avec des $CI_{50}$ généralement supérieures à $10^{-7}$ M.

Le TABLEAU XV qui suit illustre les résultats pharmacologiques comparatifs des composés N° 17, 38, 46, 107 et 108 selon l'invention avec les composés $\alpha$ et $\beta$ de l'art antérieur sur les différents tests *in-vitro* mesurant l'affinité aux récepteurs $V_{1a}$, $V_{1b}$, $V_2$ et ocytocine humains. Les résultats sont exprimés par la concentration inhibitrice de 50 % ($CI_{50}$)

nanomolaire (nM).

TABLEAU XV

| | BINDING | | | |
|---|---|---|---|---|
| | $V_{1a}$ humains $CI_{50}$ (nM) | $V_{1b}$ humains $CI_{50}$ (nM) | $V_2$ humains $CI_{50}$ (nM) | Ocytocine humains $CI_{50}$ (nM) |
| Composé α | 23 | 210 | 19 | 100 |
| Composé β | 18 | 180 | 13 | 69 |
| Composé N°17 | 2,4 | >1000 | 450 | 390 |
| Composé N°38 | 1,1 | >1000 | >1000 | 500 |
| Composé N°46 | 2,4 | 640 | >1000 | 380 |
| Composé N°107 | 8,5 | 140 | >1000 | >1000 |
| Composé N° 108 | 5 | 320 | >1000 | >1000 |

Le caractère agoniste ou antagoniste des composés est déterminé *in vitro* dans un test de mesure de calcium intracellulaire (FLIPR) sur cellules exprimant les récepteurs V1a humains selon la technique générale décrite dans Sullivan et al, Methods Mol. Biol., 1999, 114, 125-133, en utilisant 1 μM de Fluo4 AM et dans un test d'agrégation plaquettaire induite à l'AVP sur PRP (plasma riche en plaquettes) humain selon la méthodologie décrite dans J. Clin. Invest., 1993, 92, 224-231. Les composés sont préincubés 30 minutes avant l'addition de l'arginine vasopressine afin de déterminer les propriétés agonistes et antagonistes de ces molécules. Les $CI_{50}$ des composés selon l'invention pour les récepteurs $V_{1a}$ mesurées dans ces études sont faibles (inférieures à $2.10^{-8}$ M).

Ces résultats pharmacologiques montrent que les composés selon l'invention, en particulier le composé N° 13 et le composé N°17 sont des antagonistes des récepteurs $V_{1a}$ en bloquant les effets pharmacologiques provoqués par l'arginine-vasopressine.

Les composés selon l'invention peuvent donc être utilisés pour la préparation de médicaments, en particulier de médicaments antagonistes des récepteurs $V_{1a}$ humains de l'arginine-vasopressine et en plus pour certains composés des récepteurs $V_{1b}$ humains de l'AVP.

Ainsi, selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Ces médicaments trouvent leur emploi en thérapeutique, et sont avantageusement utiles dans les désordres de la sphère urogénitale notamment dans les domaines obstétrique et gynécologique, notamment comme agent tocolytique ou relaxant utérin ou pour contrôler l'hyperactivité utérine, les contractions de l'utérus avant que la grossesse soit arrivée à terme, pour contrôler le travail prénatal, ou encore contrôler le travail préparatoire en vue d'un accouchement par césarienne, favoriser la croissance du foetus in-utero, diminuer le stress et l'anoxie au moment des contractions, pour résoudre les problèmes de stérilité, fertilité, contrôler les naissances (usage vétérinaire en particulier), contrôler l'oestrus, le sevrage, le transfert et l'implantation d'embryon lors de la fécondation in vitro ; traiter l'endométriose, les dysménorrhées, ainsi que l'incontinence urinaire à l'effort ou d'urgence, l'hypertrophie bénigne de la prostate, les désordres de la miction, les infections urogénitales, les lithiases urinaires et les dysfonctions érectiles

Ces médicaments sont également utiles dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase, la thrombose ; les affections du système nerveux central comme la migraine, le vasospasme cérébral, l'hémorragie cérébrale, les traumatismes et les oedèmes cérébraux, la dépression, l'anxiété, le stress, les troubles émotionnels, le trouble obsessionnel-compulsif, les attaques de panique, les états psychotiques, l'agressivité, les troubles de la mémoire, du sommeil, les désordres cognitifs par exemple ; les affections du système rénal comme le vasospasme rénal, la nécrose du cortex rénal ; le diabète insipide néphrogénique ; la néphropathie diabétique ; les affections du système gastrique comme le vasospasme gastrique, l'hépatocirrhose, les ulcères, la pathologie des vomissements, par exemple la nausée y compris la nausée due à une chimiothérapie, le mal des transports. Les composés selon l'invention peuvent également être utilisés dans le traitement des troubles du comportement sexuel ; chez la femme, les composés selon l'invention peuvent être utilisés pour traiter la dysménorrhée primaire et secondaire, le travail prématuré ou l'endométriose. On peut également utiliser les composés selon l'invention

dans le traitement des cancers comme les cancers pulmonaires à petites cellules ou les cancers mammaires ; des encéphalopathies hyponatrémiques ; du syndrome pulmonaire, de la maladie de Ménière ; des hypertensions oculaires, du glaucome, de la cataracte ; de l'obésité ; du diabète de type I et II ; de l'athérosclérose ; du syndrome métabolique ; de l'hyperlipidémie ; de la résistance à l'insuline ; de l'hypertriglycéridémie ; dans les traitements post-opératoires, notamment après une chirurgie abdominale ; de l'autisme ; de l'hypercortisolémie ; de l'hyperaldostéronémie ; des phéochromocytomes ; du syndrome de Cushing ; de la préeclempsie ; des désordres de la micturation ; de l'éjaculation prématurée.

Les composés selon l'invention peuvent aussi être utilisés dans le traitement ou la prévention de toutes les pathologies consécutives au stress comme la fatigue et ses syndromes, les désordres ACTH dépendants, les troubles cardiaques, la douleur, les modifications de la vidange gastrique, de l'excrétion fécale (colite, syndrome du colon irritable, maladie de Crohn), de la sécrétion acide, l'hyperglycémie, l'immunosuppression, les processus inflammatoires (arthrite rhumatoïde et ostéoarthrite), les infections multiples, le choc septique, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance (alcool ou drogue), le sevrage, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysosurrénalien.

Les composés selon l'invention peuvent également être utilisés comme psychostimulants, provoquant l'augmentation de l'éveil, la réactivité émotionnelle face à l'environnement et facilitant l'adaptation.

Les composés selon la présente invention peuvent enfin être utilisés dans la cicatrisation, dans l'analgésie, dans l'anxiolyse, dans la prévention de la douleur, dans la prévention de l'anxiété, la dépression, la schizophrénie, l'autisme, les syndromes obsessionnels compulsifs, dans le comportement maternel (facilitation de la reconnaissance et de l'acceptation de la mère par l'enfant) et social, la mémoire ; la régulation de la prise de nourriture et de boisson, la dépendance aux drogues, le sevrage et la motivation sexuelle ; l'hypertension, l'hyponatrémie, l'insuffisance cardiaque, l'arthérosclérose, l'angiogénèse, la prolifération de tumeurs, le sarcome de Kaposi, réguler le stockage des graisses par l'adipocyte, contrôler les hyperlipidémies, triglycéridémie et le syndrome métabolique.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvate ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention : | 50,0 mg |
| Mannitol : | 223,75 mg |
| Croscarmellose sodique : | 6,0 mg |
| Amidon de maïs : | 15,0 mg |
| Hydroxypropyl-méthylcellulose : | 2,25 mg |
| Stéarate de magnésium : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également l'utilisation d'un composé de formule (I) ou un de ses sels pharmaceutiquement acceptables ou hydrates ou solvates pour la préparation d'un médicament destiné au traitement des pathologies ci-dessus indiquées.

## Revendications

1. Composé répondant à la formule (I):

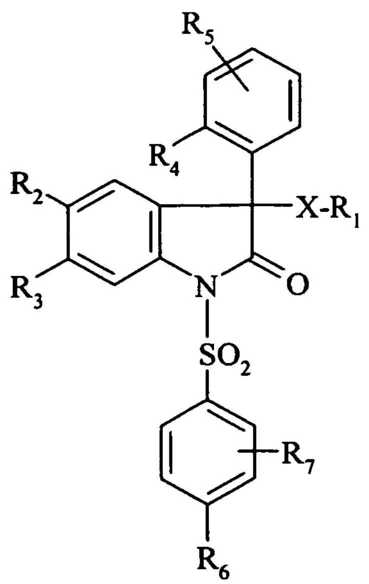

(I)

dans laquelle :

- X représente un radical bivalent ($C_1$-$C_5$)alkylène non substitué ou substitué une ou plusieurs fois sur un atome de carbone par un atome de fluor ou par un ($C_1$-$C_3$)alkyle ;
- $R_1$ représente :
    - . un groupe -$NR_8R_9$;
    - .un radical pipéridin-4-yle ou un radical pipéridin-3-yle non substitué ou substitué une ou plusieurs fois par un ($C_1$-$C_4$)alkyle, un ($C_3$-$C_5$)cycloalkyle, les atomes de carbone pouvant être également substitués par un ou plusieurs atomes de fluor ;
- $R_2$ représente un atome d'halogène, un groupe Alk, un groupe OAlk;
- $R_3$ représente un méthoxy ;
- $R_4$ représente un atome d'hydrogène, un atome d'halogène, un groupe Alk, un hydroxy, un groupe OAlk ;
- $R_5$ représente un atome d'hydrogène, un atome d'halogène, un groupe Alk, un hydroxy, un groupe OAlk, un groupe -$CO_2$Alk, un radical -$CH_2OH$ ;
- $R_6$ représente un atome d'hydrogène, un groupe Alk, un hydroxy, un groupe OAlk, un ($C_3$-$C_5$)cycloalkyoxy, un groupe -$NR_{10}CONR_{11}R_{12}$;
- $R_7$ représente un atome d'hydrogène, un atome d'halogène, un groupe Alk, un hydroxy, un groupe OAlk ;
- ou bien $R_7$ est en position -3- du phényle et ensemble avec $R_6$ ils représentent un radical triméthylène ;
- $R_8$ et $R_9$ représentent chacun indépendamment un atome d'hydrogène ou un ($C_1$-$C_4$)alkyle ;
- ou bien $R_8$ et $R_9$ ensemble avec l'atome d'azote auquel ils sont liés constituent un radical hétérocyclique saturé ou insaturé, de 3 à 10 chaînons, ledit radical hétérocyclique étant non substitué ou substitué une ou plusieurs fois par un amino, un diméthylamino, un hydroxy, un groupe Alk, un ($C_3$-$C_5$)cycloalkyle, un groupe OAlk, un radical -$SO_2$ Alk , les atomes de carbone pouvant être également substitués par un ou plusieurs atomes de fluor ;

- $R_{10}$ représente un atome d'hydrogène ou un ($C_1$-$C_4$)alkyle ;
- $R_{11}$ et $R_{12}$ représentent chacun indépendamment un atome d'hydrogène ou un ($C_1$-$C_4$)alkyle ;
- Alk représente un ($C_1$-$C_4$)alkyle non substitué ou substitué une ou plusieurs fois par un atome de fluor ;

à l'état de base ou de sel d'addition à un acide, ainsi qu'à l'état d'hydrates ou de solvates.

**2.** Composés de formule (I) selon la revendication 1 dans laquelle :

- X représente un radical bivalent ($C_1$-$C_5$)alkylène non substitué ou substitué une ou plusieurs fois sur un atome de carbone par un atome de fluor ou par un ($C_1$-$C_3$) alkyle ;
- $R_1$ représente :

  . un groupe $-NR_8R_9$ ;
  . un radical pipéridin-4-yle ou un radical pipéridin-3-yle non substitué ou substitué par un méthyle ;

- $R_2$ représente un atomed'halogène, un méthyle, un méthoxy ;
- $R_3$ représente un méthoxy ;
- $R_4$ représente un atome d'hydrogène, un atome de fluor, un méthyle, un méthoxy ;
- $R_5$ représente un atome d'hydrogène, un atome d'halogène, un groupe $-CO_2Alk$, un radical $-CH_2OH$ ;
- $R_6$ représente un atome d'hydrogène, un groupe Alk, un groupe OAlk, un hydroxy, un cyclopentyloxy, un groupe $-NHCON(Et)_2$ ;
- $R_7$ représente un atome d'hydrogène, un atome d'halogène, un méthyle, un méthoxy, un radical $CF_3$ ;
- ou bien $R_7$ est en position -3- du phényle et ensemble avec $R_6$ ils constituent un radical triméthylène ;
- $R_8$ et $R_9$ représentent chacun un méthyle ;
- ou bien $R_8$ et $R_9$ ensemble avec l'atome d'azote auquel ils sont liés constituent un radical hétérocyclique choisi parmi : la pyrrolidin-1-yle, la pipéridin-1-yle, la morpholin-4-yle, la pipérazin-1-yle, 1,4-diazépan-1-yle, 2,5-diazabicyclo[2.2.1]hept-2-yle, 3,8-diazabicyclo[3.2.1]oct-3-yle, 2,5-diazabicyclo[2.2.2]oct-2-yle, 1,4-diazabicyclo[3.2.1]oct-4-yle, hexahydropyrrolo[1,2-a]pyrazin-2(1*H*)-yle, octahydro-2H-pyrido[1,2-a]pyrazin-2-yle, ledit radical hétérocyclique étant non substitué ou substitué une ou deux fois par un atome de fluor, un amino, un diméthylamino, un hydroxy, un groupe Alk, un cyclobutyle, un radical $-SO_2Me$ ;
- Alk représente un ($C_1$-$C_4$)alkyle non substitué ou substitué une ou plusieurs fois par un atome de fluor ;

à l'état de base ou de sel d'addition à un acide, ainsi qu'à l'état d'hydrate ou de solvate.

**3.** Composés de formule (I) selon la revendication 1 dans laquelle :

- X représente un radical bivalent ($C_1$-$C_5$)alkylène ;
- $R_1$ représente :

  . un diméthylamino, un 3-aminopyrrolidin-1-yle, un 3-diméthylaminopyrrolidin-1-yle, un pipéridinyl-1-yle, un 3,3-difluoropipéridin-1-yle, un 4,4-difluoropipéridin-1-yle, un 4-hydroxypipéridin-1-yle, un morpholin-4-yle, un 4-méthylpipérazin-1-yle, un pipérazin-1-yle un 4-éthylpipérazin-1-yle, un 1,4-diazépan-1-yle, un 2,5-diazabicyclo[2.2.1]hept-2-yle, un 4-isopropylpipérazin-1-yle, un 3-méthylpipérazin-1-yle, un 3,4-diméthylpipérazin-1-yle, un 4-cyclobutylpipérazin-1-yle, un 5-méthyl-2,5-diazabicyclo[2.2.1]hept-2-yle, un 3,5-diméthylpipérazin-1-yle, un 4-méthyl-1,4-diazepan-1-yle, un 8-méthyl-3,8-diazabicyclo[3.2.1]oct-3-yle, un 2,6-diméthylpipérazin-1-yle, un 3-isopropylpipérazin-1-yle, un 2,2-diméthylpipérazin-1-yle, un 2,5-diazabicyclo[2.2.2]oct-2-yle, un 2,5-diméthylpipérazin-1-yle, un 2,2,4-triméthylpipérazin-1-yle, un 1,4-diazabicyclo [3.2.1]oct-4-yle, un 2-isopropylpipérazin-1-yle, un hexhydropyrrolo[1.2-a]pyrazin-2(1H)yle, un octahydro-2H-pyrido[1.2-a]pyrazin-2-yle, un 3- trifluorométhylpipérazin-1-yle, un 4-méthylsulfonylpipérazin-1-yle, un 4-(diméthylamino)pipéridin-1-yle ;
  . un pipéridin-4-yle un 1-méthylpipéridin-4-yle, un 1-méthylpipéridin-3-yle, un pipéridin-3-yle ;

- $R_2$ représente un atome de chlore, de fluor, de brome, un méthyle, un méthoxy ;
- $R_3$ représente un méthoxy ;
- $R_4$ représente un atome d'hydrogène, un atome de fluor, un méthyle, un méthoxy ;
- $R_5$ représente un atome d'hydrogène, un 3-chloro, un 5-fluoro, un 6-fluoro, un 5-méthoxycarbonyle, un 5-hydroxyméthyle ;
- $R_6$ représente un atome d'hydrogène, un méthyle, , un isopropyle un méthoxy, un éthoxy, un isopropoxy, un

butyloxy, un tert-butyloxy, un cyclopentyloxy, un 1,1,2,2-tétrafluoroéthyloxy, un 2,2-diéthyluréido un difluorométhoxy, un trifluorométhoxy, un hydroxy ;

- $R_7$ représente un atome d'hydrogène, un 3-méthyle, un 2-méthoxy, un 3-méthoxy, un 3-fluoro, un 3-chloro, un 3-$CF_3$ ;
- ou bien $R_7$ est en position -3- du phényle et ensemble avec $R_6$ ils constituent un radical triméthylène ;

à l'état de base ou de sel d'addition à un acide, ainsi qu'à l'état d'hydrate ou de solvate.

**4.** Composé selon la revendication 1 de formule (I) dans laquelle :

- X représente un radical bivalent triméthylène ou pentaméthylène ;
- $R_1$ représente :

. un diméthylamino, un 3-aminopyrrolidin-1-yle, un pipéridin-1-yle, un 4,4-difluoropipéridin-1-yle, un 4-hydroxypipéridin-1-yle, un 4-(diméthylamino)pipéridin-1-yle, un morpholin-4-yle, un 4-méthylpipérazin-1-yle, un pipérazin-1-yle, un 3,4-diméthylpipérazin-1-yle, un 5-méthyl-2,5-diazabicyclo[2.2.1]hept-2-yle, un 4-méthyl-1,4-diazepan-1-yle, un 8-méthyl-3,8-diazabicyclo[3.2.1]oct-3-yle, un 1,4-diazabicyclo[3.2.1]oct-4-yle ;

. un pipéridin-4-yle ;

- $R_2$ représente un atome de chlore, de fluor, de brome ou un méthyle ;
- $R_3$ représente un méthoxy ;
- $R_4$ représente un atome d'hydrogène ou un atome de fluor ;
- $R_5$ représente un atome d'hydrogène, un 5-méthoxycarbonyle, un 5-hydroxyméthyle ;
- $R_6$ représente un atome d'hydrogène, un méthyle, un méthoxy, un isopropoxy, un éthoxy, un butyloxy, un 1,1,2,2-tétrafluoroéthyloxy, un 2,2-diéthyluréido, un difluorométhoxy, un trifluorométhoxy, un hydroxy ;
- $R_7$ représente un atome d'hydrogène, un 2-méthoxy, un 3-méthoxy, un 3-méthyle, un 3-fluoro ;

à l'état de base ou de sel d'addition à un acide, ainsi qu'à l'état d'hydrate ou de solvat.

**5.** Composé selon la revendication 1 de formule (I) choisi parmi :

- 5-Chloro-1-[(2,4-diméthoxyphényl)sulfonyl]-3-(2-fluorophényl)-6-méthoxy-3-(3-pipéridin-4-ylpropyl)-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-(3-pipéridin-4-ylpropyl)-1,3-dihydro-2*H*-indol-2-one, isomère lévogyre ;
- 5-Chloro-3-[3-(diméthylamino)propyl]-3-(2-fluorophényl)-1-[(4-isopropoxyphényl)sulfonyl]-6-méthoxy-1,3-dihydro-2*H*-indol-2-one, isomère dextrogyre ;
- 3-{3-[(3*R*)-3-Aminopyrrolidin-1-yl]propyl}-5-chloro-3-(2-fluorophényl)-1-[(4-isopropoxyphényl)sulfonyl]-6-méthoxy-1,3-dihydro-2*H*-indol-2-one, isomère dextrogyre ;
- 3-{3-[(3*S*)-3-Aminopyrrolidin-1-yl]propyl}-5-chloro-3-(2-fluorophényl)-1-[(4-isopropoxyphényl)sulfonyl]-6-méthoxy-1,3-dihydro-2*H*-indol-2-one, isomère dextrogyre ;
- 3-{3-[(3*R*)-3-Aminopyrrolidin-1-yl]propyl}-5-chloro-1-[(4-éthoxy-3-méthoxyphényl)sulfonyl]-3-(2-fluorophényl)-6-méthoxy-1,3-dihydro-2*H*-indol-2-one, isomère dextrogyre ;
- 3-{3-[(3*S*)-3-Aminopyrrolidin-1-yl]propyl}-5-chloro-1-[(4-éthoxy-3-méthoxyphényl)sulfonyl]-3-(2-fluorophényl)-6-méthoxy-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-(3-pipéridin-1-ylpropyl)-1,3-dihydro-2*H*-indol-2-one, isomère dextrogyre ;
- 5-Chloro-3-[3-(4,4-difluoropipéridin-1-yl)propyl]-1-[(4-éthoxy-3-méthoxyphényl)sulfonyl]-3-(2-fluorophényl)-6-méthoxy-1,3-dihydro-2*H*-indol-2-one, isomère dextrogyre ;
- 5-Chloro-1-[(4-éthoxy-3-méthoxyphényl)sulfonyl]-3-(2-fluorophényl)-3-[3-(4-hydroxypipéridin-1-yl)propyl]-6-méthoxy-1,3-dihydro-2*H*-indol-2-one, isomère dextrogyre ;
- 5-Chloro-1-[(4-éthoxy-3-méthoxyphényl)sulfonyl]-3-(2-fluorophényl)-6-méthoxy-3-(3-morpholin-4-ylpropyl)-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-1-[(4-isopropoxyphényl)sulfonyl]-6-méthoxy-3-[3-(4-méthylpipérazin-1-yl)propyl]-1,3-dihydro-2*H*-indol-2-one, isomère dextrogyre ;

- 5-Chloro-1-[(4-éthoxy-3-méthoxyphényl)sulfonyl]-3-(2-fluorophényl)-6-méthoxy-3-[3-(4-méthylpipérazin-1-yl) propyl]-1,3-dihyàro-2*H*-indol-2-one, isomère dextrogyre ;
- 5-Chloro-1-[(3,4-diméthoxyphényl)sulfonyl]-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-1-[(2,4-diméthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-1-[(4-isopropoxyphényl)sulfonyl]-6-méthoxy-3-(3-pipérazin-1-ylpropyl)-1,3-dihydro-2*H*-indol-2-one, isomère dextrogyre ;
- 5-Chloro-1-[(4-éthoxy-3-méthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-1-[(3,4-diméthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-1-[(3,4-diméthylphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-6-méthoxy-1-[(4-méthoxy-3-méthylphényl)sulfonyl]-3-(3-pipérazin-1-ylpropyl)-1,3-dihydro-2*H*-indol-2-one ;
- 5-Chloro-1-[(4-éthoxyphényl)sulfonyl]-3-(2-fluorophényl)-6-méthoxy-3-(3-pipérazin-1-ylpropyl)-1,3-dihydro-2*H*-indol-2-one, isomère dextrogyre ;
- 5-Chloro-1-[(4-éthoxy-3-méthylphényl)sulfonyl]-3-(2-fluorophényl)-6-méthoxy-3-(3-pipérazin-1-ylpropyl)-1,3-dihydro-2*H*-indol-2-one ;
- 5-Chloro-1-{[4-(difluorométhoxy)phényl]sulfonyl}-3-(2-fluorophényl)-6-méthoxy-3-(3-pipérazin-1-ylpropyl)-1,3-dihydro-2*H*-indol-2-one, isomère dextrogyre ;
- 5-Chloro-3-(2-fluorophényl)-6-méthoxy-3-(3-pipérazin-1-ylpropyl)-1-{[4-(trifluorométhoxy)phényl]sulfonyl}-1,3-dihydro-2*H*-indol-2-one, isomère dextrogyre ;
- 5-Chloro-3-(2-fluorophényl)-6-méthoxy-1-(ph2nylsulfonyl)-3-(3-pipérazin-1-ylpropyl)-1,3-dihydro-2*H*-indol-2-one, isomère dextrogyre ;
- 5-Chloro-1-(2,3-dihydro-1*H*-inden-5-ylsulfonyl)-3-(2-fluoroph2nyl)-6-méthoxy-3-(3-pipérazin-1-ylpropyl)-1,3-dihydro-2*H*-indol-2-one, isomère dextrogyre ;
- 5-Chloro-1-{[3-fluoro-4-(1-méthyléthoxy)phényl]sulfonyl}-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-6-méthoxy-3-[3-(4-méthylpipérazin-1-yl)propyl]-1-{[4-(1,1,2,2-tétrafluoroéthoxy) phényl]sulfonyl}-1,3-dihydro-2*H*-indol-2-one, isomère dextrogyre ;
- 3-(2-Fluorophenyl)-6-methoxy-5-methyl-1-{[4-(1-methylethoxy)phenyl]sulfonyl}-3-[3-(4-methylpiperazin-1-yl) propyl]-1,3-dihydro-2*H*-indol-2-one, isomère dextrogyre ;
- 5-Fluoro-3-(2-fluorophényl)-6-méthoxy-1-{[4-(1-méthyléthoxy)phényl]sulfonyl}-3-[3-(4-méthylpipérazin-1-yl) propyl]-1,3-dihydro-2*H*-indol-2-one, isomère dextrogyre ;
- 5-Bromo-3-(2-fluorophényl)-6-méthoxy-1-{[4-(1-méthyléthoxy)phényl]sulfonyl}-3-[3-(4-méthylpipérazin-1-yl) propyl]-1,3-dihydro-2*H*-indol-2-one, isomère dextrogyre ;
- 3-[4-({3-[3-(Diméthylamino)propyl]-3-(2-fluorophényl)-6-méthoxy-5-méthyl-2-oxo-2,3-dihydro-1*H*-indol-1-yl} sulfonyl)-2-fluorophényl]-1,1-diéthylurée, isomère dextrogyre ;
- 3-[4-({3-[3-(Diméthylamino)propyl]-3-(2-fluorophényl)-6-méthoxy-5-méthyl-2-oxo-2,3-dihydro-1*H*-indol-1-yl} sulfonyl)-2-méthylphényl]-1,1-diéthylurée, isomère dextrogyre ;
- 5-Chloro-3-(2-fluorophényl)-6-méthoxy-3-[3-(8-méthyl-3,8-diazabicyclo[3.2.1] oct-3-yl)propyl]-1-{[4-(1-méthyléthoxy)phényl]sulfonyl}-1,3-dihydro-2*H*-indol-2-one, isomère dextrogyre ;
- 5-Chloro-3-(2-fluorophényl)-6-méthoxy-3-[3-(5-méthyl-2,5-diazabicyclo[2.2.1] hept-2-yl)propyl]-1-{[4-(1-méthyléthoxy)phényl]sulfonyl}-1,3-dihydro-2*H*-indol-2-one, isomère dextrogyre ;
- 5-Chloro-3-(2-fluorophényl)-1-[(4-hydroxyphényl)sulfonyl]-6-méthoxy-3-[3-(4-méthylpipérazin-1-yl)propyl]-1,3-dihydro-2*H*-indol-2-one, isomère dextrogyre ;
- 5-Chloro-3-(2-fluorophényl)-6-méthoxy-3-[3-(4-méthyl-1,4-diazépan-1-yl)propyl]-1-{[4-(1-méthyléthoxy)phényl]sulfonyl}-1,3-dihydro-2*H*-indol-2-one, isomère dextrogyre ;
- 5-Chloro-3-(2-fluorophényl)-6-méthoxy-1-{[4-(1-méthyléthoxy)phényl]sulfonyl}-3-(5-pipéridin-1-ylpentyl)-1,3-dihydro-2*H*-indol-2-one, isomère dextrogyre ;
- 5-Chloro-3-[5-(diméthylamino)pentyl]-3-(2-fluorophényl)-6-méthoxy-1-{[4-(1-méthyléthoxy)phényl]sulfonyl}-1,3-dihydro-2*H*-indol-2-one, isomère dextrogyre ;
- 5-Chloro-3-{3-[(3*S*)-3,4-diméthylpipérazin-1-yl]propyl}-3-(2-fluorophényl)-6-méthoxy-1-{[4-(1-méthyléthoxy) phényl]sulfonyl}-1,3-dihydro-2*H*-indol-2-one, isomère dextrogyre ;
- 5-Chloro-6-méthoxy-1-{[4-(1-méthyléthoxy)phényl]sulfonyl}-3-[3-(4-méthylpipérazin-1-yl)propyl]-3-phényl-1,3-dihydro-2*H*-indol-2-one, isomère dextrogyre ;

- 5-Chloro-3-{3-[(5*S*)-1,4-diazabicyclo[3.2.1]oct-4-yl]propyl}-3-(2-fluorophényl)-6-méthoxy-1-{[4-(1-méthyléthoxy)phényl]sulfonyl}-1,3-dihydro-2*H*-indol-2-one, isomère dextrogyre ;
- 1-[(4-Butoxyphenyl)sulfonyl]-5-chloro-3-(2-fluorophényl)-6-méthoxy-3-[3-(4-méthylpipérazin-1-yl)propyl]-1,3-dihydro-2*H*-indol-2-one, isomère dextrogyre ;
- 5-Chloro-3-[3-[4-(diméthylamino)pipéridin-1-yl]propyl]-3-(2-fluorophényl)-1-[(4-isopropoxyphényl)sulfonyl]-6-méthoxy-1,3-dihydro-2H-indol-2-one ;
- 1-[(3-Fluoro-4-isopropoxyphényl)sulfonyl]-3-(2-fluorophényl)-5,6-diméthoxy-3-[3-(4-méthylpipérazin-1-yl)propyl]-1,3-dihydro-2H-indol-2-one ;
- 5-Fluoro-1-[(3-fluoro-4-isopropoxyphényl)sulfonyl]-3-(2-fluorophényl)-6-méthoxy-3-[3-(4-méthylpipérazin-1-yl)propyl]-1,3-dihydro-2H-indol-2-one ;
- 3-(2-Fluorophényl)-5,6-diméthoxy-1-[(4-méthoxy-3-méthylphényl)sulfonyl]-3-[3-(4-méthylpipérazin-1-yl)propyl]-1,3-dihydro-2H-indol-2-one ;
- 3-[5-Chloro-1-[(4-isopropoxyphényl)sulfonyl]-6-méthoxy-3-[3-(4-méthylpipérazin-1-yl)propyl]-2-oxo-2,3-dihydro-1H-indol-3-yl]-4-fluorobenzoate de méthyle ;
- 5-Chloro-3-[2-fluoro-5-(hydroxyméthyl)phényl]-1-[(4-isopropoxyphényl) sulfonyl]-6-méthoxy-3-[3-(4-méthylpipérazin-1-yl)propyl]-1,3-dihydro-2H-indol-2-one ;

à l'état de base ou de sels d'addition à des acides, ainsi qu'à l'état d'hydrates ou de solvates.

6. Procédé de préparation des composés de formule (I) selon l'une quelconque des revendications 1 à 5, **caractérisé en ce que** :

on fait réagir, en présence d'une base, un composé de formule :

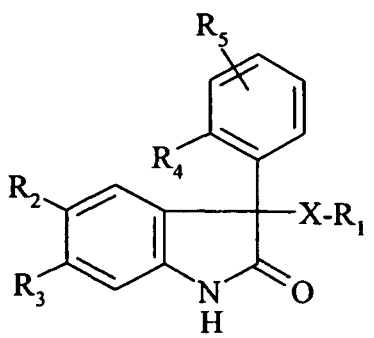

(II)

dans laquelle X, $R_1$, $R_2$, $R_3$, $R_4$ et $R_5$ sont tels que définis pour un composé de formule (I) à la revendication 1, avec un halogénure de sulfonyle de formule :

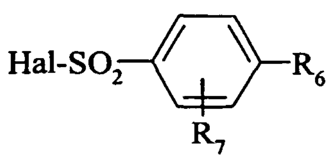

(III)

dans laquelle $R_6$ et $R_7$ sont tels que définis pour un composé de formule (I) à la revendication 1 et Hal représente un atome d'halogène.

7. Procédé de préparation des composés de formule (I) selon l'une quelconque des revendications 1 à 5, **caractérisé en ce que** :

on fait réagir un composé de formule :

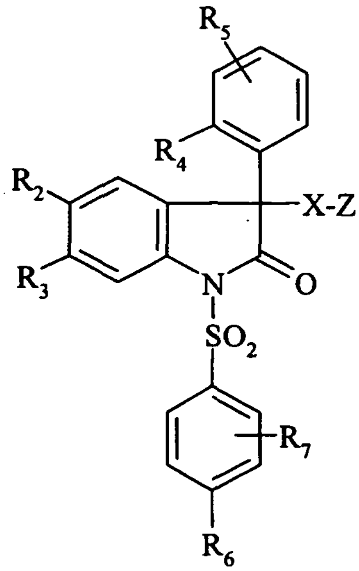

(IV)

dans laquelle X, $R_2$, $R_3$, $R_4$, $R_5$, $R_6$ et $R_7$ sont tels que définis pour un composé de formule (I) dans la revendication 1 et Z représente un groupe partant choisi parmi un atome d'halogène ou un groupe méthanesulfonate ou p-toluènesulfonate, avec un composé de formule :

$R_1$-H (V)

dans laquelle $R_1$ est tel que défini pour un composé de formule (I) dans la revendication 1.

**8.** Médicament **caractérisé en ce qu'**il comprend un composé de formule (I) selon l'une quelconque des revendications 1 à 5, ou un sel d'addition de ce composé à un acide pharmaceutiquement acceptable, ou encore un hydrate ou un solvate du composé de formule (I).

**9.** Composition pharmaceutique **caractérisée en ce qu'**elle comprend un composé de formule (I) selon l'une quelconque des revendications 1 à 5, ou un sel pharmaceutiquement acceptable, un hydrate ou un solvate de ce composé, ainsi qu'au moins un excipient pharmaceutiquement acceptable.

**10.** Utilisation d'un composé de formule (I) selon l'une quelconque des revendications 1 à 5 pour la préparation d'un médicament destiné au traitement et à la prévention de l'hypertension, de l'hypertension pulmonaire, de l'insuffisance cardiaque, de l'infarctus du myocarde, du vasospasme coronaire, en particulier chez le fumeur, de la maladie de Raynaud, des angines instables et PTCA (de l'anglais percutaneous transluminal coronary angioplasty), du vasospasme cérébral, de l'hémorragie cérébrale, des traumatismes et des oedèmes cérébraux, de la dépression, de l'anxiété, du stress, des troubles émotionnels, des troubles de la mémoire, du sommeil, de la néphropathie diabétique, des cancers comme les cancers pulmonaires à petites cellules ou les cancers mammaires ; de l'athérosclérose ; du syndrome métabolique ; de l'hyperlipidémie ; de la résistance à l'insuline, de l'hypertriglycéridémie, de l'hypercortisolémie, l'hyperaldostéronémie, des phéochromocytomes, du syndrome de Cushing, de la colite, du syndrome du colon irritable, de la maladie de Crohn, de la douleur (fibromyalgie), des maladies neurodégénératrices (maladie d'Alzheimer, maladie de Parkinson, maladie d'Huntington), de la dépendance à une substance (alcool ou drogue) ; comme agent tocolytique ou relaxant utérin ou pour contrôler l'hyperactivité utérine, les contractions précoces de l'utérus, favoriser la croissance du foetus in-utero, traiter l'endométriose, les dysménorrhées, les dysfonctions érectiles.

## Claims

**1.** Compound corresponding to the formula (I):

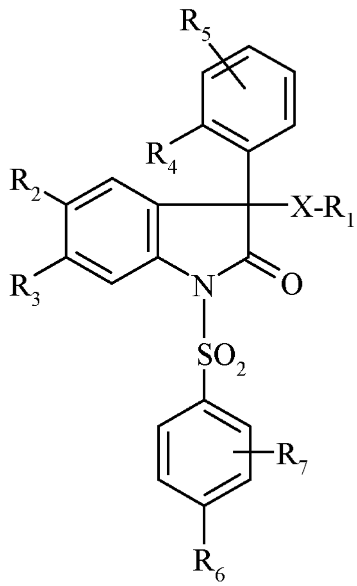

(I)

in which:

- X represents a divalent $(C_1-C_5)$alkylene radical which is unsubstituted or substituted one or more times on a carbon atom by a fluorine atom or by a $(C_1-C_3)$ alkyl;
- $R_1$ represents:

  - . an $-NR_8R_9$ group;
  - . a piperidin-4-yl radical or a piperidin-3-yl radical which is unsubstituted or substituted one or more times by a $(C_1-C_4)$ alkyl or a $(C_3-C_5)$cycloalkyl, it being possible for the carbon atoms to be also substituted by one or more fluorine atoms;

- $R_2$ represents a halogen atom, an Alk group or an OAlk group;
- $R_3$ represents a methoxy;
- $R_4$ represents a hydrogen atom, a halogen atom, an Alk group, a hydroxyl or an OAlk group;
- $R_5$ represents a hydrogen atom, a halogen atom, an Alk group, a hydroxyl, an OAlk group, a - $CO_2$Alk group or a $-CH_2OH$ radical;
- $R_6$ represents a hydrogen atom, an Alk group, a hydroxyl, an OAlk group, a $(C_3-C_5)$cycloalkyloxy or an $-NR_{10}CONR_{11}R_{12}$ group;
- $R_7$ represents a hydrogen atom, a halogen atom, an Alk group, a hydroxyl or an OAlk group;
- or else $R_7$ is in the 3 position of the phenyl and, together with $R_6$, represents a trimethylene radical;
- $R_8$ and $R_9$ each independently represent a hydrogen atom or a $(C_1-C_4)$ alkyl;
- or else $R_8$ and $R_9$, together with the nitrogen atom to which they are bonded, constitute a saturated or unsaturated 3- to 10-membered heterocyclic radical, said heterocyclic radical being unsubstituted or substituted one or more times by an amino, a dimethylamino, a hydroxyl, an Alk group, a $(C_3-C_5)$cycloalkyl, an OAlk group or an $-SO_2$Alk radical, it being possible for the carbon atoms to be also substituted by one or more fluorine atoms;
- $R_{10}$ represents a hydrogen atom or a $(C_1-C_4)$ alkyl;
- $R_{11}$ and $R_{12}$ each independently represent a hydrogen atom or a $(C_1-C_4)$ alkyl;
- Alk represents a $(C_1-C_4)$ alkyl which is unsubstituted or substituted one or more times by a fluorine atom;

in the form of the base or of an addition salt with an acid, and also in the form of hydrates or solvates.

**2.** Compound of formula (I) according to Claim 1, in which:

- X represents a divalent $(C_1-C_5)$alkylene radical which is unsubstituted or substituted one or more times on a carbon atom by a fluorine atom or by a $(C_1-C_3)$ alkyl;
- $R_1$ represents:

  • an $-NR_8R_9$ group;

• a piperidin-4-yl radical or a piperidin-3-yl radical which is unsubstituted or substituted by a methyl;

- $R_2$ represents a halogen atom, a methyl or a methoxy;
- $R_3$ represents a methoxy;
- $R_4$ represents a hydrogen atom, a fluorine atom, a methyl or a methoxy;
- $R_5$ represents a hydrogen atom, a halogen atom, a $-CO_2Alk$ group or a $-CH_2OH$ radical;
- $R_6$ represents a hydrogen atom, an Alk group, an OAlk group, a hydroxyl, a cyclopentyloxy or an - NHCON $(Et)_2$ group;
- $R_7$ represents a hydrogen atom, a halogen atom, a methyl, a methoxy or a $CF_3$ radical;
- or else $R_7$ is in the 3 position of the phenyl and, together with $R_6$, constitutes a trimethylene radical;
- $R_8$ and $R_9$ each represent a methyl;
- or else $R_8$ and $R_9$, together with the nitrogen atom to which they are bonded, constitute a heterocyclic radical chosen from: pyrrolidin-1-yl, piperidin-1-yl, morpholin-4-yl, piperazin-1-yl, 1,4-diazepan-1-yl, 2,5-diazabicyclo [2.2.1]hept-2-yl, 3,8-diazabicyclo[3.2.1]oct-3-yl, 2,5-diazabicyclo[2.2.2]oct-2-yl, 1,4-diazabicyclo-[3.2.1]oct-4-yl, hexahydropyrrolo[1,2-a]pyrazin-2(1H)-yl or octahydro-2H-pyrido[1,2-a]pyrazin-2-yl, said heterocyclic radical being unsubstituted or substituted once or twice by a fluorine atom, an amino, a dimethylamino, a hydroxyl, an Alk group, a cyclobutyl or an $-SO_2Me$ radical;
- Alk represents a $(C_1-C_4)$ alkyl which is unsubstituted or substituted one or more times by a fluorine atom;

in the form of the base or of an addition salt with an acid, and also in the hydrate or solvate form.

**3.** Compound of formula (I) according to Claim 1, in which:

- X represents a divalent $(C_1-C_5)$alkylene radical;
- $R_1$ represents:

. a dimethylamino, a 3-aminopyrrolidin-1-yl, a 3-dimethylaminopyrrolidin-1-yl, a piperidin-1-yl, a 3,3-difluoropiperidin-1-yl, a 4,4-difluoropiperidin-1-yl, a 4-hydroxypiperidin-1-yl, a morpholin-4-yl, a 4-methylpiperazin-1-yl, a piperazin-1-yl, a 4-ethylpiperazin-1-yl, a 1,4-diazepan-1-yl, a 2,5-diazabicyclo[2.2.1]hept-2-yl, a 4-isopropylpiperazin-1-yl, a 3-methylpiperazin-1-yl, a 3,4-dimethylpiperazin-1-yl, a 4-cyclobutylpiperazin-1-yl, a 5-methyl-2,5-diazabicyclo[2.2.1]hept-2-yl, a 3,5-dimethylpiperazin-1-yl, a 4-methyl-1,4-diazepan-1-yl, an 8-methyl-3,8-diazabicyclo[3.2.1]oct-3-yl, a 2,6-dimethylpiperazin-1-yl, a 3-isopropylpiperazin-1-yl, a 2,2-dimethylpiperazin-1-yl, a 2,5-diazabicyclo[2.2.2]oct-2-yl, a 2,5-dimethylpiperazin-1-yl, a 2,2,4-trimethylpiperazin-1-yl, a 1,4-diazabicyclo[3.2.1]oct-4-yl, a 2-isopropylpiperazin-1-yl, a hexahydropyrrolo[1,2-a] pyrazin-2(1H)-yl, an octahydro-2H-pyrido-[1,2-a]pyrazin-2-yl, a 3-trifluoromethylpiperazin-1-yl, a 4-methylsulfonylpiperazin-1-yl or a 4-(dimethylamino)piperidin-1-yl;
. a piperidin-4-yl, a 1-methylpiperidin-4-yl, a 1-methylpiperidin-3-yl or a piperidin-3-yl;

- $R_2$ represents a chlorine, fluorine or bromine atom, a methyl or a methoxy;
- $R_3$ represents a methoxy;
- $R_4$ represents a hydrogen atom, a fluorine atom, a methyl or a methoxy;
- $R_5$ represents a hydrogen atom, a 3-chloro, a 5-fluoro, a 6-fluoro, a 5-methoxycarbonyl or a 5-hydroxymethyl;
- $R_6$ represents a hydrogen atom, a methyl, an isopropyl, a methoxy, an ethoxy, an isopropoxy, a butyloxy, a tert-butyloxy, a cyclopentyloxy, a 1,1,2,2-tetrafluoroethyloxy, a 2,2-diethylureido, a difluoromethoxy, a trifluoromethoxy or a hydroxyl;
- $R_7$ represents a hydrogen atom, a 3-methyl, a 2-methoxy, a 3-methoxy, a 3-fluoro, a 3-chloro or a $3-CF_3$;
- or else $R_7$ is in the 3 position of the phenyl and, together with $R_6$, constitutes a trimethylene radical;

in the form of the base or of an addition salt with an acid, and also in the hydrate or solvate form.

**4.** Compound according to Claim 1 of formula (I), in which:

- X represents a divalent trimethylene or pentamethylene radical;
- $R_1$ represents:

. a dimethylamino, a 3-aminopyrrolidin-1-yl, a piperidin-1-yl, a 4,4-difluoropiperidin-1-yl, a 4-hydroxypiperidin-1-yl, a 4-(dimethylamino)piperidin-1-yl, a morpholin-4-yl, a 4-methylpiperazin-1-yl, a piperazin-1-yl, a 3,4-dimethylpiperazin-1-yl, a 5-methyl-2,5-diazabicyclo[2.2.1]hept-2-yl, a 4-methyl-1,4-diazepan-1-yl, an

8-methyl-3,8-diazabicyclo[3.2.1]oct-3-yl or a 1,4-diazabicyclo[3.2.1]oct-4-yl;
. a piperidin-4-yl;

- $R_2$ represents a chlorine, fluorine or bromine atom or a methyl;
- $R_3$ represents a methoxy;
- $R_4$ represents a hydrogen atom or a fluorine atom;
- $R_5$ represents a hydrogen atom, a 5-methoxycarbonyl or a 5-hydroxymethyl;
- $R_6$ represents a hydrogen atom, a methyl, a methoxy, an isopropoxy, an ethoxy, a butyloxy, a 1,1,2,2-tetrafluoroethyloxy, a 2,2-diethylureido, a difluoromethoxy, a trifluoromethoxy or a hydroxyl;
- $R_7$ represents a hydrogen atom, a 2-methoxy, a 3-methoxy, a 3-methyl or a 3-fluoro;

in the form of the base or of an addition salt with an acid, and also in the hydrate or solvate form.

**5.** Compound according to Claim 1 of formula (I), chosen from:

- 5-chloro-1-[(2,4-dimethoxyphenyl)sulfonyl]-3-(2-fluorophenyl)-6-methoxy-3-(3-(piperidin-4-yl)propyl)-1,3-dihydro-2*H*-indol-2-one, dextrorotatory isomer;
- 5-chloro-1-[(2,4-dimethoxyphenyl)sulfonyl]-3-(2-fluorophenyl)-6-methoxy-3-(3-(piperidin-4-yl)propyl)-1,3-dihydro-2*H*-indol-2-one, levorotatory isomer;
- 5-chloro-3-[3-(dimethylamino)propyl]-3-(2-fluorophenyl)-1-[(4-isopropoxyphenyl)sulfonyl]-6-methoxy-1,3-dihydro-2*H*-indol-2-one, dextrorotatory isomer;
- 3-{3-[(3*R*)-3-aminopyrrolidin-1-yl]propyl}-5-chloro-3-(2-fluorophenyl)-1-[(4-isopropoxyphenyl)sulfonyl]-6-methoxy-1,3-dihydro-2*H*-indol-2-one, dextrorotatory isomer;
- 3-{3-[(3*S*)-3-aminopyrrolidin-1-yl]propyl}-5-chloro-3-(2-fluorophenyl)-1-[(4-isopropoxyphenyl)sulfonyl]-6-methoxy-1,3-dihydro-2*H*-indol-2-one, dextrorotatory isomer;
- 3-{3-[(3*R*)-3-aminopyrrolidin-1-yl]propyl}-5-chloro-1-[(4-ethoxy-3-methoxyphenyl)sulfonyl]-3-(2-fluorophenyl)-6-methoxy-1,3-dihydro-2*H*-indol-2-one, dextrorotatory isomer;
- 3-{3-[(3*S*)-3-aminopyrrolidin-1-yl]propyl}-5-chloro-1-[(4-ethoxy-3-methoxyphenyl)sulfonyl]-3-(2-fluorophenyl)-6-methoxy-1,3-dihydro-2*H*-indol-2-one, dextrorotatory isomer;
- 5-chloro-1-[(2,4-dimethoxyphenyl)sulfonyl]-3-(2-fluorophenyl)-6-methoxy-3-(3-(piperidin-1-yl)propyl)-1,3-dihydro-2*H*-indol-2-one, dextrorotatory isomer;
- 5-chloro-3-[3-(4,4-difluoropiperidin-1-yl)propyl]-1-[(4-ethoxy-3-methoxyphenyl)sulfonyl]-3-(2-fluorophenyl)-6-methoxy-1,3-dihydro-2*H*-indol-2-one, dextrorotatory isomer;
- 5-chloro-1-[(4-ethoxy-3-methoxyphenyl)sulfonyl]-3-(2-fluorophenyl)-3-[3-(4-hydroxypiperidin-1-yl)propyl]-6-methoxy-1,3-dihydro-2*H*-indol-2-one, dextrorotatory isomer;
- 5-chloro-1-[(4-ethoxy-3-methoxyphenyl)sulfonyl]-3-(2-fluorophenyl)-6-methoxy-3-(3-(morpholin-4-yl)propyl)-1,3-dihydro-2*H*-indol-2-one, dextrorotatory isomer;
- 5-chloro-1-[(2,4-dimethoxyphenyl)sulfonyl]-3-(2-fluorophenyl)-6-methoxy-3-[3-(4-methylpiperazin-1-yl)propyl]-1,3-dihydro-2*H*-indol-2-one, dextrorotatory isomer;
- 5-chloro-3-(2-fluorophenyl)-1-[(4-isopropoxyphenyl)sulfonyl]-6-methoxy-3-[3-(4-methylpiperazin-1-yl)propyl]-1,3-dihydro-2*H*-indol-2-one, dextrorotatory isomer;
- 5-chloro-1-[(4-ethoxy-3-methoxyphenyl)sulfonyl]-3-(2-fluorophenyl)-6-methoxy-3-[3-(4-methylpiperazin-1-yl) propyl]-1,3-dihydro-2*H*-indol-2-one, dextrorotatory isomer;
- 5-chloro-1-[(3,4-dimethoxyphenyl)sulfonyl]-3-(2-fluorophenyl)-6-methoxy-3-[3-(4-methylpiperazin-1-yl)propyl]-1,3-dihydro-2*H*-indol-2-one, dextrorotatory isomer;
- 5-chloro-1-[(2,4-dimethoxyphenyl)sulfonyl]-3-(2-fluorophenyl)-6-methoxy-3-(3-(piperazin-1-yl)propyl)-1,3-dihydro-2*H*-indol-2-one, dextrorotatory isomer;
- 5-chloro-3-(2-fluorophenyl)-1-[(4-isopropoxyphenyl)sulfonyl]-6-methoxy-3-(3-(piperazin-1-yl)propyl)-1,3-dihydro-2H-indol-2-one, dextrorotatory isomer;
- 5-chloro-1-[(4-ethoxy-3-methoxyphenyl)sulfonyl]-3-(2-fluorophenyl)-6-methoxy-3-(3-(piperazin-1-yl)propyl)-1,3-dihydro-2*H*-indol-2-one, dextrorotatory isomer;
- 5-chloro-1-[(3,4-dimethoxyphenyl)sulfonyl]-3-(2-fluorophenyl)-6-methoxy-3-(3-(piperazin-1-yl)propyl)-1,3-dihydro-2*H*-indol-2-one, dextrorotatory isomer;
- 5-chloro-1-[(3,4-dimethylphenyl)sulfonyl]-3-(2-fluorophenyl)-6-methoxy-3-(3-(piperazin-1-yl)propyl)-1,3-dihydro-2*H*-indol-2-one, dextrorotatory isomer;
- 5-chloro-3-(2-fluorophenyl)-6-methoxy-1-[(4-methoxy-3-methylphenyl)sulfonyl]-3-(3-(piperazin-1-yl)propyl)-1,3-dihydro-2*H*-indol-2-one;
- 5-chloro-1-[(4-ethoxyphenyl)sulfonyl]-3-(2-fluorophenyl)-6-methoxy-3-(3-(piperazin-1-yl)propyl)-1,3-dihydro-2*H*-indol-2-one, dextrorotatory isomer;
- 5-chloro-1-[(4-ethoxy-3-methylphenyl)sulfonyl]-3-(2-fluorophenyl)-6-methoxy-3-(3-(piperazin-1-yl)propyl)-1,3-dihydro-2*H*-indol-2-one;
- 5-chloro-1-{[4-(difluoromethoxy)phenyl]sulfonyl}-3-(2-fluorophenyl)-6-methoxy-3-(3-(piperazin-1-yl)propyl)-1,3-dihydro-2*H*-indol-2-one, dextrorotatory isomer;
- 5-chloro-3-(2-fluorophenyl)-6-methoxy-3-(3-(piperazin-1-yl)propyl)-1-{[4-(trifluoromethoxy)phenyl]sulfonyl}-1,3-dihydro-2*H*-indol-2-one, dextrorotatory isomer;
- 5-chloro-3-(2-fluorophenyl)-6-methoxy-1-(phenylsulfonyl)-3-(3-(piperazin-1-yl)propyl)-1,3-dihydro-2*H*-indol-2-one, dextrorotatory isomer;
- 5-chloro-1-(2,3-dihydro-1*H*-inden-5-ylsulfonyl)-3-(2-fluorophenyl)-6-methoxy-3-(3-(piperazin-1-yl)propyl)-1,3-dihydro-2*H*-indol-2-one, dextrorotatory isomer;
- 5-chloro-1-{[3-fluoro-4-(1-methylethoxy)phenyl]sulfonyl}-3-(2-fluorophenyl)-6-methoxy-3-[3-(4-methylpiperazin-1-yl)propyl]-1,3-dihydro-2*H*-indol-2-one, dextrorotatory isomer;
- 5-chloro-3-(2-fluorophenyl)-6-methoxy-3-[3-(4-methylpiperazin-1-yl)propyl]-1-{[4-(1,1,2,2-tetrafluoroethoxy)phenyl]sulfonyl}-1,3-dihydro-2*H*-indol-2-one, dextrorotatory isomer;
- 3-(2-fluorophenyl)-6-methoxy-5-methyl-1-{[4-(1-methylethoxy)phenyl]sulfonyl}-3-[3-(4-methylpiperazin-1-yl)propyl]-1,3-dihydro-2*H*-indol-2-one, dextrorotatory isomer;
- 5-fluoro-3-(2-fluorophenyl)-6-methoxy-1-{[4-(1-methylethoxy)phenyl]sulfonyl}-3-[3-(4-methylpiperazin-1-yl)propyl]-1,3-dihydro-2*H*-indol-2-one, dextrorotatory isomer;
- 5-bromo-3-(2-fluorophenyl)-6-methoxy-1-{[4-(1-methylethoxy)phenyl]sulfonyl}-3-[3-(4-methylpiperazin-1-yl)propyl]-1,3-dihydro-2*H*-indol-2-one, dextrorotatory isomer;
- 3-[4-({3-[3-(dimethylamino)propyl]-3-(2-fluorophenyl)-6-methoxy-5-methyl-2-oxo-2,3-dihydro-1*H*-indol-1-yl}sulfonyl)-2-fluorophenyl]-1,1-diethylurea, dextrorotatory isomer;
- 3-[4-({3-[3-(dimethylamino)propyl]-3-(2-fluorophenyl)-6-methoxy-5-methyl-2-oxo-2,3-dihydro-1*H*-indol-1-yl}sulfonyl)-2-methylphenyl]-1,1-diethylurea, dextrorotatory isomer;
- 5-chloro-3-(2-fluorophenyl)-6-methoxy-3-[3-(8-methyl-3,8-diazabicyclo[3.2.1]oct-3-yl)propyl]-1-{[4-(1-methylethoxy)phenyl]sulfonyl}-1,3-dihydro-2*H*-indol-2-one, dextrorotatory isomer;
- 5-chloro-3-(2-fluorophenyl)-6-methoxy-3-[3-(5-methyl-2,5-diazabicyclo[2.2.1]hept-2-yl)propyl]-1-{[4-(1-methylethoxy)phenyl]sulfonyl}-1,3-dihydro-2*H*-indol-2-one, dextrorotatory isomer;
- 5-chloro-3-(2-fluorophenyl)-1-[(4-hydroxyphenyl)sulfonyl]-6-methoxy-3-[3-(4-methylpiperazin-1-yl)propyl]-1,3-dihydro-2*H*-indol-2-one, dextrorotatory isomer;
- 5-chloro-3-(2-fluorophenyl)-6-methoxy-3-[3-(4-methyl-1,4-diazepan-1-yl)propyl]-1-{[4-(1-methylethoxy)phenyl]sulfonyl}-1,3-dihydro-2*H*-indol-2-one, dextrorotatory isomer;
- 5-chloro-3-(2-fluorophenyl)-6-methoxy-1-{[4-(1-methylethoxy)phenyl]sulfonyl}-3-(5-(piperidin-1-yl)pentyl)-1,3-dihydro-2*H*-indol-2-one, dextrorotatory isomer;
- 5-chloro-3-[5-(dimethylamino)pentyl]-3-(2-fluorophenyl)-6-methoxy-1-{[4-(1-methylethoxy)phenyl]sulfonyl}-1,3-dihydro-2*H*-indol-2-one, dextrorotatory isomer;
- 5-chloro-3-{3-[(3*S*)-3,4-dimethylpiperazin-1-yl]propyl}-3-(2-fluorophenyl)-6-methoxy-1-{[4-(1-methylethoxy)phenyl]sulfonyl}-1,3-dihydro-2*H*-indol-2-one, dextrorotatory isomer;
- 5-chloro-6-methoxy-1-{[4-(1-methylethoxy)phenyl]sulfonyl}-3-[3-(4-methyl-piperazin-1-yl)propyl]-3-phenyl-1,3-dihydro-2*H*-indol-2-one, dextrorotatory isomer;
- 5-chloro-3-{3-[(5S)-1,4-diazabicyclo[3.2.1]oct-4-yl]propyl}-3-(2-fluorophenyl)-6-methoxy-1-{[4-(1-methylethoxy)phenyl]sulfonyl}-1,3-dihydro-2*H*-indol-2-one, dextrorotatory isomer;
- 1-[(4-butoxyphenyl)sulfonyl]-5-chloro-3-(2-fluorophenyl)-6-methoxy-3-[3-(4-methylpiperazin-1-yl)propyl]-1,3-dihydro-2*H*-indol-2-one, dextrorotatory isomer;
- 5-chloro-3-[3-[4-(dimethylamino)piperidin-1-yl]propyl]-3-(2-fluorophenyl)-1-[(4-isopropoxyphenyl)sulfonyl]-6-methoxy-1,3-dihydro-2*H*-indol-2-one;
- 1-[(3-fluoro-4-isopropoxyphenyl)sulfonyl]-3-(2-fluorophenyl)-5,6-dimethoxy-3-[3-(4-methylpiperazin-1-yl)propyl]-1,3-dihydro-2*H*-indol-2-one;
- 5-fluoro-1-[(3-fluoro-4-isopropoxyphenyl)sulfonyl]-3-(2-fluorophenyl)-6-methoxy-3-[3-(4-methylpiperazin-1-yl)propyl]-1,3-dihydro-2*H*-indol-2-one;
- 3-(2-fluorophenyl)-5,6-dimethoxy-1-[(4-methoxy-3-methylphenyl)sulfonyl]-3-[3-(4-methylpiperazin-1-yl)propyl]-1,3-dihydro-2*H*-indol-2-one;
- methyl 3-[5-chloro-1-[(4-isopropoxyphenyl)sulfonyl]-6-methoxy-3-[3-(4-methylpiperazin-1-yl)propyl]-2-oxo-2,3-dihydro-1*H*-indol-3-yl]-4-fluorobenzoate;
- 5-chloro-3-[2-fluoro-5-(hydroxymethyl)phenyl]-1-[(4-isopropoxyphenyl)sulfonyl]-6-methoxy-3-[3-(4-methylpiperazin-1-yl)propyl]-1,3-dihydro-2*H*-indol-2-one;

in the form of the base or of addition salts with acids, and also in the hydrate or solvate form.

6. Process for the preparation of the compounds of formula (I) according to any one of Claims 1 to 5, **characterized in that**:

a compound of formula:

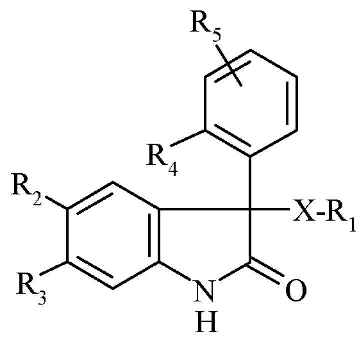

(II)

in which X, $R_1$, $R_2$, $R_3$, $R_4$ and $R_5$ are as defined for a compound of formula (I) in Claim 1, is reacted, in the presence of a base, with a sulfonyl halide of formula:

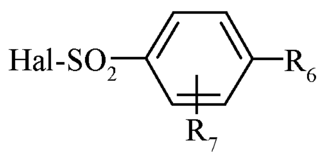

(III)

in which $R_6$ and $R_7$ are as defined for a compound of formula (I) in Claim 1 and Hal represents a halogen atom.

7. Process for the preparation of the compounds of formula (I) according to any one of Claims 1 to 5, **characterized in that**:

a compound of formula:

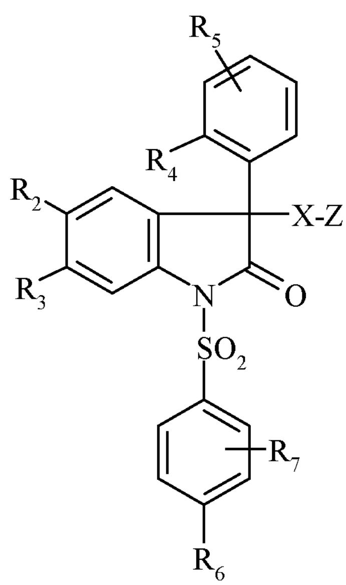

(IV)

in which X, $R_2$, $R_3$, $R_4$, $R_5$, $R_6$ and $R_7$ are as defined for a compound of formula (I) in Claim 1 and Z represents a leaving group chosen from a halogen atom or a methanesulfonate or p-toluenesulfonate group, is reacted

with a compound of formula:

$R_1$-H (V)

in which $R_1$ is as defined for a compound of formula (I) in Claim 1.

**8.** Medicament, **characterized in that** it comprises a compound of formula (I) according to any one of Claims 1 to 5, or an addition salt of this compound with a pharmaceutically acceptable acid, or also a hydrate or a solvate of the compound of formula (I).

**9.** Pharmaceutical composition, **characterized in that** it comprises a compound of formula (I) according to any one of Claims 1 to 5, or a pharmaceutically acceptable salt, a hydrate or a solvate of this compound, and at least one pharmaceutically acceptable excipient.

**10.** Use of a compound of formula (I) according to any one of Claims 1 to 5 in the preparation of a medicament intended for the treatment or prevention of hypertension, pulmonary hypertension, cardiac insufficiency, myocardial infarction, coronary vasospasm, in particular in smokers, Raynaud's disease, unstable angina and PTCA (percutaneous transluminal coronary angioplasty), cerebral vasospasm, cerebral hemorrhage, trauma and cerebral edema, depression, anxiety, stress, emotional disorders, memory or sleep disorders, diabetic nephropathy, cancers, such as small cell lung cancers or breast cancers; atherosclerosis; metabolic syndrome; hyperlipidemia; insulin resistance, hypertriglyceridemia, hypercortisolemia, hyperaldosteronemia, pheochromocytoma, Cushing's syndrome, colitis, irritable bowel syndrome, Crohn's disease, pain (fibromyalgia), neurodegenerative diseases (Alzheimer's disease, Parkinson's disease or Huntington's disease), substance (alcohol or drug) dependence; as uterine relaxant or tocolytic agent or for controlling uterine hyperactivity, premature contractions of the uterus, promoting the growth of the fetus in utero, treating endometriosis, dysmenorrhea or erectile dysfunctions.

**Patentansprüche**

**1.** Verbindung der Formel (I):

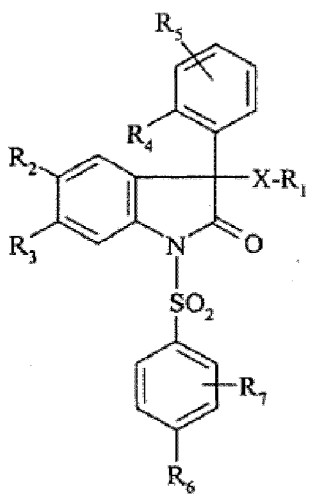

(I)

mit den folgenden Bedeutungen:

- X bedeutet einen zweiwertigen ($C_1$-$C_5$)Alkylenrest, der unsubstituiert oder einfach oder mehrfach an einem Kohlenstoffatom durch ein Fluoratom oder ($C_1$-$C_3$)Alkyl substituiert ist;
- $R_1$ bedeutet:
    - eine -$NR_8R_9$-Gruppe;
    - einen Piperidin-4-ylrest oder einen Piperidin-3-ylrest, der unsubstituiert oder einfach oder mehrfach durch ein ($C_1$-$C_4$)Alkyl, ein ($C_3$-$C_5$)Cycloalkyl substituiert ist, wobei die Kohlenstoffatome auch durch ein oder mehrere Fluoratome substituiert sein können;

- $R_2$ bedeutet ein Wasserstoffatom, eine Alk-Gruppe, eine OAlk-Gruppe;
- $R_3$ bedeutet Methoxy;
- $R_4$ bedeutet ein Wasserstoffatom, ein Halogenatom, eine Alk-Gruppe, ein Hydroxyl, eine OAlk-Gruppe;
- $R_5$ bedeutet ein Wasserstoffatom, ein Halogenatom, eine Alk-Gruppe, ein Hydroxyl, eine OAlk-Gruppe, eine $-CO_2$Alk-Gruppe, einen $-CH_2OH$-Rest;
- $R_6$ bedeutet ein Wasserstoffatom, eine Alk-Gruppe, ein Hydroxyl, eine OAlk-Gruppe, ein $(C_3-C_5)$Cycloalkyloxy, eine $-NR_{10}CONR_{11}R_{12}$-Gruppe;
- $R_7$ bedeutet ein Wasserstoffatom, ein Halogenatom, eine Alk-Gruppe, ein Hydroxyl, eine OAlk-Gruppe;
- oder $R_7$ befindet sich in der -3-Stellung des Phenyls und bedeutet zusammen mit $R_6$ einen Trimethylenrest;
- $R_8$ und $R_9$ bedeuten jeweils unabhängig voneinander ein Wasserstoffatom oder ein $(C_1-C_4)$Alkyl;
- oder $R_8$ und $R_9$ bilden gemeinsam mit dem Stickstoffatom, an das sie gebunden sind, einen gesättigten oder ungesättigten heterocyclischen Ring mit 3 bis 10 Ringgliedern, wobei der heterocyclische Rest unsubstituiert oder einfach oder mehrfach durch ein Amino, ein Dimethylamino, ein Hydroxyl, eine Alk-Gruppe, ein $(C_3-C_5)$ Cycloalkyl, eine OAlk-Gruppe, einen $-SO_2$-Alk-Rest substituiert ist, wobei die Kohlenstoffatome auch durch ein oder mehrere Fluoratome substituiert sein können;
- $R_{10}$ bedeutet ein Wasserstoffatom oder ein $(C_1-C_4)$Alkyl;
- $R_{11}$ und $R_{12}$ bedeuten jeweils unabhängig voneinander ein Wasserstoffatom oder ein $(C_1-C_4)$Alkyl;
- Alk bedeutet ein $(C_1-C_4)$Alkyl, das unsubstituiert oder einfach oder mehrfach durch ein Fluoratom substituiert ist;

als Base oder als Säureadditionssalz sowie als Hydrate oder Solvate.

**2.** Verbindungen der Formel (I) nach Anspruch 1, mit den folgenden Bedeutungen:

- X bedeutet einen zweiwertigen $(C_1-C_5)$Alkylenrest, der unsubstituiert oder einfach oder mehrfach an einem Kohlenstoffatom durch ein Fluoratom oder durch ein $(C_1-C_3)$Alkyl substituiert ist;
- $R_1$ bedeutet:

  • eine $-NR_8R_9$-Gruppe;
  • einen Piperidin-4-ylrest oder einen Piperidin-3-ylrest, der unsubstituiert oder durch Methyl substituiert ist;

- $R_2$ bedeutet ein Halogenatom, Methyl, Methoxy;
- $R_3$ bedeutet Methoxy;
- $R_4$ bedeutet ein Wasserstoffatom, ein Fluoratom, Methyl oder Methoxy;
- $R_5$ bedeutet ein Wasserstoffatom, ein Halogenatom, eine $-CO_2$Alk-Gruppe, einen $-CH_2OH$-Rest;
- $R_6$ bedeutet ein Wasserstoffatom, eine Alk-Gruppe, eine OAlk-Gruppe, Hydroxyl, Cyclopentyloxy, eine $-NHCON(Et)_2$-Gruppe;
- $R_7$ bedeutet ein Wasserstoffatom, ein Halogenatom, Methyl, Methoxy, einen $CF_3$-Rest;
- oder $R_7$ steht in -3-Stellung des Phenyls und bildet gemeinsam mit $R_6$ einen Trimethylenrest;
- $R_8$ und $R_9$ bedeuten jeweils Methyl;
- oder $R_8$ und $R_9$ bilden gemeinsam mit dem Stickstoffatom, an das sie gebunden sind, einen heterocyclischen Rest, der aus der folgenden Reihe ausgewählt ist: Pyrrolidin-1-yl, Piperidin-1-yl, Morpholin-4-yl, Piperazin-1-yl, 1,4-Diazepan-1-yl, 2,5-Diazabicyclo[2.2.1]hept-2-yl, 3,8-Diazabicyclo[3.2.1]oct-3-yl, 2,5-Diazabicyclo[2.2.2]oct-2-yl, 1,4-Diazabicyclo[3.2.1]oct-4-yl, Hexahydropyrrolo[1,2-a]pyrazin-2-(1*H*)-yl, Octahydro-2H-pyrido[1,2-a]pyrazin-2-yl, wobei der heterocyclische Rest unsubstituiert oder einfach oder zweifach durch ein Fluoratom, Amino, Dimethylamino, Hydroxyl, eine Alk-Gruppe, Cyclobutyl, einen $-SO_2Me$-Rest substituiert ist;
- Alk bedeutet ein $(C_1-C_4)$Alkyl, das unsubstituiert oder einfach oder mehrfach durch ein Fluoratom substituiert ist;

als Base oder als Säureadditionssalz, sowie als Hydrat oder Solvat.

**3.** Verbindungen der Formel (I) nach Anspruch 1, mit den folgenden Bedeutungen:

- X bedeutet einen zweiwertigen $(C_1-C_5)$Alkylenrest;
- $R_1$ bedeutet:

  • Dimethylamino, 3-Aminopyrrolidin-1-yl, 3-Dimethylaminopyrrolidin-1-yl, Piperidinyl-1-yl, 3,3-Difluorpiperidin-1-yl, 4,4-Difluorpiperidin-1-yl, 4-Hydroxypiperidin-1-yl, Morpholin-4-yl, 4-Methylpiperazin-1-yl, Piperazin-1-yl, 4-Ethylpiperazin-1-yl, 1,4-Diazepan-1-yl, 2,5-Diazabicyclo[2.2.1]hept-2-yl, 4-Isopropylpiperazin-1-yl, 3-Methylpiperazin-1-yl, 3,4-Dimethylpiperazin-1-yl, 4-Cyclobutylpiperazin-1-yl, 5-Methyl-2,5-diazabicyclo[2.2.1]hept-2-yl, 3,5-Dimethylpiperazin-1-yl, 4-Methyl-1,4-diazepan-1-yl, 8-Methyl-3,8-diazabicyclo [3.2.1]oct-3-yl, 2,6-Dimethylpiperazin-1-yl, 3-Isopropylpiperazin-1-yl, 2,2-Dimethylpiperazin-1-yl, 2,5-Diazabicyclo[2.2.2]oct-2-yl, 2,5-Dimethylpiperazin-1-yl, 2,2,4-Trimethylpiperazin-1-yl, 1,4-Diazabicyclo[3.2.1] oct-4-yl, 2-Isopropylpiperazin-1-yl, Hexhydropyrrolo[1.2-a]pyrazin-2-(1H)yl, Octahydro-2H-pyrido[1.2-a] pyrazin-2-yl, 3-Trifluormethylpiperazin-1-yl, 4-Methylsulfonylpiperazin-1-yl, 4-(Dimethylamino)piperidin-1-yl,

• Piperidin-4-yl, 1-Methylpiperidin-4-yl, 1-Methylpiperidin-3-yl, Piperidin-3-yl;

- $R_2$ bedeutet ein Chloratom, Fluoratom, Bromatom, Methyl, Methoxy;
- $R_3$ bedeutet Methoxy;
- $R_4$ bedeutet ein Wasserstoffatom, ein Fluoratom, Methyl, Methoxy;
- $R_5$ bedeutet ein Wasserstoffatom, 3-Chlor-, 5-Fluor-, 6-Fluor-, 5-Methoxycarbonyl, 5-Hydroxymethyl;
- $R_6$ bedeutet ein Wasserstoffatom, Methyl, Isopropyl, Methoxy, Ethoxy, Isopropoxy, Butyloxy, tert.-Butyloxy, Cyclopentyloxy, 1,1,2,2-Tetrafluorethyloxy, 2,2-Diethylureido, Difluormethoxy, Trifluormethoxy, Hydroxy;
- $R_7$ bedeutet ein Wasserstoffatom, 3-Methyl, 2-Methoxy, 3-Methoxy, 3-Fluor-, 3-Chlor-, 3-$CF_3$;
- oder $R_7$ befindet sich in -3-Stellung des Phenyls und bildet gemeinsam mit $R_6$ einen Trimethylenrest;

als Base oder als Säureadditionssalz sowie als Hydratsolvat.

**4.** Verbindung nach Anspruch 1 der Formel (I), mit den folgenden Bedeutungen:

- X bedeutet einen zweiwertigen Trimethylen- oder Pentamethylenrest;
- $R_1$ bedeutet:

• Dimethylamino, 3-Aminopyrrolidin-1-yl, Piperidin-1-yl, 4,4-Difluorpiperidin-1-yl, 4-Hydroxypiperidin-1-yl, 4-(Dimethylamino)piperidin-1-yl, Morpholin-4-yl, 4-Methylpiperazin-1-yl, Piperazin-1-yl, 3,4-Dimethylpiperazin-1-yl, 5-Methyl-2,5-diazabicyclo[2.2.1]hept-2-yl, 4-Methyl-1,4-diazepan-1-yl, 8-Methyl-3,8-diazabicyclo[3.2.1]oct-3-yl, 1,4-Diazabicyclo[3.2.1]oct-4-yl;

• Piperidin-4-yl;

- $R_2$ bedeutet ein Chlor-, Fluor-, Bromatom oder Methyl;
- $R_3$ bedeutet Methoxy;
- $R_4$ bedeutet ein Wasserstoffatom oder ein Fluoratom;
- $R_5$ bedeutet ein Wasserstoffatom, 5-Methoxycarbonyl, 5-Hydroxymethyl;
- $R_6$ bedeutet ein Wasserstoffatom, Methyl, Methoxy, Isopropoxy, Ethoxy, Butyloxy, 1,1,2,2-Tetrafluorethyloxy, 2,2-Diethylureido, Difluormethoxy, Trifluormethoxy, Hydroxyl;
- $R_7$ bedeutet ein Wasserstoffatom, 2-Methoxy, 3-Methoxy, 3-Methyl, 3-Fluor;

als Base oder als Säureadditionssalz sowie als Hydrat oder Solvat.

**5.** Verbindung nach Anspruch 1 der Formel (I), ausgewählt aus der folgenden Reihe:

- 5-Chlor-1-[(2,4-Dimethoxyphenyl)sulfonyl]-3-(2-fluorphenyl)-6-methoxy-3-(3-piperidin-4-ylpropyl)-1,3-dihydro-2*H*-indol-2-on, rechtsdrehendes Isomer;
- 5-Chlor-1-[(2,4-Dimethoxyphenyl)sulfonyl]-3-(2-fluorphenyl)-6-methoxy-3-(3-piperidin-4-ylpropyl)-1,3-dihydro-2*H*-indol-2-on, linksdrehendes Isomer;
- 5-Chlor-3-[3-(dimethylamino)propyl]-3-(2-fluorphenyl)-1-[(4-isopropoxyphenyl)sulfonyl]-6-methoxy-1,3-dihydro-2*H*-indol-2-on, rechtsdrehendes Isomer;
- 3-{3-[(3*R*)-3-Aminopyrrolidin-1-yl]propyl}-5-chlor-3-(2-fluorphenyl)-1-[(4-isopropoxyphenyl)sulfonyl]-6-methoxy-1,3-dihydro-2*H*-indol-2-on, rechtsdrehendes Isomer;
- 3-{3-[(3S)-3-Aminopyrrolidin-1-yl]propyl}-5-chlor-3-(2-fluorphenyl)-1-[(4-isopropoxyphenyl)sulfonyl]-6-methoxy-1,3-dihydro-2*H*-indol-2-on, rechtsdrehendes Isomer;
- 3-{3-[(3*R*)-3-Aminopyrrolidin-1-yl]propyl}-5-chlor-1-[(4-ethoxy-3-methoxyphenyl)sulfonyl]-3-(2-fluorphenyl)-6-methoxy-1,3-dihydro-2*H*-indol-2-on, rechtsdrehendes Isomer;
- 3-{3-[(3*S*)-3-Aminopyrrolidin-1-yl]propyl}-5-chlor-1-[(4-ethoxy-3-methoxyphenyl)sulfonyl]-3-(2-fluorphenyl)-6-methoxy-1,3-dihydro-2*H*-indol-2-on, rechtsdrehendes Isomer;
- 5-Chlor-1-[(2,4-dimethoxyphenyl)sulfonyl]-3-(2-fluorphenyl)-6-methoxy-3-(3-piperidin-1-ylpropyl)-1,3-dihydro-2*H*-indol-2-on, rechtsdrehendes Isomer;

- 5-Chlor-3-[3-(4,4-difluorpiperidin-1-yl)propyl]-1-[(4-ethoxy-3-methoxyphenyl)sulfonyl]-3-(2-fluorphenyl)-6-methoxy-1,3-dihydro-2*H*-indol-2-on, rechtsdrehendes Isomer;
- 5-Chlor-1-[(4-ethoxy-3-methoxyphenyl)sulfonyl]-3-(2-fluorphenyl)-3-[3-(4-hydroxypiperidin-1-yl)propyl]-6-methoxy-1,3-dihydro-2*H*-indol-2-on, rechtsdrehendes Isomer;
- 5-Chlor-1-[(4-ethoxy-3-methoxyphenyl)sulfonyl]-3-(2-fluorphenyl)-6-methoxy-3-(3-morpholin-4-ylpropyl)-1,3-dihydro-2*H*-indol-2-on, rechtsdrehendes Isomer;
- 5-Chlor-1-[(2,4-dimethoxyphenyl)sulfonyl]-3-(2-fluorphenyl)-6-methoxy-3-[3-(4-methylpiperazin-1-yl)propyl]-1,3-dihydro-1*H*-indol-2-on, rechtsdrehendes Isomer;
- 5-Chlor-3-(2-fluorphenyl)-1-[(4-isopropoxyphenyl)sulfonyl]-6-methoxy-3-[3-(4-methylpiperazin-1-yl)propyl]-1,3-dihydro-2*H*-indol-2-on, rechtsdrehendes Isomer;
- 5-Chlor-1-[(4-ethoxy-3-methoxyphenyl)sulfonyl]-3-(2-fluorphenyl)-6-methoxy-3-[3-(4-methylpiperazin-1-yl) propyl]-1,3-dihydro-2*H*-indol-2-on, rechtdrehendes Isomer;
- 5-Chlor-1-[(3,4-dimethoxyphenyl)sulfonyl]-3-(2-fluorphenyl)-6-methoxy-3-[3-(4-methylpiperazin-1-yl)propyl]-1,3-dihydro-2*H*-indol-2-on, rechtsdrehendes Isomer;
- 5-Chlor-1-[(2,4-dimethoxyphenyl)sulfonyl]-3-(2-fluorphenyl)-6-methoxy-3-(3-piperazin-1-ylpropyl)-1,3-dihydro-2*H*-indol-2-on, rechtsdrehendes Isomer;
- 5-Chlor-3-(2-fluorphenyl)-1-[(4-isopropoxyphenyl)sulfonyl]-6-methoxy-3-(3-piperazin-1-ylpropyl)-1,3-dihydro-2*H*-indol-2-on, rechtsdrehendes Isomer;
- 5-Chlor-1-[(4-ethoxy-3-methoxyphenyl)sulfonyl]-3-(2-fluorphenyl)-6-methoxy-3-(3-piperazin-1-ylpropyl)-1,3-dihydro-2*H*-indol-2-on, rechtsdrehendes Isomer;
- 5-Chlor-1-[(3,4-dimethoxyphenyl)sulfonyl]-3-(2-fluorphenyl)-6-methoxy-3-(3-piperazin-1-ylpropyl)-1,3-dihydro-2*H*-indol-2-on, rechtsdrehendes Isomer;
- 5-Chlor-1-[(3,4-dimethylphenyl)sulfonyl]-3-(2-fluorphenyl)-6-methoxy-3-(3-piperazin-1-ylpropyl)-1,3-dihydro-2*H*-indol-2-on, rechtsdrehendes Isomer;
- 5-Chlor-3-(2-fluorphenyl)-6-methoxy-1-[(4-methoxy-3-methylphenyl)sulfonyl]-3-(3-piperazin-1-ylpropyl)-1,3-dihydro-2*H*-indol-2-on;
- 5-Chlor-1-[(4-ethoxyphenyl)sulfonyl]-3-(2-fluorphenyl)-6-methoxy-3-(3-piperazin-1-ylpropyl)-1,3-dihydro-2*H*-indol-2-on, rechtsdrehendes Isomer;
- 5-Chlor-1-[(4-ethoxy-3-methylphenyl)sulfonyl]-3-(2-fluorphenyl)-6-methoxy-3-(3-piperazin-1-ylpropyl)-1,3-dihydro-2*H*-indol-2-on;
- 5-Chlor-1-{[4-(difluormethoxy)phenyl]sulfonyl}-3-(2-fluorphenyl)-6-methoxy-3-(3-piperazin-1-ylpropyl)-1,3-dihydro-2*H*-indol-2-on, rechtsdrehendes Isomer;
- 5-Chlor-3-(2-fluorphenyl)-6-methoxy-3-(3-piperazin-1-ylpropyl)-1-{[4-(trifluormethoxy)phenyl]sulfonyl}-1,3-dihydro-2*H*-indol-2-on, rechtdrehendes Isomer;
- 5-Chlor-3-(2-fluorphenyl)-6-methoxy-1-(phenylsulfonyl)-3-(3-piperazin-1-ylpropyl)-1,3-dihydro-2*H*-indol-2-on, rechtdrehendes Isomer;
- 5-Chlor-1-(2,3-dihydro-1*H*-inden-5-ylsulfonyl)-3-(2-fluorphenyl)-6-methoxy-3-(3-piperazin-1-ylpropyl)-1,3-dihydro-2*H*-indol-2-on, rechtsdrehendes Isomer;
- 5-Chlor-1-{[3-fluor-4-(1-methylethoxy)phenyl]sulfonyl}-3-(2-fluorphenyl)-6-methoxy-3-[3-(4-methylpiperazin-1-yl)propyl]-1,3-dihydro-2*H*-indol-2-on, rechtsdrehendes Isomer;
- 5-Chlor-3-(2-fluorphenyl)-6-methoxy-3-[3-(4-methylpiperazin-1-yl)propyl]-1-{[4-(1,1,2,2-tetrafluorethoxy)phenyl]sulfonyl}-1,3-dihydro-2*H*-indol-2-on, rechtsdrehendes Isomer;
- 3-(2-Fluorphenyl)-6-methoxy-5-methyl-1-{[4-(1-methylethoxy)phenyl]sulfonyl}-3-[3-(4-methylpiperazin-1-yl) propyl]-1,3-dihydro-2*H*-indol-2-on, rechtsdrehendes Isomer;
- 5-Fluor-3-(2-fluorphenyl)-6-methoxy-1-{[4-(1-methylethoxy)phenyl]sulfonyl}-3-[3-(4-methylpiperazin-1-yl)propyl]-1,3-dihydro-2*H*-indol-2-on, rechtsdrehendes Isomer;
- 5-Brom-3-(2-fluorphenyl)-6-methoxy-1-{[4-(1-methylethoxy)phenyl]sulfonyl}-3-[3-(4-methylpiperazin-1-yl) propyl]-1,3-dihydro-2*H*-indol-2-on, rechtsdrehendes Isomer;
- 3-[4-({3-[3-(Dimethylamino)propyl]-3-(2-fluorphenyl)-6-methoxy-5-methyl-2-oxo-2,3-dihydro-1*H*-indol-1-yl} sulfonyl)-2-fluorphenyl]-l,l-diethylharnstoff, rechtsdrehendes Isomer;
- 3-[4-({3-[3-(Dimethylamino)propyl]-3-(2-fluorphenyl)-6-methoxy-5-methyl-2-oxo-2,3-dihydro-1*H*-indol-1-yl} sulfonyl)-2-methylphenyl]-1,1-diethylharnstoff, rechtsdrehendes Isomer;
- 5-Chlor-3-(2-fluorphenyl)-6-methoxy-3-[3-(8-methyl-3,8-diazabicyclo[3.2.1]oct-3-yl)propyl]-1-{[4-(1-methylethoxy)phenyl]sulfonyl}-1,3-dihydro-2*H*-indol-2-on, rechtsdrehendes Isomer;
- 5-Chlor-3-(2-fluorphenyl)-6-methoxy-3-[3-(5-methyl-2,5-diazabicyclo[2.2.1]hept-2-yl)propyl]-1-{[4-(1-methylethoxy)phenyl]sulfonyl}-1,3-dihydro-2*H*-indol-2-on, rechtsdrehendes Isomer;
- 5-Chlor-3-(2-fluorphenyl)-1-[(4-hydroxyphenyl)sulfonyl]-6-methoxy-3-[3-(4-methylpiperazin-1-yl)propyl]-1,3-dihydro-2*H*-indol-2-on, rechtsdrehendes Isomer;

- 5-Chlor-3-(2-fluorphenyl)-6-methoxy-3-[3-(4-methyl-1,4-diazepan-1-yl)propyl]-1-{[4-(1-methylethoxy)phenyl] sulfonyl}-1,3-dihydro-2*H*-indol-2-on, rechtsdrehendes Isomer;
- 5-Chlor-3-(2-fluorphenyl)-6-methoxy-1-{[4-(1-methylethoxy)phenyl]sulfonyl}-3-(5-piperidin-1-ylpentyl)-1,3-dihydro-2*H*-indol-2-on, rechtsdrehendes Isomer;
- 5-Chlor-3-[5-(dimethylamino)pentyl]-3-(2-fluorphenyl)-6-methoxy-1-{[4-(1-methylethoxy)phenyl]sulfonyl}-1,3-dihydro-2*H*-indol-2-on, rechtsdrehendes Isomer;
- 5-Chlor-3-{3-[(3S)-3,4-dimethylpiperazin-1-yl]propyl}-3-(2-fluorphenyl)-6-methoxy-1-{[4-(1-methylethoxy) phenyl]sulfonyl}-1,3-dihydro-2H-indol-2-on, rechtsdrehendes Isomer;
- 5-Chlor-6-methoxy-1-{[4-(1-methylethoxy)-phenyl]sulfonyl}-3-[3-(4-methylpiperazin-1-yl)propyl]-3-phenyl-1,3-dihydro-2*H*-indol-2-on, rechtsdrehendes Isomer;
- 5-Chlor-3-{3-[(5S)-1,4-diazabicyclo[3.2.1]oct-4-yl]propyl}-3-(2-fluorphenyl)-6-methoxy-1-{[4-(1-methylethoxy) phenyl]sulfonyl}-1,3-dihydro-2*H*-indol-2-on, rechtsdrehendes Isomer;
- 1-[(4-Butoxyphenyl)sulfonyl]-5-chlor-3-(2-fluorphenyl)-6-methoxy-3-[3-(4-methylpiperazin-1-yl)propyl]-1,3-dihydro-2*H*-indol-2-on, rechtsdrehendes Isomer;
- 5-Chlor-3-[3-[4-(dimethylamino)piperidin-1-yl]propyl]-3-(2-fluorphenyl)-1-[(4-isopropoxyphenyl)sulfonyl]-6-methoxy-1,3-dihydro-2H-indol-2-on;
- 1-[(3-Fluor-4-isopropoxyphenyl)sulfonyl]-3-(2-fluorphenyl)-5,6-dimethoxy-3-[3-(4-methylpiperazin-1-yl)propyl]-1,3-dihydro-2H-indol-2-on;
- 5-Fluor-1-[(3-fluor-4-isopropoxyphenyl)sulfonyl]-3-(2-fluorphenyl)-6-methoxy-3-[3-(4-methylpiperazin-1-yl) propyl]-1,3-dihydro-2H-indol-2-on;
- 3-(2-Fluorphenyl)-5,6-dimethoxy-1-[(4-methoxy-3-methylphenyl)sulfonyl]-3-[3-(4-methylpiperazin-1-yl)propyl]-1,3-dihydro-2H-indol-2-on;
- Methyl-3-[5-Chlor-1-[(4-isopropoxyphenyl)sulfonyl]-6-methoxy-3-[3-(4-methylpiperazin-1-yl)propyl]-2-oxo-2,3-dihydro-1H-indol-3-yl]-4-fluorbenzoat
- 5-Chlor-3-[2-fluor-5-(hydroxymethyl)phenyl]-1-[(4-isopropoxyphenyl)sulfonyl]-6-methoxy-3-[3-(4-methylpiperazin-1-yl)propyl]-1,3-dihydro-2H-indol-2-on;

als Base oder als Säureadditionssalze sowie als Hydrate oder Solvate.

6. Verfahren zur Herstellung von Verbindungen der Formel (I) nach einem der Ansprüche 1 bis 5, **dadurch gekennzeichnet, dass** man:

eine Verbindung der Formel:

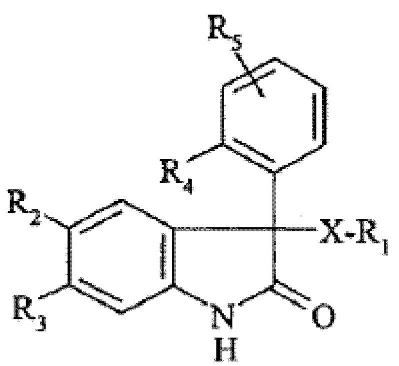

(II)

in der X, $R_1$, $R_2$, $R_3$, $R_4$ und $R_5$ wie für eine Verbindung der Formel (I) in Anspruch 1 definiert sind, mit einem Sulfonylhalogenid der Formel:

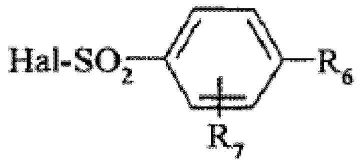

(III)

in der $R_6$ und $R_7$ wie für eine Verbindung der Formel (I) in Anspruch 1 definiert sind und Hal ein Halogenatom

bedeutet, in Gegenwart einer Base umsetzt.

7. Verfahren zur Herstellung von Verbindungen von Formel (I) nach einem der Ansprüche 1 bis 5, **dadurch gekennzeichnet, dass** man:

eine Verbindung der Formel

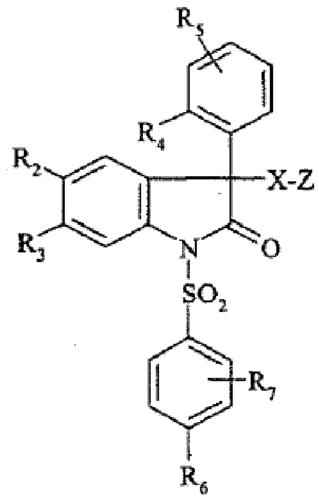

(IV)

in der X, $R_2$, $R_3$, $R_4$, $R_5$, $R_6$ und $R_7$ wie für eine Verbindung der Formel (I) in Anspruch 1 definiert sind und Z eine Abgangsgruppe, ausgewählt aus der Reihe Halogenatom oder Methansulfonatgruppe oder p-Toluolsulfonatgruppe bedeutet, mit einer Verbindung der Formel:

$R_1$-H (V)

in der $R_1$ wie für eine Verbindung der Formel (I) in Anspruch 1 definiert ist, umsetzt.

8. Arzneimittel, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure oder ein Hydrat oder ein Solvat der Verbindung der Formel (I) umfasst.

9. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Grundstoff umfasst.

10. Verwendung einer Verbindung der Formel (I) nach einem der Ansprüche 1 bis 5 für die Herstellung eines Arzneimittels zur Behandlung und Vorbeugung von Hypertonie, pulmonaler Hypertonie, Herzinsuffizienz, Myokardinfarkt, koronarem Vasospasmus, insbesondere bei Rauchern, Raynaud-Krankheit, instabiler Angina und PTCA (englisch: Percutaneous Transluminal Coronary Angioplasty), zerebralem Vasospasmus, Hirnblutung, Hirntraumen und -ödemen, Depression, Angstzuständen, Stress, emotionalen Problemen, Gedächtnisproblemen, Schlafproblemen, diabetischer Nephropathie, Karzinomen wie kleinzelligem Lungenkarzinom oder Mammakarzinomen; Arteriosklerose; metabolischem Syndrom; Hyperlipidämie; Insulinresistenz, Hypertriglyceridämie, Hypercortisolämie, Hyperaldosteronämie, Phäochromocytomen, Cushing-Syndrom, Colitis, Reizkolon, Morbus Chron, Schmerzen (Fibromyalgie), neurodegenerativen Erkrankungen (Morbus Alzheimer, Morbus Parkinson, Huntington-Chorea), Substanzabhängigkeit (Alkohol oder Drogen); als Tokolytikum oder als Uterusrelaxant oder für die Kontrolle von Uterushyperaktivität, von vorzeitigen Wehen, zur Förderung des Fötalwachstums in utero, zum Behandeln von Endometriose, von Dysmenorrhö und von Erektionsstörungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9315051 A
- EP 636608 A
- EP 636609 A
- WO 9715556 A
- WO 9825901 A
- WO 0155130 A
- WO 0155134 A
- WO 0164668 A
- WO 0198295 A
- WO 03008407 A
- WO 06080574 A
- WO 08025735 A
- WO 9518105 A
- WO 95018105 A
- EP 0469984 B
- EP 061741 A


### Littérature non-brevet citée dans la description


- Progress in Endocrinology. Experta Medica, 1988, 1183-1188
- *J. Lab. Clin. Med.,* 1989, vol. 114 (6), 617-632
- *Pharmacol. Rev.,* 1991, vol. 43 (1), 73-108
- **G. GUILLON et al.** *Endocrinology,* 1995, vol. 136 (3), 1285-1295
- **G. MAZZOCCHI et al.** *Peptides,* 1997, vol. 18 (2), 191-195
- **E. GRAZZINI et al.** *J. Clin. Endocrinol. Metab.,* 1999, vol. 84 (6), 2195-2203
- **P.J. WOLL et al.** *Biochem. Biophys. Res. Commun.,* 1989, vol. 164 (1), 66-73
- **S. Jard et al.** *Mol. Pharmacol.,* 1986, vol. 30, 171-177
- **Y. Arsenijevic et al.** *J. Endocrinol.,* 1994, vol. 141, 383-391
- **J. Schwartz et al.** *Endocrinology,* 1991, vol. 129 (2), 1107-1109
- **Y. de Keyser et al.** *FEBS Letters,* 1994, vol. 356, 215-220
- **G. E. Gdjjes et al.** *Nature,* 1982, vol. 299, 355
- *Neuroendocrinology,* 1994, vol. 60, 503-508
- **Y. de Keyser.** *FEBS Letters,* 1994, vol. 356, 215-220
- **T. Sugimoto et al.** *J. Biol. Chem.,* 1994, vol. 269 (43), 27088-27092
- **M. Saito et al.** *Biochem. Biophys. Res. Commun.,* 1995, vol. 212 (3), 751-757
- **S. J. Lolait et al.** *Neurobiology,* 1996, vol. 92, 6783-6787
- **M. A. Ventura et al.** *Journal of Molecular Endocrinology,* 1999, vol. 22, 251-260
- **B. Lee et al.** *Am. J. Physiol. 269 (Endocrinol. Metab. 32),* 1995, vol. 269 (32), E1095-E1100
- **E. Grazzini et al.** *Endocrinology,* 1996, vol. 137 (a), 3906-3914
- **G. Guillon et al.** *Endocrinology,* 1995, vol. 136 (3), 12851295
- **J. Bertherat et al.** *Eur. J. Endocrinol.,* 1996, vol. 135, 173
- **G. A. Wuinert et al.** *Lancet,* 1990, vol. 335, 991-994
- **G. Dickstein et al.** *J. Clin. Endocrinol. Metab.,* 1996, vol. 81 (8), 2934-2941
- **P. J. Woll et al.** *Biochem. Biophys. Res. Commun.,* 1989, vol. 164 (1), 66-73
- **T. Sugimoto et al.** Molecular cloning and functional expression of V1b receptor gene, dans Neurohypophysis : Recent Progress of Vasopressin and Oxytocin Research. Elsevier Science, 1995, 409413
- **Green et al.** Protective Group in Organic Synthesis. John Wiley & Sons, Inc, 2007
- **M. B. Smith ; J. March.** Advanced Organic Chemistry. Wiley Interscience, 2007, 496-501
- **R. J. Cremlyn.** Chlorosulfonic Acid. The Royal Society of Chemistry, 2002
- **Katristzky.** Advances in Heterocyclic Chemistry. Academic Press, 1975, vol. 18, 1-58
- *Bioorg. Med. Chem. Lett.,* 175-178
- *J. Med. Chem.,* 1994, vol. 37 (16), 2537-2551
- *J. Am. Chem. Soc.,* 2008, vol. 1952, 74
- *J. Org. Chem.,* 2006, vol. 71, 9580
- *Chem. Ber.,* 1906, vol. 39, 2777
- **M. Thibonnier et al.** *J. Biol. Chem.,* 1994, vol. 269, 3304-3310
- **Y. de Keyser et al.** *Febs Letters,* 1994, vol. 356, 215-220
- **M. Birnbaumer et al.** *Nature (Lond.),* 1992, vol. 357, 333-335
- **J. Elands et al.** *Eur. J. Pharmacol.,* 1987, vol. 147, 197-207

- **Sullivan et al.** *Methods Mol. Biol.,* 1999, vol. 114, 125-133
- *J. Clin. Invest.,* 1993, vol. 92, 224-231